# EUROPEAN PATENT APPLICATION

(11) **EP 1 264 820 A1**
(43) Date of publication of application: **11.12.2002**
(21) Application number: 01912338.9
(22) Date of filing: 13.03.2001
(51) Int. Cl.: C07C 233/75

(54) **NOVEL AMIDE COMPOUNDS**

(30) Priority: 14.03.2000 JP 2000070127; 05.10.2000 JP 2000305947
(71) Applicant: FUJISAWA PHARMACEUTICAL CO., LTD., Osaka-shi Osaka 541-8514 (JP)
(72) Inventor: YAMADA, Akira, Fujisawa Pharmaceutical Co., Ltd., Osaka-shi, Osaka 541-8514 (JP); TOMISHIMA, Masaki, Fujisawa Pharmaceutical Co. Ltd, Osaka-shi, Osaka 541-8514 (JP); HAYASHIDA, Hisashi, Fujisawa Pharmaceutical Co.Ltd, Osaka-shi, Osaka 541-8514 (JP); IMANISHI, Masashi, Fujisawa Pharmaceutical Co. Ltd, Osaka-shi, Osaka 541-8514 (JP); SPEARS, Glen W., Fujisawa Pharmaceutical Co., Ltd., Osaka-shi, Osaka 541-8514 (JP); ITO, Kiyotaka, Fujisawa Pharmaceutical Co., Ltd., Osaka-shi, Osaka 541-8514 (JP); TAKAHASHI, Fumie, Fujisawa Pharmaceutical Co., Ltd, Osaka-shi, Osaka 541-8514 (JP); MIYAKE, Hiroshi, Fujisawa Pharmaceutical Co., Ltd., Osaka-shi, Osaka 541-8514 (JP)
(74) Representative: Gille Hrabal Struck Neidlein Prop Roos
(86) International application number: JP0101993
(87) International publication number: WO01068585

(57) **Abstract**

A compound of the formula (I):

R¹-A-X-NHCO-Y-R²

wherein
R¹ is heterocyclic group which may have suitable substituents, or phenyl which may have suitable substituents,
R² is condensed phenyl which may have suitable substituents, phenyl which may have suitable substituents, or thienyl which may have suitable substituents,
A is a group of the formula:

   -(CH₂)ₜ-(O)ₘ-

   or 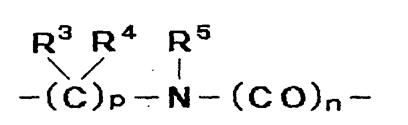 in which R³ and R⁴ are each hydrogen or linked together to form imino,
   R⁵ is hydrogen or lower alkyl,
   t is 0, 1 or 2,
   p, m and n are each 0 or 1,
X is phenylene which may have suitable substituents, or bivalent heterocyclic group containing nitrogen which may have suitable substituents,
Y is bond, lower alkylene, or lower alkenylene,
and a salt thereof.

## Description

### TECHNICAL FIELD

The present invention relates to novel amide compounds and salts thereof. More particularly, it relates to novel amide compounds and salts thereof which have pharmacological activities such as 5-hydroxytryptamine (5-HT) antagonism and the like.

Said compounds or salts thereof are useful as a 5-HT antagonist, especially as a 5-HT_{2c} receptor antagonist, for treating or preventing central nervous system (CNS) disorders such as anxiety, depression, obsessive compulsive disorders, migraine, anorexia, Alzheimer's disease, sleep disorders, bulimia, panic attacks; withdrawal from drug abuse such as cocaine, ethanol, nicotine and benzodiazepines; schizophrenia; and also disorders associated with spinal trauma and/or head injury such as hydrocephalus, and the like in human beings or animals.

### BACKGROUND ART

Some compounds having 5-HT_{2c} receptor antagonism activity have been known as described in the international patent application (International Publication Number WO95/21844, WO95/29177) published based on the Patent Cooperation Treaty, and in JP11-130750A.

### DISCLOSURE OF INVENTION

The compounds and salts thereof of the present invention are novel and can be represented by the following general formula (I):

R¹-A-X-NHCO-Y-R²

wherein
R¹ is heterocyclic group which may have suitable substituents, or phenyl which may have suitable substituents,
R² is condensed phenyl which may have suitable substituents, phenyl which may have suitable substituents, or thienyl which may have suitable substituents,
A is a group of the formula:

   -(CH₂)ₜ-(O)ₘ-

   or 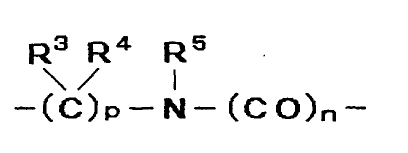 in which R³ and R⁴ are each hydrogen or linked together to form imino,
   R⁵ is hydrogen or lower alkyl,
   t is 0, 1 or 2,
   p, m and n are each 0 or 1,
X is phenylene which may have suitable substituents, or bivalent heterocyclic group containing nitrogen which may have suitable substituents,
Y is bond, lower alkylene, or lower alkenylene.

According to the present invention, the object compounds (I) can be prepared by the following processes: 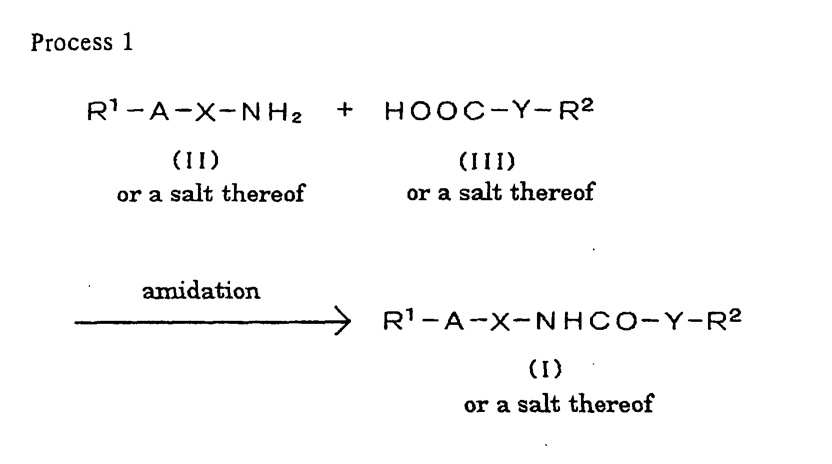 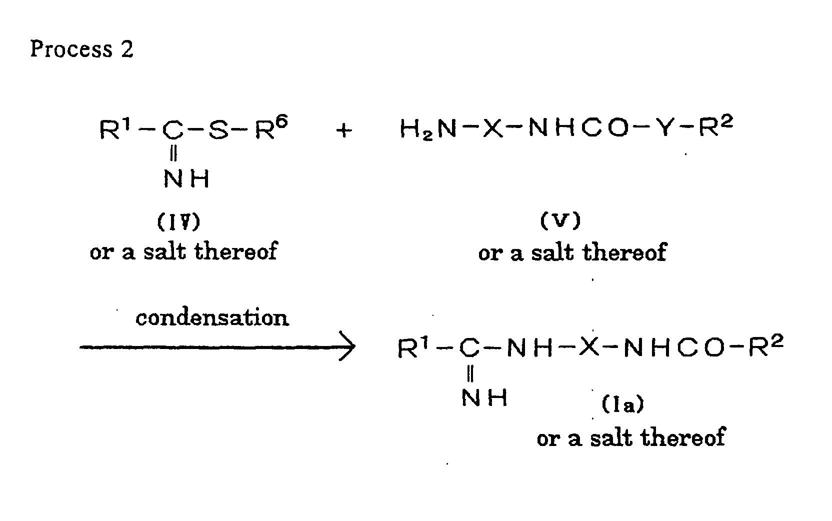 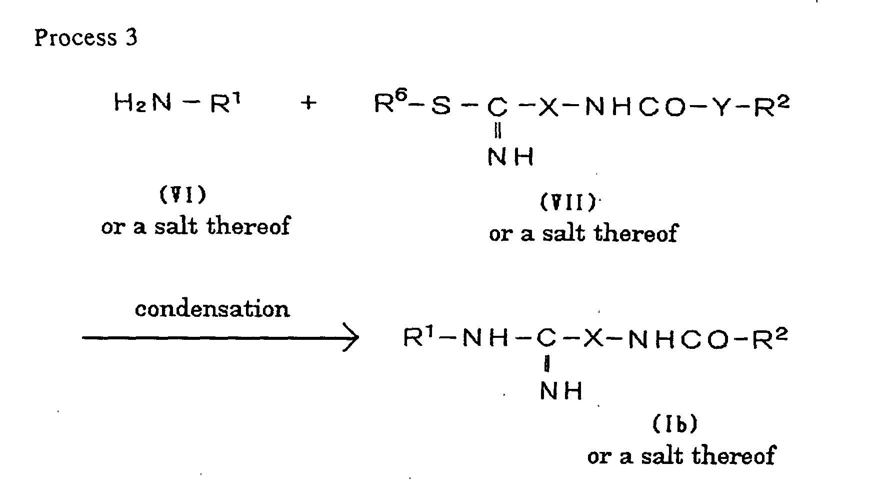 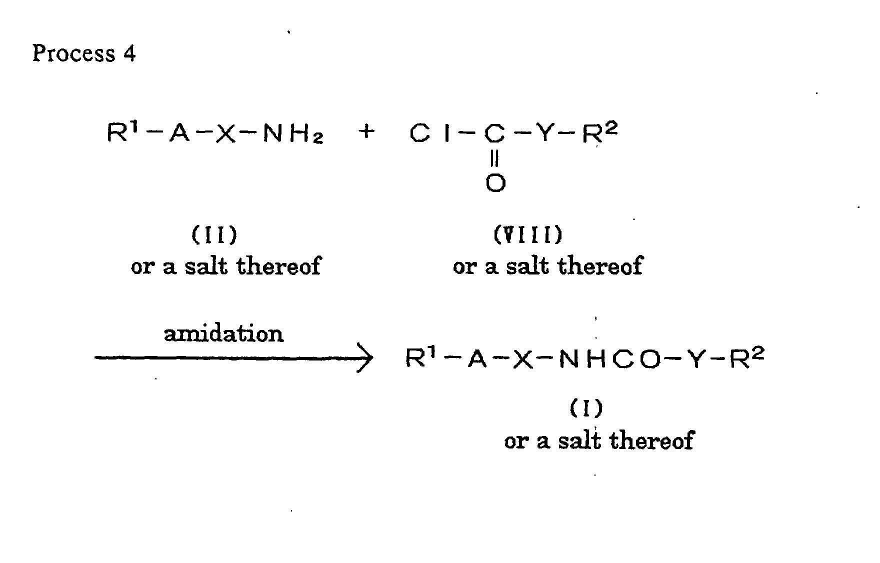 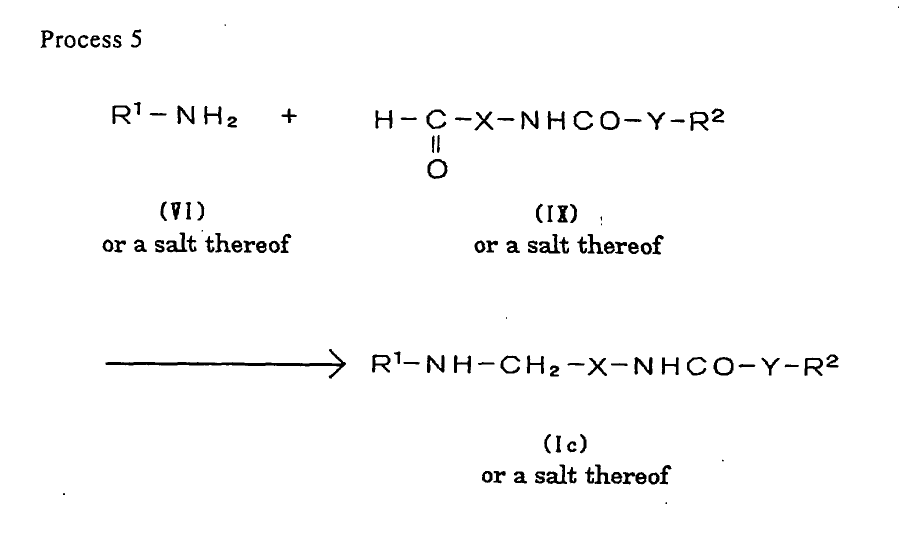 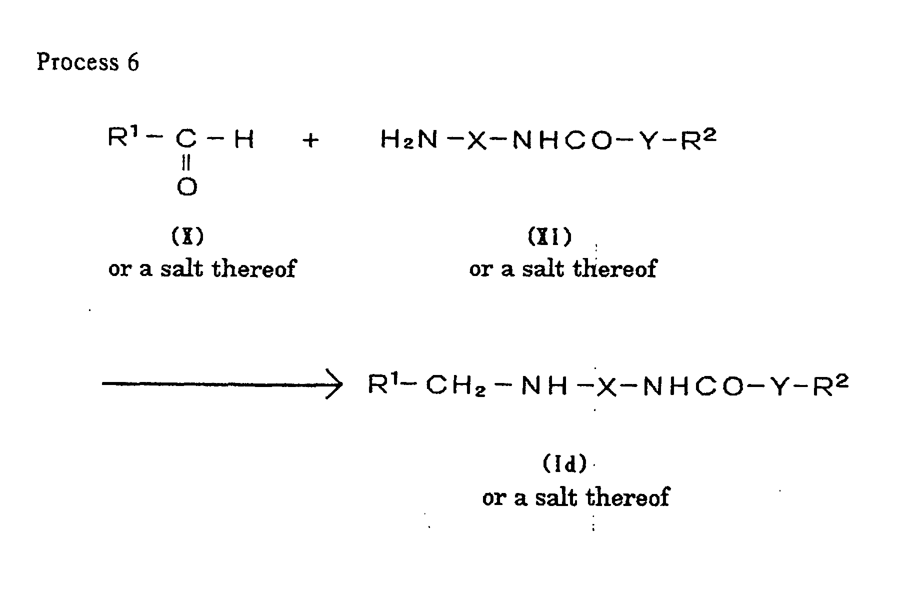 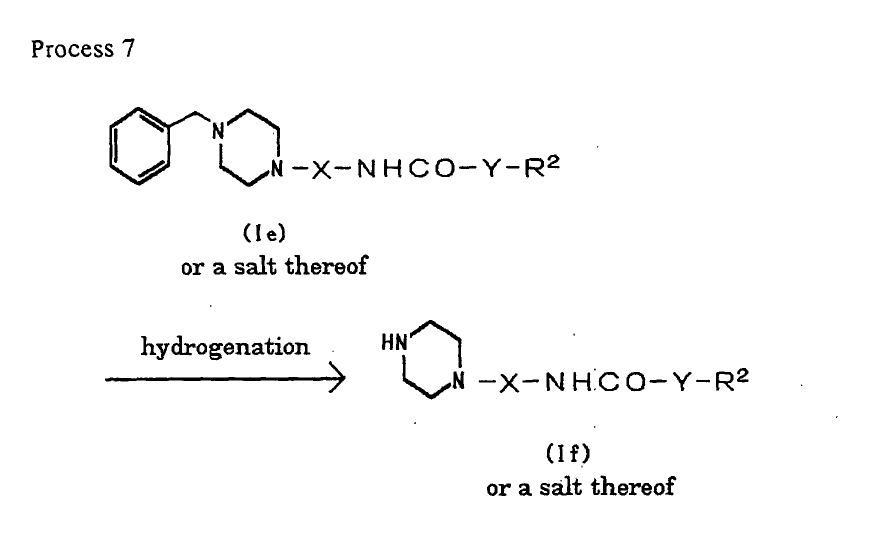 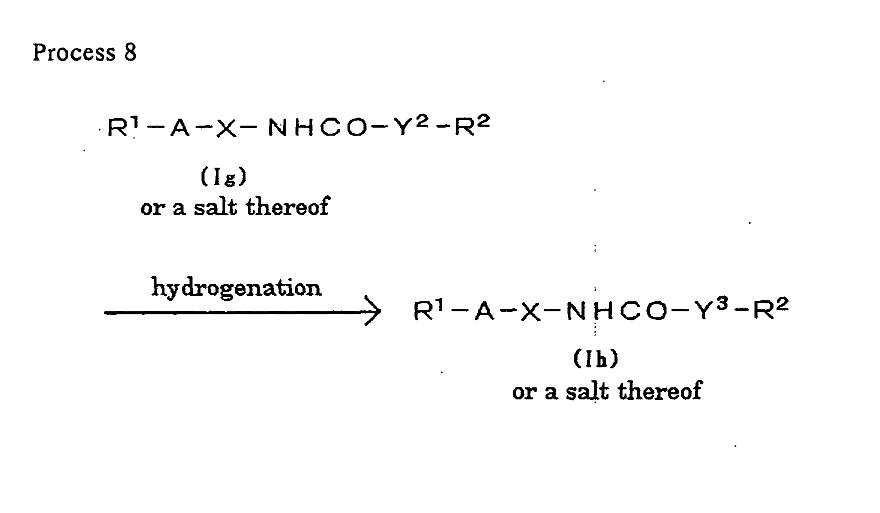 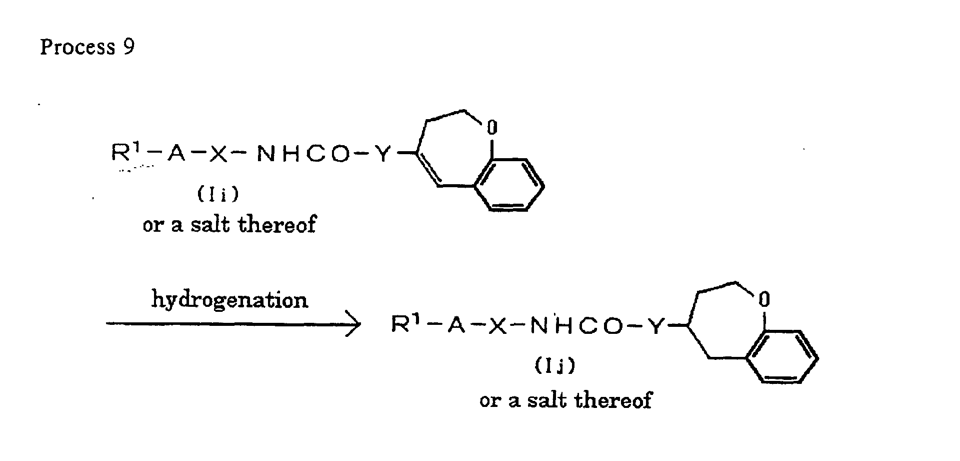 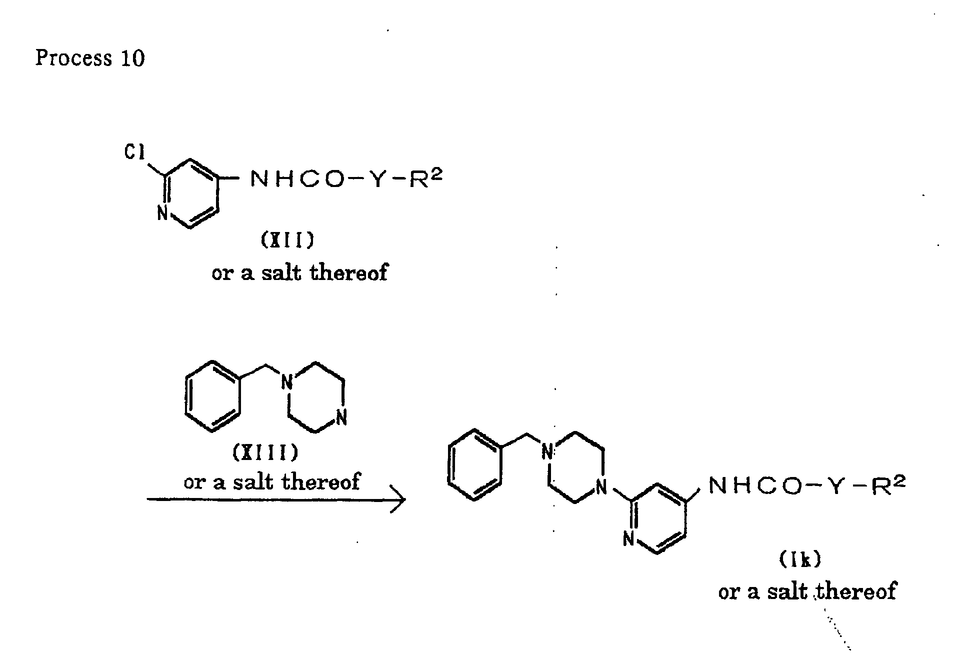 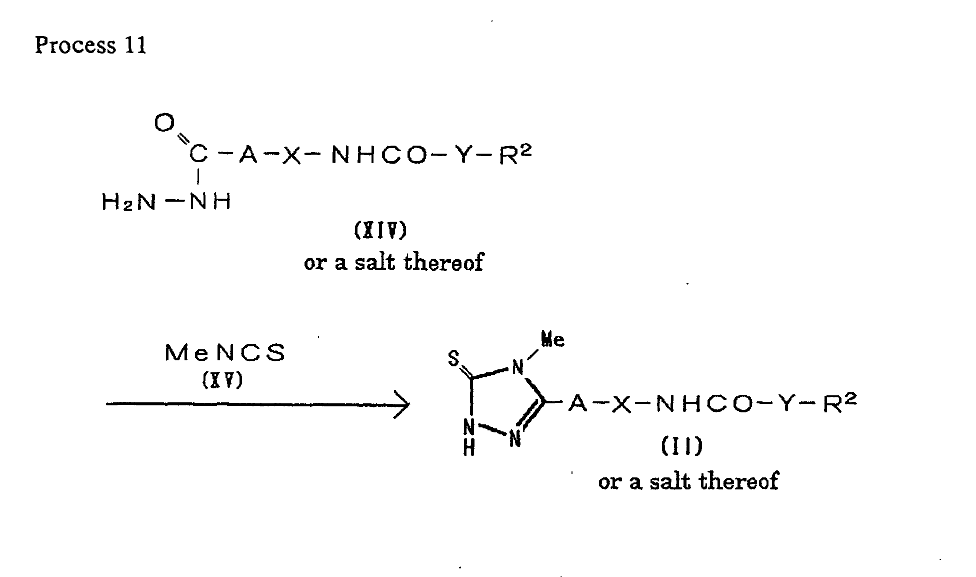 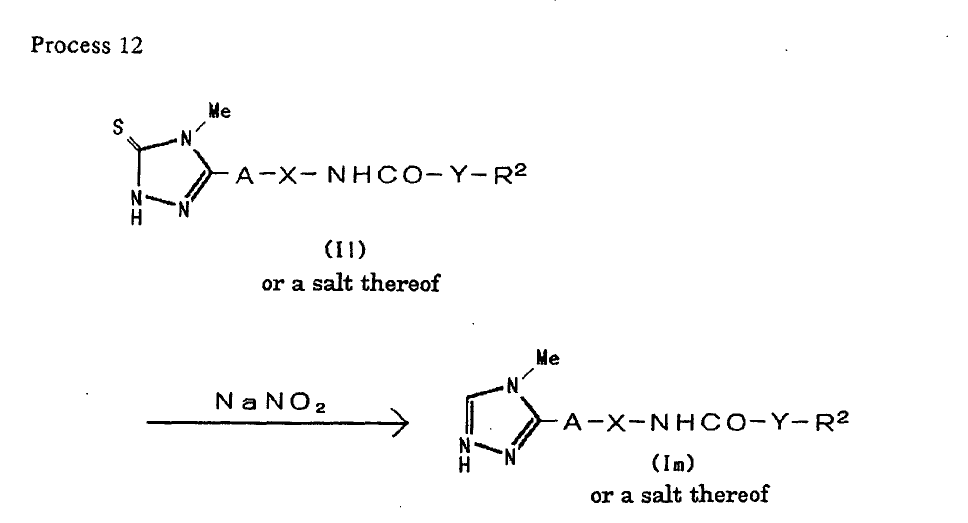 wherein
R¹, R², A, X and Y are each as defined above,
Y² is lower alkenylene,
Y³ is lower alkylene,
R⁶ is lower alkyl.

The object compounds (I) or salts thereof can be prepared, for example, in accordance with the procedures described in the Examples in the present description or similar procedures thereto.

The material used for the object compounds (I) or salts thereof can be prepared, for example, in accordance with the procedures described in the Preparations in the present description or similar procedures thereto.

Suitable salt of the compounds (I), (Ia), (Ib), (Ic), (Id), (Ie), (If), (Ig), (Ih), (Ii), (Ij), (Ik), (Il), (Im), (II), (III), (IV), (V), (VI), (VII), (VIII), (IX), (X), (XI), (XII), (XIII), (XIV), and (XV) are conventional non-toxic pharmaceutically acceptable salt and may include a salt with a base or an acid addition salt such as a salt with an inorganic base, for example, an alkali metal salt (e.g. sodium salt, potassium salt, cesium salt, etc.), and an alkaline earth metal salt (e.g. calcium salt, magnesium salt, etc.), an ammonium salt;
a salt with an organic base, for example, an organic amine salt (e.g. triethylamine salt, pyridine salt, picoline salt, ethanolamine salt, triethanolamine salt, dicyclohexylamine salt, N,N'-dibenzylethylenediamine salt, etc.);
an inorganic acid addition salt (e.g. hydrochloride, hydrobromide, hydriodide, sulfate, phosphate, etc.);
an organic carboxylic or sulfonic acid addition salt (e.g. formate, acetate, trifluoroacetate, maleate, tartrate, methanesulfonate, benzenesulfonate, p-toluensulfonate, etc.);
a salt with a basic or acidic amino acid (e.g. arginine, aspartic acid, glutamic acid, etc.); and the like.

In the above and subsequent description of the present specification, suitable examples and illustrations of the various definitions which the present invention includes within the scope thereof are explained in detail as follows.

The term "lower" is intended to mean 1 to 6 carbon atom(s), preferably 1 to 4 carbon atom(s), unless otherwise indicated.

Suitable "lower alkyl" may include a straight or branched alkane residue having 1 to 6 carbon atom(s) such as methyl, ethyl, propyl, isopropyl, butyl, isobutyl, tert-butyl, pentyl, neopentyl, hexyl.

Suitable "heterocyclic group" may include saturated or unsaturated monocyclic or polycyclic heterocyclic group containing at least one hetero-atom such as an oxygen, sulfur, nitrogen atom and the like, such as
unsaturated 3 to 8-membered (more preferable 5 or 6-membered) heteromonocyclic group containing 1 to 4 nitrogen atom(s), for example, pyrrolyl, pyrrolinyl, imidazolyl, pyrazolyl, pyridyl, dihydropyridyl, pyrimidinyl, pyrazinyl, pyridazinyl, triazolyl (e.g. 4H-1,2,4-triazolyl, 1H-1,2,3-triazolyl, 2H-1,2,3-triazolyl, etc.), tetrazolyl (e.g. 1H-tetrazolyl, 2H-tetrazolyl, etc.), 3-triazolthionyl, triazinyl etc.;
saturated 3 to 8-membered (more preferably 5 or 6-membered) heteromonocyclic group containing 1 to 4 nitrogen atom(s), for example, pyrrolidinyl, imidazolidinyl, piperidyl, piperazinyl, etc.;
unsaturated condensed heterocyclic group containing 1 to 4 nitrogen atom(s), for example, indolyl, isoindolyl, indolinyl, indolizinyl, benzimidazolyl, tetrahydrobenzimidazolyl, quinolinyl, isoquinolinyl, tetrahydroisoquinolinyl, indazolyl, benzotriazolyl, quinazolinyl, quinoxalinyl, phthalazinyl, 3H-pyrrolo[1,2-c][1,4]oxadinyl, etc.;
unsaturated 3 to 8-membered (more preferably 5 or 6-membered) heteromonocyclic group containing 1 or 2 sulfur atom(s), for example; thiophenyl, dihydrodithinyl, etc.;
unsaturated heterocyclic group containing 1 or 2 oxygen atom(s) and 1 to 3 nitrogen atom(s), for example, benzothiazolyl, benzothiadiazolyl, etc.;
unsaturated 3 to 8-membered (more preferably 5 or 6-membered) heteromonocyclic group containing 1 or 2 sulfur atom(s) and 1 to 3 nitrogen atom(s), for example, thiazolyl, thiadiazolyl (e.g. 1,2,3-thiadiazolyl, 1,2,4-thiadiazolyl, 1,3,4-thiadiazolyl, 1,2,5-thiadiazolyl, etc.), etc.;
unsaturated condensed heterocyclic group containing 1 or 2 sulfur atom(s) and 1 to 3 nitrogen atom(s), for example, indeno[3,2-d]thiazolyl, etc.;
unsaturated 3 to 8-membered (more preferably 5 or 6-membered) heteromonocyclic group containing 1 or 2 oxygen atom(s) and 1 to 3 nitrogen atom(s), for example, oxazolyl, isoxazoly, etc.;
saturated 3 to 8-membered (more preferably 5 or 6-membered) heteromonocyclic group containing 1 or 2 oxygen atom(s) and 1 to 3 nitrogen atom(s), for example, morpholinyl, etc.;
unsaturated condensed heterocyclic group containing 1 or 2 oxygen atom(s) and 1 to 3 nitrogen atom(s), for example, benzisoxazolyl, etc.;
unsaturated heterocyclic group containing 1 or 2 oxygen atom(s), for example, chromenyl, etc., and the like.

Suitable "condensed phenyl" may include fluorenyl, naphthyl, indolyl, carbazolyl, 2,3-cyclopentenoindolyl, 2,3-dihydrobenzo[b]oxepinyl, 2,3-dihydrobenzo[b]thiepinyl, chromenyl.

Suitable "bivalent heterocyclic group containing nitrogen" may include a saturated or unsaturated monocyclic or policyclic heterocyclic group containing at least one hetero-atom as a nitrogen atom, such as
unsaturated 3 to 8-membered (more preferably 5 or 6-membered) heteromonocyclic group containing 1 to 4 nitrogen atom(s), for example, pyridinylene, pyrimidinylene, etc.;
saturated 3 to 8-membered (more preferably 5 or 6-membered) heteromonocyclic group containing 1 to 4 nitrogen atom(s), for example, piperadinylene, etc.;
unsaturated condensed heterocyclic group containing 1 to 4 nitrogen atom(s), for example, isoindolinylene, etc.

Suitable " lower alkenylene" may include a straight or branched one having 2 to 6 carbon atom(s) such as vinylene, 1-propenylene, isopropenylene, 1-butenylene, 2-butenylene, 1-pentenylene, 1-hexenylene, and the like, in which preferable one having 2 to 4 carbon atoms, and the most preferred one is vinylene or isopropenylene.

Suitable "lower alkylene" may include a straight or branched one having 1 to 6 carbon atom(s) such as methylene, ethylene, trimethylene, propylene, tetramethylene, pentamethylene, hexamethylene, dimethylmethylene, and the like, in which preferable one having 1 to 4 carbon atoms, and the most preferred one is methylene.

Suitable substituent group in the term "heterocyclic group which may have substituents" or "phenyl which may have substituents" may include lower alkyl (can be referred to the ones exemplified above), lower alkoxy (e.g. methoxy, ethoxy, propoxy, butoxy, t-butoxy, pentyloxy, 4-methylpentyloxy, hexyloxy, etc.), halogen (e.g. chloro, bromo, fluoro, iodo), trihalo (lower) alkyl or dihalo (lower) alkyl (lower alkyl moiety and halogen moiety can be referred to the ones exemplified above), heterocyclic group (can be referred to the ones exemplified above), lower alkylamino (e.g. dimethylamino, etc.), lower alkylsulfonylamino (e.g. methylsulfonylamino, etc.), lower alkanoyl (e.g. acetyl, propanoyl, etc.), lower alkanoylamino (e.g. acetylamino, etc.) oxid, oxo, thioxo.

More Suitable substituent groups in the term "heterocyclic group which may have substituents" may include lower alkyl (can be referred to the ones exemplified above, preferably methyl).

More suitable substituents groups in the term "condensed phenyl which may have substituents" or "phenyl which may have substituents" or "thienyl which may have substituents" in R² may include halogen (can be referred to the ones exemplified above, preferably fluoro or chloro), trihalo (lower) alkyl (can be referred to the ones exemplified above, preferably trifluoromethyl), lower alkoxy (can be referred to the ones exemplified above), lower alkyl (can be referred to the ones exemplified above), cyano, thienyl, halothienyl, phenyl, halophenyl, lower alkylation phenyl (e.g. 1-methyl-1-phenylmethyl, etc.), lower alkansulfonyl (can be referred to the ones exemplified above).

Suitable substituents in the term "phenylene which may have substituents" or "bivalent heterocyclic group containing nitrogen which may have suitable substituents " in X may include lower alkyl (can be referred to the ones exemplified above), halogen (can be referred to the ones exemplified above), lower alkoxy (can be referred to the ones exemplified above), trihalo (lower) alkyl (can be referred to the ones exemplified above), heterocyclic group (can be referred to the ones exemplified above), lower alkylsulfonylamino (can be referred to the ones exemplified above), cyano.

The embodiment of the compounds (I) will be explained as follows.

Preferable compounds (I) may be compounds (I) such as
R¹ is imidazolyl, pyridyl, isoquinolinyl, thienyl, phenyl, benzothiazonyl, thiazolyl, pyrazolyl, oxazolyl, chromenyl, benzimidazonyl, indolyl, isoquinolyl, piperazinyl, triazolyl, pyrimidinyl, pyrazinyl, isoxazolyl, triazinyl, pyridazinyl, thiaziazolyl, benzimidazolyl, benzisoxazolyl,
R² is fluorenyl, naphthyl, indolyl, carbazolyl, 2,3-cyclopentenoindolyl, 2,3-dihydrobenzo[b]oxepinyl, 2,3-dihydro-benzo[b]thiepinyl, chromenyl
A is a group of the formula:

   - (CH₂)ₜ-(O)ₘ-

   or 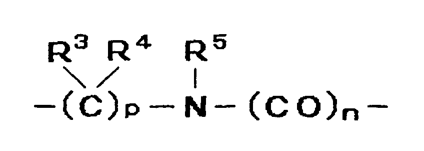 in which R³ and R⁴ are each hydrogen or linked together to form imino,
   R⁵ is hydrogen,
   t is 0,1 or 2,
   p, m and n are each 0 or 1,
X is pyridinylene, phenylene, pyrimidinylene, piperazinylene, isoindolynylene, and,
Y is vinylene, 1-propenylene, methylene or ethylene,
and a salt thereof.

More preferable compound such as
R¹ is phenyl, unsaturated heteromonocyclic group containing 1 to 4 nitrogen atom(s) which have 1 to 3 lower alkyl,
R² is phenyl which have trihalo (lower) alkyl, indolyl which have 1 to 3 lower alkyl, calbazolyl,
A is a bond or a group of the formula: 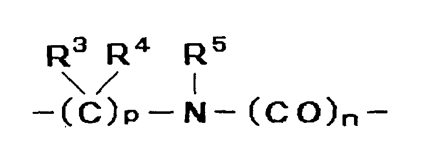 in which R³ and R⁴ are linked together to form imino,
   R⁵ is hydrogen,
   p is 1, n is 0,
X is phenylene which have 1 to 3 halogen and lower alkoxy,
   and
Y is bond or vinylene
and a salt thereof.

The processes 1 to 12 for preparing the object compounds (I) of present invention are explained in detail in the following.

### Process 1

The object compound (I) or a salt thereof can be prepared by reacting the compound (II) or a salt thereof with the compound (III) or a salt thereof, and by subjecting them to amidation reaction.

This reaction is usually carried out in a conventional organic solvent which does not adversely influence the reaction such as water, methanol, ethanol, propanol, diethyl ether, dioxane, tetrahydrofuran, N,N-dimethylformamide, dimethylacetamide, acetone, acetonitrile, chloroform, methylene chloride, ethylene chloride, ethyl acetate, pyridine, triethylamine, benzene, or a mixture thereof.

The reaction may also be carried out in the presence of an inorganic or organic base such as an alkali metal carbonate, alkali metal bicarbonate, tri(lower)alkylamine [e.g. triethylamine, etc.], pyridine, dimethylaminopyridine, N-(lower)alkylmorpholine, N,N-di(lower)alkylbenzylamine, or the like.

The reaction may also be carried out in the presence of a conventional condensing agent such as N,N'-dicyclohexylcarbodiimide; N-cyclohexyl-N'-morpholinoethylcarbodiimide; N-cyclohexyl-N'-(4-di-ethylaminocyclohexl) carbodiimide; N,N'-diethylcarbodiimide, N,N'-diisopropylcarbodiimide; N-ethyl-N'-(3-dimethylaminopropyl)carbodiimide, 1-ethyl-3-(3-dimethylaminopropyl) carbodiimide, N,N'-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; diphenyl phosphorylazide; thionyl chloride; oxalyl chloride; lower alkyl haloformate [e.g. ethyl chloroformate, isopropyl chloroformate, etc.]; triphenylphosphine; 2-ethyl-7-hydroxybenzisoxazolium salt; 2-ethyl-5-(m-sulfophenyl) isoxazolium hydroxide intramolecular salt; benzotriazol-1-yl-oxy-tris-(dimethylamino) phosphoniumhexafluorophosphate; 1-(p- chlorobenzenesulfonyloxy)-6-chloro-1H-benzotriazole; so-called Vilsmeier reagent prepared by the reaction of N,N-dimethylformamide with thionyl chloride, phosgene, trichloromethyl chloroformate, phosphorus oxychloride, etc.; or the like.

The reaction temperature is not critical and the reaction is usually carried out at ambient temperature or under heating.

The reaction of this method can be carried out by a conventional manner or the manners of Examples mentioned below.

### Process 2

The object compound (Ia) or a salt thereof can be prepared by reacting the compound (IV) or a salt thereof with the compound (V) or a salt thereof, and by subjecting them to condensation reaction.

This reaction is usually carried out in a conventional organic solvent which does not adversely influence the reaction such as water, methanol, ethanol, propanol, isopropyl alcohol, diethyl ether, dioxane, tetrahydrofuran, N,N-dimethylformamide, acetone, acetonitrile, chloroform, methylene chloride, ethylene chloride, ethyl acetate, pyridine, triethylamine, benzene, or a mixture thereof.

The reaction may also be carried out in the presence of an organic or inorganic acid such as formic acid, acetic acid, propionic acid, trichloroacetic acid, trifluoroacetic acid, methanesulfonic acid, hydrochloric acid, hydroiodic acid, hydrobromic acid, sulfuric acid, or the like.

The reaction temperature is not critical and the reaction is usually carried out at ambient temperature or under heating.

The reaction of this method can be carried out by a conventional manner or the manners of Examples mentioned below.

### Process 3

The object compound (Ib) or a salt thereof can be prepared by reacting the compound (VI) or a salt thereof with the compound (VII) or a salt thereof, and by subjecting them to condensation reaction.

The reaction is usually carried out in a conventional organic solvent which does not adversely influence the reaction such as water, methanol, ethanol, propanol, diethyl ether, dioxane, tetrahydrofuran, N,N-dimethylformamide, acetone, acetonitrile, chloroform, methylene chloride, ethylene chloride, ethyl acetate, pyridine, triethylamine, benzene, or a mixture thereof.

### Process 4

The object compound (I) or a salt thereof can be prepared by reacting the compound (II) or a salt thereof with the compound (VIII) or a salt thereof, and by subjecting them to amidation reaction.

This method can be carried out in substantially the same manner as Process 1, and therefore the reaction method and reaction conditions (e.g. solvent, reaction temperature, etc.) are to be referred to those as explained in Process 1.

### Process 5

The object compound (Ic) or a salt thereof can be prepared by reacting the compound (VI) or a salt thereof with the compound (IX) or a salt thereof.

This method can be carried out by a conventional manner or the manner of Example 122 mentioned below.

### Process 6

The object compound (Id) or a salt thereof can be prepared by reacting the compound (X) or a salt thereof with the compound (XI) or a salt thereof.

This method can be carried out by a conventional manner or the manner of Example 92 mentioned below.

### Process 7

The object compound (If) or a salt thereof can be prepared by hydrogenating the compound (Ie) or a salt thereof.

This method can be carried out by a conventional manner or the manner of Example 100 mentioned below.

### Process 8

The object compound (Ih) or a salt thereof can be prepared by hydrogenating the compound (Ig) or a salt thereof.

This method can be carried out by a conventional manner or the manner of Example 108 mentioned below.

### Process 9

The object compound (Ij) or a salt thereof can be prepared by hydrogenating the compound (Ii) or a salt thereof.

This method can be carried out by a conventional manner or the manner of Example 194 mentioned below.

### Process 10

The object compound (Ik) or a salt thereof can be prepared by reacting the compound (XII) or a salt thereof with the compound (XIII) or a salt thereof.

This method can be carried out by a conventional manner or the manner of Example 99 mentioned below.

### Process 11

The object compound (II) or a salt thereof can be prepared by reacting the compound (XIV) or a salt thereof with the compound (XV) or a salt thereof.

This method can be carried out by a conventional manner or the manner of Example 209 mentioned below.

### Process 12

The object compound (Im) or a salt thereof can be prepared by reacting the compound (Il) or a salt thereof with the sodium nitrite.

This method can be carried out by a conventional manner or the manner of Example 210 mentioned below.

The reaction may also be carried out in the presence of inorganic or organic acid such as formic acid, acetic acid, propionic acid, trichloroacetic acid, trifluoroacetic acid, methanesulfonic acid, hydrochloric acid, hydroiodic acid, hydrobromic acid, sulfuric acid, or the like.

The reaction temperature is not critical, and the reaction is usually carried out at ambient temperature or under heating.

The reaction of this method can be carried out by a conventional manner or the manners of Examples mentioned below.

The object compound (I) of the present invention can be isolated and purified in a conventional manner, for example, extraction, precipitation, fractional crystallization, recrystallization, column chromatography, and the like.

The object compound (I) thus obtained can be converted to its salt by a conventional method.

The object compound (I) and a salt thereof may include a solvate [e.g., enclosure compound (e.g., hydrate, etc.)].

The object compound (I) of the present invention exhibit pharmacological activities such as 5-HT antagonism, especially, 5-HT_{2c} antagonism, and the like and therefore are useful as 5-HT antagonist for treating or preventing central nervous system (CNS) disorders such as anxiety, depression, obsessive compulsive disorders, migraine, anorexia, Alzheimer's disease, sleep disorders, bulimia, panic attacks; withdrawal from drug abuse such as cocain, ethanol, nicotine, and benzodiazepines; schizophrenia; and also disorders associated with spinal trauma and/or head injury such as hydrocephalus, and the like.

In order to illustrate the usefulness of the object compounds (I) of the present invention, pharmacological activity of the representative compounds of the present invention are shown below.

### Test Method

### [³H]-mesulergine binding

The affinity of test drugs for the 5-HT_{2c} binding site can be determined by assessing their ability to displace [³H]-mesulergine in the rat prefrontal cortex. The method employed was similar to that of Pazos et al, 1984.

The membrane suspension (500 µl) was incubated with [³H]-mesulergine (1nM) in Tris hydrochloric acid buffer solution (pH 7.4) containing calcium chloride 4mM and ascorbic acid 0.1% at 37°C for 30 minutes. Non-specific binding was measured in the presence of mianserin (1µM). 30nM spiperone was used to prevent binding to 5-HT_{2A} sites. Test drugs (10⁻⁶M) were added in a volume of 100 µl. The total assay volume was 1000µl. Incubation was stopped by rapid filtration using a Brandel cell harvester and radioactivity measured by scintillation counting.

### Test compound

### (a) (3E)-N-[3-(imidazol-1-yl)phenyl]-4-phenyl-3-butenamide

### Test Result

An examination result is shown in Table 1.

**Table 1**

| Compound | Inhibition (%) |
|---|---|
| (a) | 82 |

For therapeutic or preventive administration, the compound (I) of the present invention and a salt thereof can be used in the form of the conventional pharmaceutical preparation which contains said compounds as an active ingredient, in admixture with a conventional pharmaceutically acceptable carrier such as an organic or inorganic solid or liquid excipient suitable for oral, parenteral and external administration. The pharmaceutical preparations may be in solid form such as tablet, granule, powder, capsule, or liquid form such as solution, suspension, syrup, emulsion, lemonade, and the like.

If needed, there may be included in the above preparation auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, ethylene glycol, and the like.

While the dosage of the compound (I) may vary from and also depend upon the age and conditions of the patient, a kind of diseases, a kind of the compounds (I) to be applied, etc. In general amounts between 0.01mg and about 500mg per day may be administered to a patient. An average single dose of about 0.05mg, 0.1mg, 0.25mg, 0.5mg, 1mg, 20mg, 50mg, 100mg of the object compound (I) of the present invention may be used in treating diseases.

The following Preparations and Examples are given for the purpose of illustrating the present invention in more detail.

### Preparation 1

A mixture of 3-nitroaniline, phenylacetic acid, 4-dimethylaminopyridine, 1-[3-(dimethylaminopropyl)-3-ethylcarbodiimide hydrochloride and dichloromethane was stirred overnight at room temperature. Diluted with ethyl acetate, washed in turn with brine, diluted hydrochloric acid (three times), water, diluted aqueous solution of sodium hydroxide (three times), and brine, and dried over magnesium sulfate, filtered, and evaporated. Recrystallized from ethyl acetate to give N-(3-nitrophenyl) phenylacetamide.
mp:133-134°C
IR(KBr):3242,1657cm⁻¹
MS:257(M+1)
NMR(DMSO, δ ):3.70(2H,s), 7.20-7.38(5H,m), 7.60(1H,dd,J=8.2Hz,8.2Hz), 7.88-7.95 (2H,m), 8.63(1H,dd,J=2.1Hz,2.1Hz), 10.66(1H,br s)

### Preparation 2

A mixture of N-(3-nitrophenyl)phenylacetamide, iron powder, ammonium chloride, ethanol, and water was heated at reflux for 1 hour. After cooling to ambient temperature, filtered through Celite, washed with methanol and evaporated. Dissolved into dichloromethane and diluted aqueous solution of sodium hydroxide. Separated, and extracted with dichloromethane (twice). Combined organic layers were dried with magnesium sulfate, evaporated, then purified by silica gel column chromatography (dichloromethane/methanol/ammonia) and then recrystalized with methanol to give N-(3-aminophenyl)phenylacetamide.
mp:141-142°C
IR(KBr):1678cm⁻¹
MS:227(M+1)
NMR(DMSO, δ ):3.58(2H,s), 5.03(2H,s), 6.23(1H,d,J=7.8Hz), 6.68(1H,d,J=7.7Hz), 6.80-6.96(2H,m), 7.18-7.42(5H,m), 9.82(1H,s)
Elemental analysis:
Calculated+(H₂O)_{1/5}:C;73,15,H;6.31,N;12.19
Found:C;73.37,H;6.17,N;12.26

### Preparation 3

This was prepared in a manner similar to Preparation 1 to give N-(3-nitrophenyl) cinnamamide from 3-nitroaniline and cinnamic acid.
mp:162-164°C
IR(KBr):3354,1687,1627,1550cm⁻¹
MS:269(M+1)
NMR(DMSO, δ ):6.82(1H,d,J=15.8Hz), 7.40-7.58(3H,m), 7.60-7.71(4H,m), 7.91-8.03 (2H,m), 8.76(1H,dd,J=2.1Hz,2.1Hz), 10.72(1H,br s)

### Preparation 4

This was prepared in a manner similar to Preparation 2 to give N-(3-aminophenyl)cinnamamide.
MS:239(M+1)
NMR(DMSO, δ ):5.09(2H,br s), 6.28(1H,d,J=7Hz), 6.77-7.04(4H,m), 7.35-7.68(6H,m), 9.87(1H,s)

### Preparation 5

A mixture of 1-fluoro-3-iodo-5-nitrobenzene (2.45g), 1,2-dimethylimidazole (1.77g), potassium carbonate (2.54g), palladium (II) acetate (103mg), and N,N-dimethylformamide (60ml) were stirred at 140°C for 14 hours. After cooling, the reaction mixture was poured into water (100ml) and extracted with ethyl acetate (twice, 100ml each time). The combined extracts were washed with water (twice, 200ml each time) and brine (100ml). The separated organic layer was dried over magnesium sulfate, decolored (activated carbon), and filtered. The solvent was evaporated to give a residue which was purified by silica gel chromatography (dichloromethane-methyl alcohol) to give 5-(3-fluoro-5-nitrophenyl)-1, 2-dimethyl-1H-imidazole (1.01g) as yellow crystals.
MS:236(M+1)
NMR(DMSO, δ ):2.37(3H,s), 3.62(3H,s), 7.17(1H,s), 7.8-7.9(1H,m), 8.0-8.2(2H,m)

### Preparation 6

To a solution of 5-(3-fluoro-5-nitrophenyl)-1,2-dimethyl-1H-imidazole (400mg) in methyl alcohol (5ml) was added sodium methoxide (28% solution in methanol (1.64g)). The mixture was heated at reflux for 44 hours. After cooling, the reaction mixture was poured into water (30ml) and extracted with ethyl acetate (30ml) and dichloromethane (30ml). The separated organic layer was evaporated to give a residue which was purified by silica gel chromatography (dichloromethane-methyl alcohol) to give 3-(1,2-dimethyl-1H-imidazol-5-yl)-5-nitrophenyl methyl ether (220mg) as yellow crystals.
MS:248(M+1)
NMR(DMSO, δ ):2.37(3H,s), 3.59(3H,s), 3.93(3H,s), 7.08(1H,s), 7.4-7.5(1H,m), 7.6-7.7(1H,m), 7.7-7.9(1H,m)

### Preparation 7

To a mixture of 3-(1,2-dimethyl-1H-imidazol-5-yl)-5-nitrophenyl methyl ether (208mg), activated carbon (312mg), and tetrahydrofuran (3.1ml) were added iron (II) chloride hexahydrate (21mg) and hydrazine monohydrate (0.31ml). The mixture was heated at 80°C for 1 hour. After cooling, the reaction mixture was evaporated. The resultant residue was diluted with ethyl acetate (40ml) and washed with water (twice, 30ml each time) and brine (20ml). The separated organic layer was dried over magnesium sulfate and filtered. The solvent was evaporated to give 3-(1,2-dimethyl-1H-imidazol-5-yl)-5-methoxyaniline (186mg) as crystals.
MS:218(M+1)
NMR(DMSO, δ ):2.32(3H,s), 3.49(3H,s), 3.68(3H,s), 5.20(2H,br s), 6.0-6.3(3H,m), 6.74(1H,s)

### Preparation 8

Trifluoroacetic acid (8.0g) was added to 2-bromo-6-chloro-4-nitroaniline (10.0g) in N,N-dimethylformamide (80ml), followed by sodium nitrite (2.88g) in water (5ml) and the pale brown suspension was stirred at ambient temperature for 1 hour. To the mixture was added triethylamine (12.1g) and the deep red effervescing mixture was stirred for a further 1 hour before being partitioned between ethyl acetate (400ml) and water (300ml). The separated organic layer was washed with 1N-hydrochloric acid (twice, 200ml each time), water (200ml), and saturated brine (100ml), and then dried over magnesium sulfate and filtered. The solvent was evaporated to give a crude which was purified by silica gel chromatography, and eluted with ethyl acetate-hexane to give 3-bromo-5-chloronitrobenzene (2.5g) as pale yellow crystals.
NMR(DMSO, δ ):8.29(1H,dd,J=1.8Hz,1.8Hz), 8.32(1H,dd,J=2.0Hz,1.9Hz), 8.38(1H, dd,J=1.9Hz,1.8Hz)

### Preparation 9

Palladium(II) acetate (119mg) was added to a mixture of 3-bromo-5-chloronitrobenzene (2.5g), 1,2-dimethylimidazole (2.03g) and potassium carbonate (2.92g) in N,N-dimethylformamide (50ml) at ambient temperature, and the brownish suspension was stirred at 140°C for 3 hours. After cooled to room temperature, the reaction mixture was partitioned between water (200ml) and ethyl acetate (twice, 100ml each time). The combined organic layers were washed with water (twice, 100ml each time) and saturated brine (100ml), and then dried over magnesium sulfate and filtered. Evaporation gave a residue which was triturated with ethyl acetate-hexane to give 5-(3-chloro-5-nitrophenyl)-1,2-dimethyl-lH-imidazole (1.53g) as yellow crystals.
MS:252(M+1)
NMR(DMSO, δ ):2.37(3H,s), 3.61(3H,s), 7.17(1H,s), 8.00(1H,dd,J=1.7Hz,1.7Hz), 8.17(1H,dd,J=1.9Hz,1.7Hz), 8.22(1H,dd,J=2.0Hz,2.0Hz)

### Preparation 10

To a solution of 5-(3-chloro-5-nitrophenyl)-1,2-dimethyl-1H-imidazole (13.6g) in a mixture of ethanol (272ml) and water (136ml) were added ammonium chloride (1.45g), Celite (27g), and iron powder (9.05g) with stirring at 70°C. The stirring was continued at reflux for 2 hours. The reaction mixture was cooled, diluted with ethyl acetate (500ml), filtered through Celite and evaporated. The resultant residue was dissolved in 1N-hydrochloric acid (200ml), washed with dichloromethane (3 times, 100ml each time), alkalized with 1N-sodium hydroxide and extracted with dichloromethane (3 times, 100ml each time). The combined organic layers were dried over magnesium sulfate and filtered. Evaporation gave a residue which was triturated with ethyl acetate-ether to give 3-chloro-5-(1,2-dimethyl-lH-imidazol-5-yl)aniline (4.8g) as pale yellow crystals..
MS:222(M+1)
NMR(DMSO, δ ):2.32(3H,s), 3.50(3H,s), 5.53(2H,br s), 6.5-6.6(3H,m), 6.81(1H,s)

### Preparation 11

To a mixture of 3-nitrobenzaldehyde (302mg), 4-amino-2-chloropyridine (308mg), and N,N-dimethylformamide (10ml) was added dropwise titanium (IV) isopropoxide (852mg) at ambient temperature with stirring. After stirring for 27 hours, the reaction mixture was added sodium triacetoxyborohydride (2.12g) and the stirring was continued for 7 days. The reaction mixture was diluted with ethyl acetate (20ml), cooled with 1N-sodium hydroxide (2.1ml), and stirred for 30 minutes. The resultant precipitate was filtered off and the filtrate was washed with water (3 times, 20ml each time). The separated organic layer was dried over magnesium sulfate and filtered. The solvent was evaporated to give a residue which was triturated with ethyl acetate-hexane to give N-(3-nitrobenzyl)-N-(2-chloro-4-pyridyl) amine (383mg) as crystals.
MS:264(M+1)
NMR(DMSO, δ ):4.52(2H,d,J=6.2Hz), 6.4-6.7(2H,m), 7.5-7.9(4H,m), 8.1-8.3(2H,m)

### Preparation 12

To a mixture of N-(3-nitrobenzyl)-N-(2-chloro-4-pyridyl)amine (264mg), ammonium chloride (27mg), ethyl alcohol (5ml), tetrahydrofuran (2.5ml), and water (2.5ml) were added Celite (270mg) and iron powder (168mg) with stirring at 70°C.

The stirring was continued at reflux for 2 hours. The reaction mixture was cooled, diluted with ethyl acetate (20ml), and filtered through Celite. The filtrate was washed with water (twice, 30ml each time) and brine (20ml). The separated organic layer was dried over magnesium sulfate and filtered. The solvent was evaporated to give N-(3-aminobenzyl)-N-(2-chloro-4-pyridyl)amine (250mg).
MS:234(M+1)
NMR(DMSO, δ ):4.16(2H,d,J=5.8Hz), 5.06(2H,br s), 6.3-6.6(5H,m), 6.97(1H,t, J=7.6Hz), 7.39(1H,t,J=5.8Hz), 7.79(1H,d,J=5.8Hz)

### Preparation 13

To a suspension of 5-fluoro-2-methoxybenzenecarbothioamide (4.04g) in acetone (22ml) was added methyl iodide (2.71ml) at room temperature. Stirred overnight. Next day added isopropyl ether(22 ml), collected, and washed with isopropyl ether (3 times, 5ml each time) to give methyl-5-fluoro-2-methoxybenzenecarbimidothioate hydroiodide (6.62g) as rusty orange crystals.
mp:122-123°C
MS:200(free+1)
NMR(DMSO, δ ):2.70(3H,s), 3.37(2H,br s), 3.86(3H,s), 7.29(1H,dd,J=9.2Hz,4.3Hz), 7.43-7.53(2H,m)

### Preparation 14

To N-[3-[(tertbutoxycarbonyl)amino]phenyl]-2-methoxycinnamide *(0.75g)* was added 4N hydrochloric acid (10ml) in ethyl acetate. After one hour at room temperature was added ethyl acetate (100ml) and IN sodium hydroxide (50ml). The separated organic layer was washed with diluted aqueous sodium hydroxide (twice), brine, dried over magnesium sulfate, filtered, evaporated, and recystallized from ethyl acetate/diethyl ether to give N-(3-aminophenyl)-2-methoxycinnamamide (339mg) as yellow crystals.
mp:137-139°C
MS:269(M+1)
NMR(DMSO, δ ):3.88(3H,s), 5.08(2H,s), 6.27(1H,d,J=8.1Hz), 6.75-7.14(6H,m), 7.39 (1H,dd,J=7.1Hz,7.1Hz), 7.55(1H,d,J=7.6Hz), 7.76(1H,d,J=15.8Hz), 9.84(1H,s)

### Preparation 15

A mixture of N-(3-cyanophenyl)-2-chlorocinnamamide (10.19g), thioacetamide (13.5g), dioxane (18ml), and 4N HCI (18ml) in dioxane was heated at 100°C for 30 minutes. Cooled, poured into ice/water (400ml), allowed to stand for 30 minutes. Collected by filtration, washed with water. Slurried in dichloromethane (500ml) and collected, and then slurried in methanol (10ml), collected, and washed with methanol (twice, 5ml each time) to give N-[3-(aminocarbothioyl)phenyl]-2-chlorocinnamamide (8.13g) as light brown crystals.
mp:195-197°C
MS:339(M+Na)
NMR(DMSO, δ ):6.91(1H,d,J=15.6Hz), 7.35-7.60(5H,m), 7.73-7.95(3H,m), 8.19 (1H,s), 9.52(1H,s), 9.90(1H,s), 10,50(1H,s)
Elemental analysis:
Calculated+(H₂O)_{0.4}:C;59.31,H;4.29,N;8.65
Found:C;59.37,H;4.14,N;8.66

### Preparation 16

To a suspension of tert-butyl 3-aminophenylcarbamate (416mg), 2-chlorocinnamic acid (365mg), 4-dimethylaminopyridine (12mg) and dichloromethane (5ml) was added at room temperature 1-[3-(dimethylamino) propyl]-3-ethylcarbodiimide hydrochloride (575mg). Stirred for 2 days. Diluted with ethyl acetate (50ml), washed with 0.1N hydrochloric acid (3 times, 25ml each time), water, saturated aqueous sodium hydrogen carbonate (3 times, 25ml each time), brine, dried over magnesium sulfate, filtered, and evaporated to give N-[3-[(tertbutoxycarbonyl)amino]phenyl]-2-chlorocinnamamide (0.77g) as a white foam.
MS:317(M-C₄H₈+1)
NMR(DMSO, δ ):1.48(9H,s), 6.91(1H,d,J=15.6Hz), 7:07(1H,d,J=7.7Hz), 7.19(1H,dd, J=8.1Hz,8.1Hz), 7.40-7.65(4H,m), 7.71-7.77(1H,m), 7.85(1H,d,J=15.6Hz), 7.86-7.89 (1H,m), 9.40(1H,s), 10.27(1H,s).

### Preparation 17

This was prepared in a manner similar to Preparation 14 to give N-(5-amino-2-fluorophenyl)-2-chlorocinnamamide.
mp:184-185°C
MS:291(M+1)
NMR(DMSO, δ ):5.00(2H,s), 6.24-6.34(1H,m), 6.91(1H,dd,J=11.0Hz,8.7Hz), 7.12(1H, d,J=15.6Hz)7.30-7.40(1H,m), 7.41-7.50(2H,m), 7.52-7.59(1H,m), 7.72-7.79(1H,m), 7.85(1H,d,J=15.7Hz), 9.76(1H,s)
Elemental analysis:
Calculated+(H₂O)_{0.2}:C;61.21,H;4.25,N;9.52
Found:C;61.13,H;3.99,N;9.38

### Preparation 18

This was prepared in a manner similar to Preparation 16 to give N-[5-[(tertbutoxycarbonyl)ammo]-2-fluorophenyl]-2-chlorocinnamamide.
mp:203-205°C
MS:335(M-C4H8+1), 291(M-BOC+1)
NMR(DMSO, δ ):1.48(9H,s), 7.04-7.21(3H,m), 7.40-7.49(2H,m), 7.53-7.60(1H,m), 7.73-7.80(1H,m), 7.88(1H,d,J=15.7Hz) 8.25(1H,d,J=7.7Hz), 9.41(1H,s), 9.99(1H,s)
Elemental analysis:
Calculated+(H₂O)_{0.2}:C;60.90,H;5.21,N;7.10
Found:C;60.87,H;5.01,N;6.98

### Preparation 19

This was prepared in a manner similar to Preparation 4 to give tert-butyl 3-amino-4-fluorophenylcarbamate.
MS:127(M-BOC+1)
NMR(DMSO, δ ):1.45(9H,s), 5.00(2H,s), 6.60-7.20(3H,m), 9.01(1H,s)
Elemental analysis:
Calculated+(H₂O)_{0.5}:C;56.16,H;6.85,N;11.91
Found:C;56.29,H;6.52,N;11.83

### Preparation 20

This was prepared in a manner similar to Preparation 4 to give 4-(1,3-thiazol-4-yl)aniline.
mp:93-95°C
MS:177(M+1)
NMR(DMSO, δ ):5.28(2H,br s), 6.56-6.64(2H,m), 7.62-7.68(2H,m), 7.73(1H,d,J =1.9Hz), 9.08(1H,d,J=1.9Hz)
Elemental analysis:
Calculated:C;61.34,H;4.58,N;15.89
Found:C;61.29,H;4.53,N;15.89

### Preparation 21

A mixture of phosphorus pentasulfide (6.66g), dioxane (75ml), and N,N-dimethylformamide (30ml) was heated at gentle reflux for one hour under nitrogen atmosphere. Cooled and decanted from the yellow foam. One third of this solution was then added to 2-bromo-1-(4-nitrophenyl) ethanone (1.22g) and the resulting solution was heated at reflux for one hour. Cooled to room temperature, added water (300ml), neutralized to basic with magnesium carbonate (5g). After one hour collected by filtration, washed with water (3 times) to give 4-(4-nitrophenyl)-1,3-thiazole (1.03g) as a brown solid.
MS:207(M+1)
NMR(DMSO, δ ):8.23-8.36(4H,m), 8.54(1H,d,J=1.8Hz), 9.29(1H,d,J=1.8Hz)

### Preparation 22

This was prepared in a manner similar to Preparation 4 to give 4-(1,3-oxazol-5-yl)aniline.
MS:161(M+1)
NMR(DMSO, δ ):5.45(2H,br s), 6.58-6.66(2H,m), 7.30(1H,s), 7.34-7.39(2H,m), 8.25 (1H,s)
Elemental analysis:
Calculated+(H₂O)_{0.25}:C;65.64,H;5.20,N;17.01
Found:C;65.53,H;4.97,N;16.85

### Preparation 23

This was prepared in a manner similar to Preparation 4 to give 4-(1H-pyrazol-1-yl)aniline.
MS:160(M+1)
NMR(DMSO, δ ):5.20(2H,s), 6.42(1H,dd,J=2.0Hz,2.0Hz), 6.58-6.67(2H,m), 7.37-7.46 (2H,m), 7.60(1H,d,J=1.5Hz), 8.18(1H,d,J=2.4Hz)

### Preparation 24

A mixture of pyrazole (0.82g), 4-fluoro-1-nitrobenzene (1.41g), potassium carbonate (1.66g) and N,N-dimethylformamide (20ml) was heated at 110°C for 4 hours. Cooled, poured into water (100ml). After one hour collected by filtration, washed with water to give 1-(4-nitrophenyl)-1H-pyrazole (2.68g) as pale yellow crystals.
MS:190(M+1)
NMR(DMSO, δ ):6.67(1H,dd,J=2.6Hz,1.8Hz), 7.89(1H,d,J=1.6Hz), 8.08-8.18(2H,m), 8.32-8.42(2H,m), 8.74(1H,d,J=2.6Hz)

### Preparation 25

A solution of di-tert-butyl dicarbonate(7.86g), 4-fluoro-3-nitroaniline(4.68g), and tetrahydrofuran (60ml) was heated at reflux for three days. Evaporated. Purified by silica gel column chromatography (hexane/ethyl acetate) to give tert-butyl 4-fluoro-3-nitrophenylcarbamate (9.08g) as orange crystals.
MS:157(M-BOC+1)
NMR(CDCl₃, δ ):1.53(9H,s), 6.62(1H,br s), 7.21(1H,dd,J=9.0Hz,9.0Hz), 7.61(1H, ddd,J=9.0Hz,3.1Hz,3.1Hz), 8.14(1H,dd,J=6.4Hz,2.8Hz)

### Preparation 26

A mixture of 2-(4-nitrophenyl)-2-oxoethylformamide (0.64g), toluene (10ml) and phosphorus oxychloride (2ml) was heated at reflux for one hour. Cooled and carefully added to ice/water (100ml), added dichloromethane (200ml), then neutralized with potassium carbonate. Separated, extracted with dichloromethane (twice). Combined organic layers were dried over magnesium sulfate, filtered, and evaporated. Purified by silica gel column chromatography, eluted with dichloromethane-methanol to give 5-(4-nitrophenyl)-1,3-oxazole (0.50g) as brown crystals.
MS:191(M+1)
NMR(DMSO, δ ):8.01(2H,d,J=9.0Hz), 8.03(1H,s), 8.34(2H,d,J=9.0Hz), 8.62(1H,s)

### Preparation 27

To a suspension of formyl[2-(4-nitrophenyl)-2-oxoethyl]formamide (0.80g) in methanol (15ml) was added one drop of triethylamine. Stirred for one hour at room temperature. Evaporated and recrystallized from ethyl acetate to give 2-(4-nitrophenyl)-2-oxoethylformamide (0.66g) as brown crystals.
MS:209(M+1)
NMR(CDCl₃, δ ):4.86(2H,dd,J=4.6Hz,0.8Hz), 6.62(1H,br s), 8.11-8.20(2H,m), 8.33-8.41(3H,m)

### Preparation 28

To a solution of 2-bromo-1-(4-nitrophenyl)ethanone (2.44g) in N,N-dimethylformamide (10ml) was added at room temperature diformylimide sodium salt (0.95g). Stirred 1 hour. Poured into water (100 ml), added ethyl acetate (100ml), separated, washed with water (twice), brine, dried over magnesium sulfate, filtered, and evaporated. Recrystallized from ethyl acetate (5ml) to give formyl[2-(4-nitrophenyl)-2-oxoethyl]formamide (0.83g) as tan crystals.
MS:237(M+1)
NMR(CDCl₃, δ ):5.12(2H,s), 8.14(2H,d,J=8.9Hz), 8.38(2H,d,J=8.9Hz), 9.05(2H,s)

### Preparation 29

This was prepared in a manner similar to Preparation 4 to give 4-(1-methyl-1H-imidazol-4-yl)aniline.
mp:168-179°C
MS:174(M+1)
NMR(DMSO, δ ):3.63(3H,s), 4.98(2H,br s), 6.52(2H,d,J=8.4Hz), 7.26(1H,d,J=1.2Hz), 7.38(2H,d,J=8.4Hz), 7.49(1H,d,J=1.2Hz)
Elemental analysis:
Calculated+(H₂O)_{0.15}:C;68.28,H;6.47,N;23.89
Found:C;68.24,H;6.35,N;24.02

### Preparation 30

To a solution of 4-(4-nitrophenyl)-1H-imidazole (2.34g) in N,N-dimethylformamide (25ml) was added potassium carbonate (1.71g) and then methyl iodide (1.56ml). Stirred at room temperature for 4 hours. Poured into water/ice (300ml). After 30 minutes collected by filtration, washed with water, and air dried to give 1-methyl-4-(4-nitrophenyl)-1H-imidazole as brown crystals.
mp:185-187°C
MS:204(M+1)
NMR(DMSO, δ ):3.72(3H,s), 7.76(1H,d,J=1.1Hz), 7.92(1H,d,J=1.1Hz), 7.95-7.99(2H,m), 8.19-8.25(2H,m)
Elemental analysis:
Calculated+(H₂O)_{0.25}:C;57.83,H;4.61,N;20.23
Found:C;57.83,H;4.34,N;20.26

### Preparation 31

A solution of 2-bromo-1-(4-nitrophenyl)ethanone (4.88g) and formamide (20ml) was placed in a 190°C oil bath for one hour. Cooled, poured into water/ice (200ml), added 3N hydrochloric acid (20ml). After 30 minutes, filterd off the insolubles. To the clear orange filtrate was added enough sodium hydroxide to make strongly basic (about 3.0g). After 30 minutes, collected by filtration, washed with water; dried at 60°C under reduced pressure to give 4-(4-nitrophenyl)-1H-imidazole (2.34g) as a brown solid.
MS:190(M+1)
NMR(DMSO, δ ):7.83(1H,s), 7.95(1H,s), 8.03(2H,d,J=8.9Hz), 8.22(2H,d,J=8.9Hz), 12.47(1H,br s)

### Preparation 32

This was prepared in a manner similar to Preparation 13 to give 5-chloro-2-methoxybenzenecarbimidothioate hydroiodide.
mp:162-163°C
MS:216(free+1)
NMR(DMSO, δ ):2.73(3H,s), 3.38(2H,br s), 3.88(3H,s), 7.32(1H,d,J=9.0Hz), 7.63(1H, d,J=2.6Hz), 7.72(1H,dd,J=9.0Hz,2.7Hz)
Elemental analysis:
Calculated+(EtOAc)_{0.1}:C;32.03,H;3.37,N;3.97
Found:C;31.96,H;3.17,N;4.30

### Preparation 33

This was prepared in a manner similar to Preparation 13 to give methyl 3-(2-chlorocinnamoylamino)benzenecarbimidothioate hydroiodide.
mp:208-210°C
MS:331(free+1)
NMR(DMSO, δ ):2.81(3H,s), 3.44(2H,s), 6.90(1H,d,J=15.6Hz), 7.41-7.69(5H,m), 7.76-7.83(1H,m), 7.86-7.99(2H,m), 8.41(1H,s), 10.72(1H,s)
Elemental analysis:
Calculated:C;44.51,H;3.51,N;6.11
Found:C;44.88,H;3.50,N;6.11

### Preparation 34

This was prepared in a manner similar to Preparation 15 to give 5-chloro-2-methoxybenzenecarbothioamide.
mp:145-146°C
MS:202(M+1)
NMR(DMSO, δ ):3.81(3H,s), 7.09(1H,d,J=8.9Hz), 7.42(1H,dd,J=8.9Hz,2.8Hz), 7.60 (1H,d,J=2.8Hz), 9.42(1H,s), 10.09(1H,s)
Elemental analysis:
Calculated:C;47.65,H;4.00,N;6.95
Found:C;47.48,H;3.87,N;6.80

### Preparation 35

This was prepared in a manner similar to Preparation 16 to give N-(3-amino-5-chlorophenyl)-2-trifluoromethylcinnamamide.
mp:167-170°C
MS:341(M+1)
NMR(DMSO, δ ):5.49(2H,s), 6.33(1H,dd,J=1.9Hz,1.9Hz), 6.80-6.94(2H,m), 6.99(1H, dd,J=1.8Hz,1.8Hz), 7.55-7.95(5H,m), 10.19(1H,s)
Elemental analysis:
Calculated+(H₂O)_{0.5}:C;54.95,H;3.75,N;8.01
Found:C;55.04,H;3.63,N;7.63

### Preparation 36

This was prepared in a manner similar to Preparation 65 to give 5-chloro-2-methoxybenzamide.
mp:137-139°C
MS:186(M+1)
NMR(DMSO, δ ):3.88(3H,s), 7.17(1H,d,J=8.9Hz), 7.52(1H,dd,J=8.9Hz,2.8Hz)7.68 (2H,br s), 7.72(1H,d,J=2.8Hz).

### Preparation 37

This was prepared in a manner similar to Preparation 66 to give methyl 5-chloro-2-methoxybenzoate.
MS:201(M+1)
NMR(DMSO, δ ):3.79(3H,s), 3.82(3H,s), 7.19(1H,d,J=8.8Hz), 7.55-7.65(2H,m)

### Preparation 38

This was prepared in a manner similar to Preparation 16 to give N-(3-amino-5-(trifluoromethyl)phenyl]-2-chlorocinnamamide.
mp:177-179°C
MS:341(M+1)
NMR(DMSO, δ ):5.66(2H,s), 6.58(1H,s), 6.86(1H,d,J=15.6Hz), 7.13(1H,s) 7.25(1H,s), 7.42-7.60(3H,m), 7.74-7.80(1H,m), 7.78(1H,d,J=15.6Hz), 10.30(1H,s)
Elemental analysis:
Calculated:C;56.40,H;3.55,N;8.22
Found:C;56.36,H;3.65,N;8.08

### Preparation 39

This was prepared in a manner similar to Preparation 16 to give N-(3-amino-5-cyanophenyl)-2-trifluoromethylcinnamamide.
mp:191-193°C
MS:332(M+1)
NMR(DMSO, δ ):5.73(2H,s), 6.62(1H,dd,J=1.6Hz,1.6Hz), 6.86(1H,d,J=15.4Hz), 7.16(1H,d,J=1.6Hz,1.6Hz), 7.27(1H,dd,J=1.6Hz,1.6Hz), 7.60-8.00(5H,m), 10.36(1H,s)

### Preparation 40

This was prepared in a manner similar to Preparation 14 to give N-(3-aminophenyl)-alphamethylcinnamamide.
mp:140-142°C
MS:253(M+1)
NMR(DMSO, δ ):2.08(3H,d,J=1.3Hz), 5.04(2H,s), 6.28(1H,dd,J=8.2Hz,1.2Hz), 6.80(1H,dd,J=8.1Hz,1.2Hz), 6.93(1H,dd,J=7.8Hz,7.8Hz), 7.03(1H,dd,J=1.2Hz,1.2), 7.23(1H,q,J=1.3Hz), 7.32-7.47(5H,m), 9.62(1H,s)
Elemental analysis:
Calculated:C;76.17,H;6.39,N;11.10
Found:C;76.08,H;6.39,N;11.14

### Preparation 41

This was prepared in a manner similar to Preparation 14 to give N-(3-aminophenyl)-4-methylcinnamamide.
mp:90-94°C
MS:253(M+1)
NMR(DMSO, δ ):2.34(3H,s), 5.08(2H,s), 6.20(1H,d,J=8.2Hz), 6.72-7.02(4H,m), 7.25(2H,d,J=7.9Hz), 7.44-7.55(3H,m), 9.83(1H,s)
Elemental analysis:
Calculated+(H₂O)_{0.9}:C;71.57,H;6.68,N;10.43
Found:C;71.59,H;6.64,N;10.37

### Preparation 42

This was prepared in a manner similar to Preparation 14 to give N-(3-aminophenyl)-3-methylcinnamamide.
MS:253(M+1)
NMR(DMSO, δ ):2.34(3H,s), 5.09(2H,s), 6.27(1H,d,J=8.2Hz), 6.76-7.02(4H,m), 7.18-7.43(4H,m), 7.49(1H,d,J=15.7Hz), 9.85(1H,s)

### Preparation 43

This was prepared in a manner similar to Preparation 14 to give N-(3-aminophenyl)-2-methylcinnamamide.
ms:141-142°C
MS:253(M+1)
NMR(DMSO, δ ):2.40(3H,s), 5.08(2H,s), 6.28(1H,d,J=8.3Hz), 6.74(1H,d,J=15.6Hz), 6.79-6.99(3H,m), 7.21-7.36(3H,m), 7.51-7.61(1H,m 7.76(1H,d,J=15.6Hz), 9.88 (1H,s)
Elemental analysis:
Calculated+(H₂O)_{0.1}:C;75.63,H;6.43,N;11.02
Found:C;75.35,H;6.34,N;11.03

### Preparation 44

This was prepared in a manner similar to Preparation 16 to give N-[3-[(tertbutoxycarbonyl)amino]phenyl]-alphamethylcinnamamide.
MS:353(M+1), 297(M-C₄H₈+1), 253(M-BOC+1)
NMR(DMSO, δ ):1.48(9H,s), 2.09(3H,d,J=1.2Hz), 7.06(1H,d,J=8.1Hz), 7.17(1H,dd,J =8.0Hz,8.0Hz), 7.26-7.49(7H,m), 7.94(1H,s), 9.34(1H,s), 9.90(1H,s)

### Preparation 45

This was prepared in a manner similar to Preparation 16 to give N-[3-[(tertbutoxycarbonyl)amino]phenyl]-4-methylcinnamamide.
MS:353(M+1), 297(M-C4H8+1), 253(M-BOC+1)
NMR(DMSO, δ ):1.48(9H,s), 2.34(3H,s), 6.80(1H,d,J=15.7Hz), 7.06(1H,d,J=8.1Hz), 7.17(1H,dd,J=8.0Hz,8.0Hz), 7.21-7.29(2H,m), 7.41-7.59(4H,m), 7.84(1H,s), 9.37 (1H,s), 10.12(1H,s)

### Preparation 46

This was prepared in a manner similar to Preparation 16 to give N-[3-[(tertbutoxycarbonyl)amino]phenyl]-3-methylcinnamamide.
MS:353(M+1), 297(M-C₄H₈+1), 253(M-BOC+1)
NMR(DMSO, δ ):1.49(9H,s), 2.35(3H,s), 6.85(1H,d,J=15.7Hz), 7.07(1H,d,J=8.4Hz), 7.12-7.59(7H,m), 7.84(1H,s), 9.38(1H,s), 10.14(1H,s)

### Preparation 47

This was prepared in a manner similar to Preparation 16 to give N-[3-[(tertbutoxycarbonyl)amino]phenyl]-2-methylcinnamamide.
MS:353(M+1), 297(M-C₄H₈+1), 253(M-BOC+1)
NMR(DMSO, δ ):1.49(9H,s), 2.41(3H,s), 6.77(1H,d,J=15.6Hz), 7.05(1H,d,J=8.6Hz). 7.18(1H,t,J=8.1Hz), 7.24-7.35(4H,m), 7.46(1H,d,J=8.2Hz), 7.51-7.61(1H,m), 7.79 (1H,d,J=15.6Hz), 7.88(1H,s), 9.38(1H,s), 10.17(1H,s)

### Preparation 48

This was prepared in a manner similar to Preparation 16 to give N-(3-amino-5-chlorophenyl)-2-chlorocinnamamide.
mp:147-149°C
MS:307(M+1)
NMR(DMSO, δ ):5.48(2H,s), 6.32(1H,dd,J=1.9Hz,1.9Hz), 6.81(1H,dd,J=1.9Hz,1.9Hz), 6.85(1H,d,J=15.7Hz), 7.00(1H,dd,J=1.9Hz,1.9Hz), 7.40-7.49(2H,m), 7.53-7.59(1H,m), 7.72-7.59(1H,m), 7.72-7.78(1H,m), 7.84(1H,d,J=15.7Hz), 10.15(1H,s)
Elemental analysis:
Calculated:C;58.65,H3.94;,N;9.12
Found:C;58.62,H;3.85,N;8.85

### Preparation 49

This was prepared in a manner similar to Preparation 4 to give 3,5-diaminobenzonitrile.
mp:156-159°C
MS:134(M+1)
NMR(DMSO, δ ):5.22(4H,s), 6.05(3H,s).

### Preparation 50

This was prepared in a manner similar to Preparation 16 to give N-(3-cyanophenyl)-2-chlorocinnamamide.
mp:198-200°C
MS:283(M+1)
NMR(DMSO, δ ):6.88(1H,d,J=15.7Hz), 7.42-7.50(2H,m), 7.51-7.64(3H,m), 7.74-7.92 (2H,m), 7.92(1H,d,J=15.7Hz), 8.22(1H,s), 10.67(1H,s)
Elemental analysis:
Calculated+(H₂O)_{0.1}:C;67.54,H;3.97,N;9.85
Found:C;67.33,H;3.81,N;9.87

### Preparation 51

A solution of tertbutyl 3-nitrophenylcarbamate (9.01g) in methanol (90ml) was stirred under an atmosphere of hydrogen for 8 hours. Filtered through Celite, washed with methanol, and evaporated. Purified by silica gel column chromatography (hexane/ethyl acetate) to give tertbutyl 3-aminophenylcarbamate as light pink crystals.
mp:104-106°C
MS:209(M+1), 109(M-BOC+1)
NMR(DMSO, δ ):1.45(9H,s), 4.96(2H,s), 6.15(1H,dd,J=7.2Hz,1.2Hz), 6.52(1H,d,J= 7.9Hz), 6.80(1H,s), 6.84(1H,dd,J=7.9Hz,7.9Hz), 8.98(1H,s)

### Preparation 52

This was prepared in a manner similar to Preparation 14 to give N-(3-aminophenyl)-2-trifluoromethylcinnamamide.
mp:141-142°C
MS:307(M+1)
NMR(DMSO, δ ):5.11(2H,s), 6.30(1H,d,J=8.7Hz), 6.78-7.01(4H,m), 7.62(1H,dd,J =7.3Hz,7.3Hz), 7.72-7.91(4H,m), 10.02(1H,s)
Elemental analysis:
Calculated:C;62.74,H;4.28,N;9.15
Found:C;62.63,H;4.27,N;9.09

### Preparation 53

This was prepared in a manner similar to Preparation 4 to give N-(3-aminophenyl)-4-chlorocinnamamide.
mp:167-168°C
MS:273(M+1)
NMR(DMSO, δ ):5.10(2H,s), 6.28(1H,d,J=8.2Hz), 6.78-7.01(4H,m), 7.46-7.69(5H,m). 9.89(1H,s)
Elemental analysis:
Calculated:C;66.06,H;4.80,N;10.27
Found:C;65.86,H;4.78,N;10.16

### Preparation 54

This was prepared in a manner similar to Preparation 16 to give N-(3-nitorophenyl)-4-chlorocinnamamide.
mp:255-256°C
MS:303(M+1)
NMR(DMSO, δ ):6.82(1H,d,J=15.7Hz), 7.53(2H,d,J=8.5Hz), 7.59-7.72(4H,m), 7.90-8.03(2H,m), 8.74(1H,dd,J=2.1Hz,2.1Hz), 10.73(1H,s)
Elemental analysis:
Calculated:C;59.52,H;3.66,N;9.25
Found:C;59.20,H;3.58,N;9.19

### Preparation 55

This was prepared in a manner similar to Preparation 16 to give N-[3-[(tertbutoxycarbonyl)amino]phenyl]-2-trifluoromethylcinnamamide.
mp:172-173°C
MS:307(M-BOC+1)
NMR(DMSO, δ ):1.48(9H,s), 6.92(1H,d,J=15.4Hz), 7.07(1H,d,J=8.1Hz), 7.19(1H,dd,J =8.1Hz,8.1Hz), 7.45(1H,d,J=8.0Hz), 7.63(1H,dd,J=7.4Hz), 7.77-7.96(5H,m), 9.40 (1H,s), 10.31(1H,s)

### Preparation 56

This was prepared in a manner similar to Preparation 14 to give N-(3-aminophenyl)-3-methoxycinnamamide.
MS:269(M+1)
NMR(DMSO, δ ):3.80(3H,s), 5.09(2H,s), 6.27(1H,d,J=8.6Hz), 6.77-7.02(5H,m), 7.15-7.21(2H,m), 7.36(1H,dd,J=8.0Hz,8.0Hz), 7.50(1H,d,J=15.7Hz), 9.86(1H,s)

### Preparation 57

This was prepared in a manner similar to Preparation 16 to give N-[3-[(tertbutoxycarbonyl)amino]phenyl]-2-methoxycinnamamide.
MS:313(M-C₄H₈+1), 269(M-BOC+1)
NMR(DMSO, δ ):1.48(9H,s), 3.89(3H,s), 6.89(1H,d,J=15.8Hz), 6.98-7.21(4H,m), 7.38(1H,d,J=7.1Hz), 7.45(1H,d,J=8.0Hz), 7.55(1H,d,J=7.6Hz), 7.78(1H,d,J=15.8Hz), 7.86(1H,s), 9.37(1H,s), 10.12(1H,s)

### Preparation 58

This was prepared in a manner similar to Preparation 16 to give N-[3-[(tertbutoxycarbonyl)amino]phenyl]-3-methoxycinnamamide.
MS:313(M-C₄H₈+1), 269(M-BOC+1)
NMR(DMSO, δ ):1.48(9H,s), 3.81(3H,s), 6.85(1H,d,J=15.7Hz), 6.94-7.24(5H,m), 7.31-7.50(2H,m), 7.54(1H,d,J=15.8Hz), 7.84(1H,s), 9.38(1H,s), 10.16(1H,s)

### Preparation 59

This was prepared in a manner similar to Preparation 14 to give N-(3-aminophenyl)-3-chlorocinnamamide.
mp:112-114°C
MS:273(M+1)
NMR(DMSO, δ ):5.10(2H,s), 6.28(1H,d,J=7Hz), 6.70-7.00(4H,m), 7.41-7.60(4H,m), 7.67(1H,s), 9.88(1H,s)

### Preparation 60

This was prepared in a manner similar to Preparation 14 to give N-(3-aminophenyl)-2-chlorocinnamamide.
mp:159-161°C
MS:273(M+1)
NMR(DMSO, δ ):5.11(2H,s), 6.29(1H,d,J=7.6Hz), 6.78=7.08(4H,m), 7.39-7.54(2H,m), 7.54-7.63(1H,m), 7.71-7.83(1H,m) 7.83(1H,d,J=15.6Hz), 9.98(1H,s)

### Preparation 61

This was prepared in a manner similar to Preparation 16 to give N-[3-[(tertbutoxycarbonyl)amino]phenyl]-3-chlorocinnamamide.
MS:395(M+Na)
NMR(DMSO, δ ):1.48(9H,s), 6.90(1H,d,J=15.8Hz), 7.47(1H,d,J=8.1Hz), 7.19(1H,dd,J =8.0Hz,8.0Hz), 7.40-7.62(5H,m), 7.68(1H,s), 7.84(1H,s), 9.40(1H,s), 10.18(1H,s)

### Preparation 62

This was prepared in a manner similar to Preparation 64 to give 5-chloro-2-methoxybenzonitrile.
mp:97-99°C
MS:168(M+1)
NMR(DMSO, δ ):3.92(3H,s), 7.28(1H,d,J=9.1Hz), 7.74(1H,dd,J=9.1Hz,2.7Hz), 7.91 (1H,d,J=2.6Hz)

### Preparation 63

This was prepared in a manner similar to Preparation 15 to give 5-fluoro-2-methoxybenzenecarbothioamide.
mp:141-142°C
MS:184(M-1)
NMR(DMSO, δ ):3.80(3H,s), 7.07(1H,dd,J=9.1Hz,4.5Hz), 7.23(1H,ddd,J=9.0Hz, 9.0Hz,3.3Hz), 7.44(1H,dd,J=9.4Hz,3.2Hz), 9.41(1H,s), 10.09(1H,s)
Elemental analysis:
Calculated:C;51.88,H;4.35,N;7.56
Found:C;51.82,H;3.98,N;7.39

### Preparation 64

At 5 °C was added phosphorus oxychloride (2.33ml) to N,N-dimethylformamide (15ml). Stirred 30 minutes at 5 °C under nitrogen and added 5-fluoro-2-methoxybenzamide (1.40g). Stirred 1 hour at 5°C. Poured into water (100ml), allowed to stand for 30 minutes, collected by filtration, washed with water, and air dried to give 5-fluoro-2-methoxybenzonitrile (1.45g) as white crystals.
mp:113-114°C
IR(KBr):2229cm⁻¹
MS:152(M+1)
NMR(DMSO, δ ):3.90(3H,s), 7.27(1H,dd,J=9.4Hz,4.2Hz), 7.58(1H,ddd,J=9.2Hz, 9.2Hz,3.2Hz), 7.75(1H,dd,J=8.2Hz,3.2Hz)

### Preparation 65

A solution of methyl 5-fluoro-2-methoxybenzoate (2.20g), N,N-dimethylformamide (10ml), and sodium methoxide (100mg) was heated at 100°C for 7 hours. Cooled to room temperature, diluted with ethyl acetate (100ml), washed with water (three times, 100ml each time), brine (100ml), dried over Magnesium sulfate, filtered, evaporated, and purified by silica gel column chromatography (dichloromethane/methanol) to give 5-fluoro-2-methoxybenzamide (1.48g) as white crystals.
mp:144-146°C
IR(KBr):3448,1674cm⁻¹
MS:170(M+1)
NMR(DMSO, δ ):3.88(3H,s), 7.16(1H,dd,J=9.1Hz,4.4Hz), 7.33(1H,ddd,J=9.1Hz, 9.1Hz,3.4Hz), 7.53(1H,dd,J=9.4Hz,3.3Hz), 7.70(2H,s)

### Preparation 66

To a mixture of 5-fluoro-2-hydroxybenzoic acid (2.0g), potassium carbonate (4.42g), and N,N-dimethylformamide (25ml) was added at room temperature methyl iodide (3.98ml). Stirred at room temperature for four hours. Diluted with ethyl acetate (100ml), washed with water (twice, 100ml each time), brine (twice, 100ml each time), dried over Magnesium sulfate, filtered, and evaporated to give 5-fluoro-2-methoxymethylbenzoate (2.23g) as a yellow liquid.
MS:185(M+1), 153(M-CH₃OH+1)
NMR(DMSO, δ ):3.79(3H,s), 3.81(3H,s), 7.18(1H,dd,J=8.7Hz,4.2Hz), 7.35-7.48 (2H,m).

### Preparation 67

This was prepared in a manner similar to Preparation 25 to give tertbutyl 3-nitrophenylcarbamate.
MS:139(M-BOC+1)
NMR(DMSO, δ ):1.50(9H,s), 7.54(1H,dd,J=8.1Hz,8.1Hz), 7.73-7.86(2H,m), 8.48(1H, dd,J=2.1Hz,2.1Hz), 9.91(1H,s)

### Preparation 68

This was prepared in a manner similar to Preparation 16 to give N-(4-cyanophenyl)-2-chlorocinnamamide.
mp:206-207°C
MS:283(M+1)
NMR(DMSO, δ ):6.90(1H,d,J=15.7Hz), 7.42-7.61(3H,m), 7.74-7.98(6H,m), 10.75 (1H,s)

### Preparation 69

This was prepared in a manner similar to Preparation 15 to give N-[4-(aminocarbothioyl)phenyl]-2-chlorocinnamamide.
MS:317(M+1)
NMR(DMSO, δ ):6.91(1H,d,J=15.6Hz), 7.40-7.99(9H,m), 9.40(1H,s), 9.74(1H,s), 10.59(1H,s)

### Preparation 70

This was prepared in a manner similar to Preparation 13 to give 4-(2-chlorocinnamoylamino)benzenecarbimidothioate hydroiodide.
MS:331(free+1)
NMR(DMSO, δ ):2.84(3H,s), 6.94(1H,d,J=15.6Hz) 7.20-8.00(9H,m), 10.90(1H,s)

### Preparation 71

This was prepared in a manner similar to Preparation 82 to give 4-[(2-methyl-3-pyridyl)oxy]benzonitrile.
MS:211(M+1)
NMR(DMSO, δ ):2.34(3H,s), 7.01-7.11(2H,s), 7.35(1H,dd,J=8.2Hz,4.7Hz), 7.54(1H, dd,J=8.2Hz,1.4Hz), 7.80,7.91(2H,m), 8.40(1H,dd,J=4.7Hz,1.4Hz)

### Preparation 72

This was prepared in a manner similar to Preparation 15 to give 4-[(2-methyl-3-pyridyl)oxy]benzenecarbothioamide.
MS:245(M+1)
NMR(DMSO, δ ):2.37(3H,s), 6.89-6.96(2H,m), 7.32(1H,dd,J=8.1Hz,4.6Hz), 7.44(1H, dd,J=8.2Hz,1.5Hz), 7.92-7.99(2H,m), 8.35(1H,dd,J=4.6Hz,1.5Hz), 9.42(1H,s), 9.79 (1H,s)

### Preparation 73

This was prepared in a manner similar to Preparation 13 to give methyl 4-[(2-methyl-3-pyridyl)oxy]benzenecarbimidothioate hydroiodide.
MS:259(free+1)
NMR(DMSO, δ ):2.45(3H,s), 2.85(3H,s), 7.0-8.2(7H,m)

### Preparation 74

This was prepared in a manner similar to Preparation 82 to give 4-[(2-pyridylmethyl)amino]benzonitrile.
MS:210(M+1)
NMR(DMSO, δ ):4.43(2H,d,J=6.1Hz), 6.63-6.70(2H,m), 7.23-7.47(5H,m), 7.75(1H, ddd,J=7.6Hz,7.6Hz,1.8Hz), 8.50-8.56(1H,m)

### Preparation 75

This was prepared in a manner similar to Preparation 15 to give 4-[(2-pyridylmethyl)amino]benzenecarbothioamide.
MS:244(M+1)
NMR(DMSO, δ ):4.43(2H,d,J=6.1Hz), 6.54(2H,d,J=8.9Hz), 7.07(1H,t,J=6.0Hz), 7.22-7.36(2H,m), 7.74(1H,ddd,J=7.7Hz,7.7Hz,1.8Hz), 7.80(2H,d,J=8.9Hz), 8.50-8.57 (1H,m), 8.97(1H,s), 9.21(1H,s)

### Preparation 76

This was prepared in a manner similar to Preparation 13 to give methyl 4-[(2-pyridylmethyl)amino]benzenecarbimidothioate hydroiodide.
MS:258(free+1)
NMR(DMSO, δ ):2.75(3H,s), 4.54(2H,d,J=6.3Hz), 6.77(2H,d,J=9.0Hz), 7.24-7.32 (1H,m), 7.34(1H,d,J=7.9Hz), 7.70-7.83(3H,m), 7.95-8.05(1H,m), 8.51-8.57(1H,m)

### Preparation 77

This was prepared in a manner similar to Preparation 16 to give N-[5-[(tertbutoxycarbonyl)amino]-2-fluorophenyl]-2-trifluoromethylcinnamamide.
MS:325(M-BOC+1)
NMR(DMSO, δ ):1.47(9H,s), 6.4-8.3(9H,m), 9.40(1H,s), 10.02(1H,s)

### Preparation 78

This was prepared in a manner similar to Preparation 14 to give N-(5-amino-2-fluorophenyl)-2-trifluoromethylcinnamamide.
mp:173-175°C
MS:325(M+1)
NMR(DMSO, δ ):5.00(2H,s), 6.25-6.35(1H,m), 6.91(1H,dd,J=10.9Hz,8.7Hz), 7.14(1H, d,J=15.3Hz), 7.36(1H,dd,J=6.6Hz,2.6Hz), 7.62(1H,dd,J=7.2Hz,7.2Hz), 7.73-7.92 (4H,m), 9.80(1H,s)

### Preparation 79

This was prepared in a manner similar to Preparation 82 to give 4-[(1-oxido-3-pyridyl)oxy]benzonitrile.
mp:174-176°C
MS:213(M+1)
NMR(DMSO, δ ):7.15(1H,dd,J=8.4Hz,1.8Hz), 7.25-7.35(2H,m), 7.46(1H,dd, J=8.6Hz,6.5Hz), 7.85-7.95(2H,m), 8.13(1H,d,J=6.4Hz), 8.28(1H,dd,J=1.9Hz,1.9Hz)

### Preparation 80

This was prepared in a manner similar to Preparation 15 to give 4-[(1-oxido-3-pyridyl)oxy]benzenecarbothioamide.
MS:247(M+1)
NMR(DMSO, δ ):7.07(1H,dd,J=8.6Hz,2.1Hz), 7.12-7.20(2H,m), 7.42(1H,dd,J=8.6Hz, 6.4Hz), 7.95-8.03(2H,m), 8.09(1H,dd,J=6.4Hz,1.9Hz), 8.18(1H,dd,J=1.9Hz, 1.9Hz), 9.50(1H,s), 9.86(1H,s)

### Preparation 81

This was prepared in a manner similar to Preparation 13 to give methyl 4-[(1 oxido-3-pyridyl)oxy]benzenecarbimidothioate hydroiodide.
MS:261(free+1)
NMR(DMSO, δ ):2.81(3H,s), 7.15-7.22(1H,m), 7.31-7.43(2H,m), 7.49(1H,dd,J=8.5Hz, 6.4Hz), 7.91-8.09(2H,m), 8.14-8.19(1H,m), 8.30(1H,dd,J=1.9Hz,1.9Hz)

### Preparation 82

A mixture of 1-acetylpiperazine (3.84g), 4-fluorobenzonitrile (3.03g), potassium carbonate (4.15g) and N,N-dimethylformamide (15ml) was stirred at 150°C for four hours. Cooled to room temperature, added to ethyl acetate (250ml), water (250ml), separated, washed with water (100ml), brine (100ml), dried over magnesium sulfate, filtered, and evaporated. Purified by silica gel columun chromatography (dichloromethane/methanol) to give 4-(4-acetyl-1-piperazinyl) benzonitrile (3.16g) as white crystals.
mp:103-105°C
MS:229(M+1)
NMR(DMSO, δ ):2.04(3H,s), 3.29-3.45(4H,m), 3.51-3.61(4H,m), 6.97-7.07(2H,m), 7.55-7.65(2H,m)

### Preparation 83

This was prepared in a manner similar to Preparation 15 to give 4-(4-acetyl-1-piperazinyl)benzenecarbothioamide.
MS:264(M+1)
NMR(DMSO, δ ):2.04(3H,s), 3.20-3.40(4H,m), 3.50-3.64(4H,m), 6.92(2H,d,J=8.9Hz), 7.92(2H,d,J=8.9Hz), 9.17(1H,s), 9.43(1H,s)

### Preparation 84

This was prepared in a manner similar to Preparation 13 to give methyl 4-(4-acetyl-1-piperazinyl)benzenecarbimidothioate hydroiodide.
MS:278(free+1)
NMR(DMSO, δ ):2.05(3H,s), 2.77(3H,s), 3.4-3.7(8H,m), 7.08(2H,d,J=9.1Hz), 7.84(2H, d,J=9.1Hz)

### Preparation 85

This was prepared in a manner similar to Preparation 16 to give N-(3-amino-5-chlorophenyl)-2-cyanocinnamamide.
mp:261-263°C
MS:298(M+1)
NMR(DMSO, δ ):5.50(2H,br s), 6.34(1H,dd,J=1.9Hz,1.9Hz), 6.84(1H,dd,J=1.8Hz, 1.8Hz), 7.00(1H,dd,J=1.8Hz,1.8Hz), 7.01(1H,d,J=15.5Hz, 7.61(1H,ddd,J=7.6Hz, 7.6Hz,1.4Hz), 7.76(1H,d,J=15.5Hz), 7.75-7.97(3H,m), 10.24(1H,s)

### Preparation 86

This was prepared in a manner similar to Preparation 16 to give (2E)-N-(3-amino-5-chlorophenyl)-3-(2-pyridyl)-2-propenamide.
mp:101-103°C
MS:273(M+1)
NMR(DMSO, δ ):5.47(2H,br s), 6.31(1H,dd,J=1.9Hz,1.9Hz), 6.86(1H,dd,J=1.8Hz, 1.8Hz), 6.98(1H,dd,J=1.8Hz,1.8Hz), 7.28(1H,d,J=15.3Hz), 7.34-7.44(1H,m), 7.52-7.66(2H,m), 7.86(1H,ddd,J=7.7Hz,7.7Hz,1.8Hz), 8.62-8.65(1H,m), 10.18(1H,s)

### Preparation 87

To a solution of 2-(aminomethyl)pyridine (20.94g) in tetrahydrofuran (100ml) was added 2-chloro-5-nitropyridine (13.95g) and the mixture was stirred at ambient temperature for 5 hours. The mixture was poured into ice-water, stirred for 10 minutes. The precipitate was collected by filtration, washed with water and dried to give 2-(pyridine-2-ylmethylamino)-5-nitropyridine (18.87g).
APCl-mass;m/z231(M+H⁺)
NMR(DMSO, δ ):4.72(2H,d,J=5.6Hz), 6.72(2H,d,J=9.1Hz), 7.2-7.4(2H,m), 7.76(1H,dt, J=1.7Hz,7.7Hz), 8.15(1H,dd,J=2.8Hz,9.4Hz), 8.53(1H,d,J=4.4Hz), 8.67(1H,t,J=5.6Hz), 8.90(1H,d,J=2.8Hz)

### Preparation 88

A suspension of 2-(pyridine-2-ylmethylamino)-5-nitropyridine (460mg), iron powder (555mg) and ammonium chloride (166mg) in ethanol (10ml) and water (1ml) was refluxed for 2 hours. The insoluble material was filtered off, and the filtrate was evaporated. The residue was diluted with ethyl acetate and washed with aqueous sodium hydrogen carbonate (saturated) and brine. The separated organic layer was dried over sodium sulfate and evaporated to give 5-amino-2-(pyridine-2-ylmethylamino)pyridine (0.36g).
APCl-mass;m/z201(M+H⁺)
NMR(DMSO, δ ):4.29(2H,s), 4.43(1H,d,J=6.1Hz), 6.23(1H,t,J=6.1Hz), 6.37(1H,d, J=8.6Hz), 6.83(1H,dd,J=2.8Hz,8.6Hz), 7.1-7.3(1H,m), 7.29(1H,d,J=8.0Hz), 7.42(1H,d, J=2.7Hz), 7.69(1H,dt,J=1.7Hz,7.6Hz), 8.48(1H,d,J=4.8Hz)

### Preparation 89

To a suspension of 3-chloro-5-nitro-2-(pyridine-2-ylmethylamino)pyridine (1.16g), charcoal (2g) and iron (III) chloride hexahydrate (116mg) in tetrahydrofuran (100ml) was added hydrazine hydrate (2.3ml) and the mixture was refluxed for 2.5 hours. The insoluble material was filtered off and the filtrate was evaporated to give 5-amino-3-chloro-2-(pyridine-2-ylmethylamino)pyridine (1.03g).
APCl-mass;m/z235(M+H⁺)
NMR(DMSO, δ ):4.54(2H,d,J=5.5Hz), 4.65(2H,broad s), 6.23(1H,t,J=5.6Hz), 7.05(1H, d,J=2.4Hz), 7.1-7.3(2H,m), 7.42(1H,d,J=2.4Hz), 7.70(1H,dt,J=1.8Hz,7.7Hz), 8.4-8.6 (2H,m)

### Preparation 90

To a suspension of 5-nitro-2-(pyridine-2-ylmethylamino)pyridine (10g) in acetonitrile (200ml) was added N-chlorosuccinimide (7.54g) and the mixture was stirred at 60°C for 5 hours and at 50°C for 14hours. The mixture was cooled to ambient temperature and the precipitate was collected by filtration, washed with acetonitrile and dried to give 3-chloro-5-nitro-2-(pyridine-2-ylmethylamino)pyridine (8.87g).
APCl-mass;m/z265(M+H⁺)
NMR(DMSO, δ ):4.85(2H,d,J=5.9Hz), 7.3-7.4(2H,m), 7.87(1H,dt,J=1.7 Hz,7.7Hz), 8.45(1H,d,J=2.4Hz), 8.5-8.7(2H,m), 8.86(1H,d,J=2.4Hz)

### Preparation 91

To a suspension of 4-fluoro-3-nitrophenylformamide (5.60g) and potassium carbonate (5.44g) in N,N-dimethylformamide (28ml) was added 2-bromo-3-butanone (4.1ml) and the mixture was stirred for 24 hours. The mixture was diluted with ethyl acetate, washed with water and brine, dried over magnesium sulfate and evaporated to give 4-fluoro-3-nitrophenyl(1-methyl-2-oxo-propyl)formamide (6.83g).
APCl-mass;m/z255(M+H)
NMR(DMSO, δ ):1.32(3H,d,J=7.0Hz), 2.10(3H,s), 4.75(1H,q,J=7.0Hz), 7.78-7.86 (2H,m), 8.14-8.19(1H,m), 8.54(1H,s)

### Preparation 92

To a suspension of 4-fluoro-3-nitrophenyl(1-methyl-2-oxo-propyl)formamide (6.80g), ammonium acetate (20.6g) and acetic acid (5.30ml) in xylene (140ml) was refluxed for 1.5 hours. To the mixture was added ethyl acetate and an aqueous solution of sodium hydroxide (1N, 200ml) and the mixture was stirred for 10 minutes. The aqueous layer was separated and extracted with ethyl acetate. The organic layer was combined, washed with water and brine, dried over magnesium sulfate and evaporated. The crude solid was washed with ethyl acetate and the washing was concentrated in vacuo. The residue was purified by silica gel column chromatography, eluted with methanol/dichloromethane (2-4%) to give 1-(4-fluoro-3-nitrophenyl)-4,5-dimethyl-1H-imidazole (1.29g).
APCl-mass;m/z236(M+H)
NMR(DMSO, δ ):2.10(6H,s), 7.75(1H,s), 7.73-7.82(1H,m), 7.87-7.95(1H,m), 8.19-8.23(1H,m)

### Preparation 93

A suspension of 1-(4-fluoro-3-nitrophenyl)-4,5-dimethyl-1H-imidazole (480mg) in methanol (2.5ml) was hydrogenated over palladium on carbon (10% w/w, 50% wet, 96mg) under hydrogen atmosphere for 3 hours. The catalyst was filtered off, and the filtrate was evaporated under reduced pressure to give 5-(4,5-dimethyl-1H-imidazol-1-yl)-2-fluoroaniline (418mg).
APCl-mass;m/z206(M+H)
NMR(DMSO, δ ):2.03(3H,s), 2.08(3H,s), 5.46(2H,s), 6.35-6.53(1H,m), 6.69(1H,dd, J=8.0Hz,2.7Hz), 7.10(1H,dd,J=8.5Hz,2.7Hz), 7.53(1H,s)

### Preparation 94

To a suspension of 3-iodo-4-methoxypyridine (0.62g), 3-nitrophenylboronic acid (0.57g) and tetrakis(triphenylphosphine)palladium(0) (152mg) in dimetoxyethane (10ml) was added aqueous sodium carbonate (2M, 3.43ml) and the mixture was stirred at 60°C for 6 hours. The mixture was diluted with ethyl acetate and washed with aqueous sodium hydroxide (1N) and brine. The separated organic layer was dried over magnesium sulfate and evaporated. The residue was purified with silica gel (25g) column chromatography and eluted with 40-80% ethyl acetate in n-hexane to give 3-(4-methoxypyridin-3-yl)nitrobenzene (84mg).
APCI-mass;m/z231(M+H⁺)
¹H-NMR(DMSO-d6): δ ;3.90(3H,s), 7.24(1H,d,J=5.8Hz), 7.75(1H,t,J=8.0Hz), 8.00 (1H,d,J=8.0Hz), 8.24(1H,dt,J=8.2Hz,1.1Hz), 8.35(1H,t,J=1.0Hz), 8.48(1H,s), 8.53(1H, d,J=7.6Hz)

### Preparation 95

This was prepared in a manner similar to Example 91 to give N-(4-nitrophenyl)-9H-fluorene-1-carboxamide.
APCI-mass;m/z331(M+H⁺)
¹H-NMR(DMSO-d6): δ ;4.22(2H,s), 7.3-7.7(4H,m), 7.79(1H,d,J=7.5Hz), 8.08(2H,d, J=9.2Hz), 8.16(1H,d,J=7.5Hz), 8.30(2H,d,J=9.2Hz), 10.94(1H,s)

### Preparation 96

This was prepared in a manner similar to Preparation 141 to give N-(4-aminophenyl)-9H-fluorene-1-carboxamide.
APCI-mass;m/z301(M+H⁺)
¹H-NMR(DMSO-d6): δ ;4.17(2H,s), 4.94(2H,m), 6.56(2H,d,J=9.2Hz), 7.3-7.7(9H,m), 7.96(1H,d,J=6.6Hz), 8.06(1H,d,J=7.3Hz), 9.93(1H,s)

### Preparation 97

To a suspension of 5-nitro-N-(2-pyridylmethyl)-2-pyridylamine (516mg) in methanol (10ml) and tetrahydrofuran (5ml) was hydrogenated over 10% palladium on carbon (50% wet, 100mg) under hydrogen atmosphere for 6 hours. The catalyst was filtered off and the filtrate was evaporated. The residue was added a solution of hydrogen chloride in 4N 1,4-dioxane (2ml) and stirred for 10 minutes. The precipitate was collected by filtration, washed with ethyl acetate and dried to give N²-pyridine-2-ylmethyl-pyridine-2,5-diamine (612mg).
APCI-mass;m/z201(M(free)+H⁺)
¹H-NMR(DMSO-d6): δ ;4.89(2H,s), 6.95(1H,d,J=9.1Hz), 7.59(1H,dd,J=9.1,2.5Hz),. 7.7-7.9(3H,m), 8.38(1H,t,J=7.9Hz), 8.77(1H,d,J=5.1Hz)

### Preparation 98

This was prepared in a manner similar to Example 91 to give N-(2-chloro-4-pyridyl)-9H-fluorene-1-carboxamide.
APCI-mass;m/z321(M+H⁺)
¹H-NMR(DMSO-d6):d;4.21(3H,s), 7.3-7.8(6H,m), 7.9-8.1(2H,m), 8.17(1H,d,J=7.2Hz), 8.34(1H,d,J=5.6Hz), 10.91(1H,s)

### Preparation 99

To a solution of 5-[(9H-fluorene-1-ylcarbonyl)amino]methylnicotinate (305mg) in methanol (3ml) and tetrahydrofuran (3ml) was added aqueous sodium hydroxide (1N,1.15ml) and the mixture was stirred at 60°C for 2 hours. After cooling, to the mixture was added hydrochloric acid (1N, 1.2ml) and the precipitate was collected by filtration to give 5-[(9H-fluorene-1-ylcarbonyl)amino]nicotinic acid (270mg).
APCI-mass;m/z331(M+H⁺)
¹H-NMR(DMSO-d6): δ ;4.23(2H,s), 7.3-7.5(4H,m), 7.82(1H,d,J=7.1Hz), 7.99(1H,d, J=8.0Hz), 8.15(1H,d,J=7.1Hz), 8.7-8.9(2H,m), 9.15(1H,d,J=2.0Hz), 10.73(1H,s), 13.0-14.0(1H,broad s)

### Preparation 100

A mixture of 2-chloropyrimidine (5.73g) and 2-aminomethylpyridine (13.53g) was stirred at 140°C for 30 minutes. To the mixture was diluted with ethyl acetate and washed with brine. The separated organic layer was dried over magnesium sulfate and evaporated. The residue was triturated with isopropyl ether, collected by filtration, wash with isopropyl ether and dried to give N-(2-pyridylmethyl)-2-pyrimidinylamine (5.91g).
APCI-mass;m/z187.20(M+H⁺)
¹H-NMR(DMSO-d6): δ ;4.58(2H,d,J=6.3Hz), 6.59(1H,t,J=4.8Hz), 7.2-7.4(2H,m), 7.6-7.8(2H,m), 8.27(2H,d,J=4.7Hz), 8.49(1H,d,J=4.7Hz)

### Preparation 101

To a suspension of N-(2-pyridylmethyl)-2-pyridinamine (3.0g) in sulfuric acid (10ml) was added potassium nitrate (6.52g) in portions and the mixture was stirred at 60°C for 14 hours and at 70°C for 20 hours. After cooling, the mixture was poured into ice-water and pH was adjusted to 9.0 with aqueous sodium hydroxide (4N). The precipitate was collected by filtration and washed with water and methanol to give 5-nitro-N-(2-pyridylmethyl)-2-pyrimidinylamine (1.57g).
APCI-mass;m/z232.20(M+H⁺)
¹H-NMR(DMSO-d6): δ ;4.73(2H,d,J=5.9Hz), 7.2-7.4(2H,m), 7.75(1H,dt,J=1.6Hz, 7.7Hz), 8.50(1H,d,J=4.7Hz), 9.08(2H,dd,J=3.4Hz,5.1Hz), 9.28(1H,t,J=5.9Hz)

### Preparation 102

This was prepared in a manner similar to Preparation 141 to give N2-(2-pyridylmethyl)-2,5-pyrimidinyldiamine.
APCI-mass;m/z202.27(M+H⁺)
¹H-NMR(DMSO-d6): δ ;4.4-4.6(4H,m), 6.78(1H,t,J=6.2Hz), 7.2-7.4(2H,m), 7.69(1H, dt,J=1.7Hz,7.7Hz), 7.79(2H,s), 8.47(1H,d,J=4.8Hz)

### Preparation 103

To a solution of 2-aminomethylpyrazine (2.18g) and triethylamine (4.05g) in tetrahydrofuran (50ml) was added 2-chloro-5-nitropyridine (3.17g). The mixture was stirred at 60°C for 6 hours, poured into ice-water and stirred for 10 minutes. The precipitate was collected by filtration, washed with water and dried to give 5-nitro-N-(2-pyrazinylmethyl)-2-pyridylamine (4.06g).
APCI-mass;m/z232.20(M+H⁺)
¹H-NMR(DMSO-d6): δ ;4.78(2H,d,J=5.9Hz), 6.72(1H,d,J=9.4Hz), 8.16(1H,dd, J=2.8Hz,9.4Hz), 8.5-8.9(4H,m), 8.89(1H,d,J=2.8Hz)

### Preparation 104

This was prepared in a manner similar to Example 142 to give 3-chloro-5-nitro-N-(2-pyrazinylmethyl)-2-pyridylamine.
APCI-mass;m/z266.27,268.27(M+H⁺)
¹H-NMR(DMSO-d6): δ ;4.87(2H,d,J=6.0Hz), 8.43(1H,d,J=2.4Hz), 8.3-8.5(4H,m), 8.86(1H,d,J=2.4Hz)

### Preparation 105

This was prepared in a manner similar to Preparation 138 to give 3-chloro-N²-(2-pyrazinylmethyl)-2,5-pyridyldiamine.
APCI-mass;m/z236.27,238.20(M+H⁺)
¹H-NMR(DMSO-d₆): δ ;4.5-4.7(4H,m), 6.32(1H,t,J=5.9Hz), 7.05(1H,d,J=2.4Hz)7.40 (1H,d,J=2.4Hz), 8.4-8.7(3H,m)

### Preparation 106

This was prepared in a manner similar to Preparation 141 to give 3-(4-methoxypyridin-3-yl)aniline.
APCI-mass;m/z201(M+H⁺)
¹H-NMR(DMSO-d₆): δ ;3.81(3H,s), 5.10(2H,s), 6.5-6.7(2H,m), 7.04(1H,d,J=7.8Hz), 7.12(1H,d,J=5.8Hz), 7.5-7.7(1H,m), 8.26(1H,s), 8.41(1H,d,J=5.7Hz)

### Preparation 107

This was prepared in a manner similar to Preparation 109 to give 3-(1-methyl-2-difluoromethyl-1H-imidazol-5-yl)nitrobenzene.
APCI-mass;m/z254(M+H⁺)
¹H-NMR(DMSO-d6): δ ;3.78(3H,s), 7.22(1H,t,J=52.1Hz), 7.33(1H,s), 7.81(1H,t, J=7.9Hz), 8.01(1H,d,J=7.9Hz), 8.2-8.4(2H,m)

### Preparation 108

This was prepared in a manner similar to Preparation 141 to give 3-(1-methyl-2-difluoromethyl-1H-imidazol-5-yl)aniline.
APCI-mass;m/z224(M+H⁺)
¹H-NMR(DMSO-d6): δ ;3.69(3H,s), 5.25(2H,s), 6.5-6.7(3H,m), 7.01(1H,t,J=26.5Hz), 7.1-7.2(2H,m)

### Preparation 109

To a solution of N-[2-[3-[hydroxy(oxido)amino]phenyl]-2-oxoethyl]acetamide (475mg) in toluene (5ml) was added a solution of ethylamine in ethanol (20% w/v, 2ml) and acetic acid (1ml). The mixture was stirred at 95°C for 30 hours and diluted with ethyl acetate. The solution was washed with saturated aqueous sodium hydrogen carbonate and brine. The organic layer was separated, dried over magnesium sulfate and evaporated. The residue was purified with column chromatography (silica gel, dichloromethane (25g) in 2% methanol) to give 3-(1-ethyl-2-methyl-1H-imidazol-5-yl)nitrobenzene (304mg).
APCI-mass;m/z232(M+H⁺)
¹H-NMR(DMSO-d6): δ ;1.17(3H,t,J=7.2Hz), 2.39(3H,s), 4.00(2H,q,J=7.2Hz), 7.03 (1H,s), 7.75(1H,t,J=7.8Hz), 7.88(1H,d,J=7.8Hz), 8.1-8.3(2H,m)

### Preparation 110

This was prepared in a manner similar to Preparation 141 to give 3-(1-ethyl-2-methyl-1H-imidazol-5-yl)aniline.
APCI-mass;m/z202.4(M+H⁺)
¹H-NMR(DMSO-d6): δ ;1.14(3H,t,J=7.1Hz), 2.34(3H,s), 3.91(2H,q,J=7.1Hz), 5.18 (2H,s), 6.4-6.6(3H,m), 6.69(1H,s), 7.0-7.2(1H,m)

### Preparation 111

This was prepared in a manner similar to Preparation 141 to give N²-(2-pyrazinylmethyl)-2,5-diaminopyridine.
APCI-mass;m/z202(M+H⁺)
¹H-NMR(DMSO-d6): δ ;4.32(2H,s), 4.49(1H,d,J=6.1 6.34(1H,t,J=6.1Hz), 6.41 (1H,d,J=8.6Hz), 6.84(1H,dd,J=8.6Hz,2.8Hz), 7.41(1H,d,J=2.7Hz), 8.46(1H,s), 8.55 (2H,s)

### Preparation 112

To a suspension of 5-bromopyrimidine (1.0g), 2-methoxyphenylboronic acid (1.05g) and sodium carbonate (2.0g) in 95% ethanol (10ml) was added 10% palladium on carbon (50% wet, 0.4g) and the mixture was refluxed for 36 hours. The solvents were removed by evaporation. To the residue was added ethyl acetate and water and the mixture was stirred for 10 minutes. The catalyst were filtered off and the organic layer of the filtrate was separated. The solution was washed with aqueous 1N sodium hydroxide and brine successively, dried over magnesium sulfate and evaporated to give 2-(pyrimidin-5-yl)anisole (984mg).
APCI-mass;m/z187.2(M+H⁺)
¹H-NMR(DMSO-d6): δ ;3.82(3H,s), 7.08(1H,dt,J=1.0Hz,7.4Hz), 7.19(1H,d,J=8.0Hz) 7.4-7.6(2H,m), 8.93(2H,s), 9.13(1H,s)

### Preparation 113

To a suspension of 2-(pyrimidine-5-yl)anisole (830mg) in sulfuric acid (5ml) and acetic acid (10ml) was added potassium nitrate (450mg) in portions at 0°C. The mixture was stirred at 5°C for 1 hour and poured into ice-water. The suspension was adjusted to pH 8.0 with aqueous 4N sodium hydroxide and extracted with ethyl acetate. The organic layer was separated, washed with saturated aqueous sodium hydrogen carbonate and brine, dried over magnesium sulfate and evaporated. The residue was triturated with methanol, collected by filtration and dried to give 3-(pyrimidine-5-yl)-4-methoxynitrobenzen (443mg).
APCI-mass;m/z232(M+H⁺)
¹H-NMR(DMSO-d6): δ ;3.96(3H,s), 7.42(1H,d,J=9.4Hz), 8.3-8.4(2H,m), 9.02(2H,s), 9.21(1H,s)

### Preparation 114

To a suspension of 3-nitrophenylisothiocyanate (1.8g) and acetylhydrazine (740mg) in n-butanol was added 1,8-diazabicyclo[5.4.0]undeca-7-ene (0.36ml). The mixture was refluxed for 8 hours and evaporated. To the residue was added 1N hydrochloric acid and extracted with ethyl acetate. The organic layer was washed with brine, dried over magnesium sulfate and evaporated. The residue was triturated with methanol, collected by filtration, washed with methanol and dried to give 5-methyl-4-(3-nitrophenyl)-2,4-dihydro-3H-1,2,4-triazol-3-thione (1.56g).
APCI-mass;m/z237.33(M+H⁺)
¹H-NMR(DMSO-d6): δ ;2.14(3H,s), 7.87(1H,t,J=7.9Hz), 7.97(1H,dt,J=8.2Hz,1.6Hz), 8.3-8.5(2H,m), 13.77(1H,broad s)

### Preparation 115

To a suspension of 5-methyl-4-(3-nitrophenyl)-2,4-dihydro-3H-1,2,4-triazol-3-thione (1.0g) in acetic acid (20ml) was added a solution of sodium nitrite (1.17g) in water (6ml) dropwise at 5°C and the mixture was stirred at 100°C for 4.5 hours. The solvents were removed by evaporation and to the residue was added water. The solution was adjusted to pH 9.0 with 1N aqueous sodium hydroxide. The precipitate was collected by filtration, washed with water and dried to give 3-methyl-4-(3-nitrophenyl)-4H-1,2,4-triazole (564mg).
APCI-mass;m/z205(M+H⁺)
¹H-NMR(DMSO-d6): δ ;2.39(3H,s), 7.88(1H,t,J=8.0Hz), 8.03(1H,m), 8.37(1H,m), 8.43(1H,t,J=4.1Hz), 8.79(1H,s)

### Preparation 116

This was prepared in a manner similar to Preparation 141 to give 3-(3-methyl-4H-1,2,4-triazol-4-yl)aniline.
APCI-mass;m/z175(M+H⁺)
¹H-NMR(DMSO-d6): δ ;2.31(3H,s), 5.49(2H,s), 6.5-6.6(2H,m), 6.67(1H,dd,J=7.9Hz, 1.8Hz), 7.17(1H,t,J=8.0Hz) 8.58(1H,s)

### Preparation 117

This was prepared in a manner similar to Preparation 141 to give 4-methoxy-3-(5-pyrimidinyl)aniline.
APCI-mass;m/z202.27(M+H⁺)
¹H-NMR(DMSO-d6): δ ;3.66(3H,s), 4.82(2H,m), 6.6-6.8(2H,m), 6.91(1H,dd,J=7.2Hz, 2.1Hz), 8.86(2H,s), 9.10(1H,s)

### Preparation 118

To a mixture of 2-(3-nitrophenyl)-1,3-dioxolane (12.8g), ammonium chloride (1.75g), ethyl alcohol (100ml) and water (50ml) were added Celite (12.8g) and iron powder (11.0g) under stirring at 70°C. The stirring was continued at reflux for 40 minutes. After cooling, the reaction mixture was diluted with ethyl acetate (250ml) and filtered through Celite. The filtrate was washed with saturated aqueous sodium hydrogen carbonate (200ml) and brine (100ml). The separated organic layer was dried over magnesium sulfate, decolored with activated carbon and filtered. The solvent was evaporated to give 3-(1,3-dioxolan-2-yl)aniline (9.76g) as oil.
MS:166(M+1)
NMR(DMSO, δ ):3.8-4.1(4H,m). 5.10(2H,br s), 5.55(1H,s), 6.4-6.7(3H,m), 7.00(1H,t, J=7.7Hz)

### Preparation 119

This was prepared in a manner similar to Example 156 to give N-[3-(1,3-dioxolan-2-yl)phenyl]-9H-fluorene-1-carboxamide (6.29g).
MS:358(M+1)
NMR(DMSO, δ ):3.8-4.2(4H,m), 4.20(2H,br s), 5.75(1H,s), 7.18(1H,d,J=7.6Hz), 7.2-8.1(9H,m), 8.11(1H,d,J=7.0Hz), 10.40(1H,s)

### Preparation 120

To a mixture of N-[3-(1,3-dioxolan-2-yl)phenyl]-9H-fluorene-1-carboxamide (5.90g) in tetrahydrofuran (110ml) was added 1N hydrochloric acid (20ml) at ambient temperature. After stirring for 3 hours, the reaction mixture was diluted with ethyl acetate (300ml) and washed with water (twice, 100ml), saturated aqueous sodium hydrogencarbonate (100ml) and brine (100ml). The organic layer was dried over magnesium sulfate and filtered. Evaporation gave a residue which was triturated with ethyl acetate (40ml) to give N-(3-formylphenyl)-9H-fluorene-1-carboxamide (4.44g) as pale brown crystals.
MS:314(M+1)
NMR(DMSO, δ ):4.22(2H,br s), 7.3-7.9(7H,m), 7.9-8.2(3H,m), 8.45(1H,br s), 10.04 (1H,s), 10.63(1H,br s)

### Preparation 121

This was prepared in a manner similar to Example 122 to give 2-chloro-N-(3-nitrobenzyl)-4-pyridylamine.
MS:264(M+1)
NMR(DMSO, δ ):4.52(2H,d,J=6.2Hz), 6.5-6.7(2H,m), 7.5-7.9(4H,m), 8.0-8.3(2H,m)

### Preparation 122

This was prepared in a manner similar to Preparation 118 to give N-(3-aminobenzyl)-N-(2-chloro-4-pyridyl)amine.
MS:234(M+1)
NMR(DMSO, δ ):4.16(2H,d,J=5.8Hz), 5.06(2H,br s), 6.36-6.6(5H,m), 6.97(1H,t, J=7.6Hz), 7.39(1H,t,J=5.8Hz), 7.79(1H,d,J=5.8Hz)

### Preparation 123

To a suspension of 2-chloro-N-(3-nitrobenzyl)-4-pyridylamine (264mg) in methanol (10ml) was added palladium on carbon(10%, 50% wet, 50mg), and the resultant mixture was hydrogenated under atmospheric pressure of hydrogen for 3 hours. The catalyst was removed by filtration and the filtrate was concentrated under reduced pressure to give N-(3-aminobenzyl)-N-(4-pyridyl)amine (232mg).
MS:200(M+1)
NMR(DMSO, δ ):4.36(2H,d,J=5.8Hz), 6.4-6.6(3H,m), 6.7-7.2(3H,m), 8.15(2H,d, J=6.5Hz), 9.22(1H,t,J=5.8Hz)

### Preparation 124

To a mixture of 1,1'-thiocarbonyldiimidazole (1.87g), imidazole (143mg), acetonitrile (27ml) were added a suspension of 3-nitrobenzylamine hydrochloride (1.32g) in acetonitrile (7ml) and imidazole (477mg) at 2∼4°C. After stirring for 3 hours at ambient temperature, to the reaction mixture was added 4-fluoro-1,2-benzenediamine (1,77g) under stirring. The reaction mixture was stirred at 50°C for 3 hours, and at ambient temperature for 20 hours. The reaction mixture was evaporated under reduced pressure to give a residue which was purified by silica gel chromatography and eluted with ethyl acetate-n-hexane=2/3 to give N-(2-amino-4-fluorophenyl)-N'-(3-nitrobenzyl)thiourea (2.28g).
MS:321(M+1)
NMR(DMSO, δ ):4.80(2H,d,J=5.9Hz), 5.17(2H,br s), 6.2-6.6(2H,m), 6.8-7.1(1H,m), 7.61(1H,t,J=7.9Hz), 7.78(1H,d,J=7.7Hz), 7.8-8.2(3H,m), 9.00(1H,br s)

### Preparation 125

To a mixture of N-(2-amino-4-fluorophenyl)-N'-(3-nitrobenzyl)thiourea (2.03g) in toluene (50ml) was added 1-ethyl-3-(3-dimethylaminopropyl)carbodiimide hydrochloride (1.82g) at ambient temperature. The mixture was heated at 110°C for 30 minutes. After cooling, the reaction mixture was diluted with ethyl acetate (50ml) and washed with 5%-aqueous sodium hydrogen carbonate (80ml), water (twice, 50ml each time) and brine (50ml). The organic layer was dried over magnesium sulfate and filtered. Evaporation gave a residue which was purified by silica gel chromatography and eluted with ethyl acetate to give 5-fluoro-N-(3-nitrobenzyl)-1H-benzimidazol-2-amine (676mg) as crystals.
MS:287(M+1)
NMR(DMSO, δ ):4.64(2H,d,J=6.1Hz), 6.5-6.8(1H,m), 6.8-7.2(2H,m), 7.2-7.6(1H,m), 7.63(1H,t,J=7.9Hz), 7.83(1H,d,J=7.6Hz), 8.12(1H,d,J=8.2Hz), 8.24(1H,br s), 11.00 (1H,br s)

### Preparation 126

This was prepared in a manner similar to Preparation 118 to give N-(3-aminobenzyl)- z5-fluoro-1H-benzimidazol-2-amine.
MS:257(M+1)
NMR(DMSO, δ ):4.35(2H,d,J=6.1Hz), 5.01(2H,br s), 6.3-6.8(4H,m), 6.8-7.2(4H,m)

### Preparation 127

This was prepared in a manner similar to Preparation 124 to give N-(2-amino-4-methoxyphenyl)-N'-(3-nitrobenzyl)thiourea.
MS:333(M+1)
NMR(DMSO, δ ):3.67(3H,s), 4.79(2H,d,J=5.9Hz), 4.87(2H,br s), 6.18(1H,dd, J=2.7Hz,8.6Hz), 6.34(1H,d,J=2.7Hz), 6.84(1H,d,J=8.6Hz), 7.60(1H,t,J=7.8Hz), 7.7-7.9(2H,m), 8.0-8.2(2H,m), 8.95(1H,br s)

### Preparation 128

This was prepared in a manner similar to Preparation 125 to give 5-methoxy-N-(3-nitrobenzyl)-1H-benzimidazol-2-amine.
MS:299(M+1)
NMR(DMSO, δ ):3.69(3H,s), 4.62(2H,d,J=6.0Hz), 6.4-6.6(1H,m), 6.73(1H,br s), 6.9-7.4(2H,m), 7.62(1H,t,J=7.9Hz), 7.84(1H,d,J=7.7Hz), 8.0-8.2(1H,m), 8.24(1H,br s), 10.7-10.9(1H,m)

### Preparation 129

This was prepared in a manner similar to Preparation 118 to give N-(3-aminobenzyl)-5-methoxy-1H-benzimidazol-2-amine.
MS:269(M+1)
NMR(DMSO, δ ):3.69(3H,s), 4.33(2H,d,J=6.0Hz), 5.00(2H,br s), 6.3-6.6(4H,m), 6.72(1H,d,J=2.3Hz), 6.8-7.1(3H,m), 10.51(1H,br s)

### Preparation 130

A mixture of 5-(3-fluoro-5-nitrophenyl)-1,2-dimethyl-1H-imidazole (400mg) and morpholine (5ml) was heated at reflux for 36 hours. After cooling, the reaction mixture was diluted with ethyl acetate (50ml) and washed with water (twice, 30ml each time). The separated organic layer was evaporated to give a residue which was triturated with isopropyl ether to give crude crystals. The crude crystals were dissolved in dichloromethane, dried over magnesium sulfate, decolored with activated carbon, and then filtered. Evaporation gave a residue which was triturated with isopropyl ether to give 4-[3-(1,2-dimethyl-1H-imidazol-5-yl)-5-nitrophenyl] morpholine (327mg) as pure crystals.
MS:303(M+1)
NMR(DMSO, δ ):2.36(3H,s), 3.2-3.4(4H,m), 3.57(3H,s), 3.6-3.9(4H,m), 7.03(1H,s), 7.3-7.4(1H,m), 7.5-7.7(2H,m)

### Preparation 131

To a mixture of 4-[3-(1,2-dimethyl-1H-imidazol-5-yl)-5-nitrophenyl] morpholine (320mg), activated carbon (480mg), and tetrahydrofuran (4.8ml) were added iron (III) chloride hexahydrate (32mg) and hydrazine monohydrate (0.48ml). The mixture was heated at 80°C for 1 hour. After cooling, the reaction mixture was evaporated. The resultant residue was diluted with ethyl acetate (50ml) and washed with water (twice, 40ml each time) and brine (30ml). The separated organic layer was dried over magnesium sulfate and filtered. The solvent was evaporated to give 3-(1,2-dimethyl-1H-imidazol-5-yl)-5-(4-morpholinyl)aniline (277mg) as crystals.
MS:273(M+1)
NMR(DMSO, δ ):2.31(3H,s), 2.9-3.1(4H,m), 3.48(3H,s), 3.6-3.8(4H,m), 5.03(2H,br s), 6.0-6.2(3H,m), 6.71(1H,s)

### Preparation 132

A solution of 3,5-diaminochlorobenzene (3.0g) in formic acid (30ml) was refluxed for 12 hours. After cooling, the mixture was poured into ice-water and stirred for 10 minutes. The precipitate was collected by filtration, washed with water and dried to give N-(3-chloro-5-formylaminophenyl)formamide (3.64g).
APCI-mass;m/z199.07(M+H⁺)
¹H-NMR(DMSO-d6): δ ;7.48(2H,s), 7.73(1H,s), 8.28(2H,s), 10.41(2H,s)

### Preparation 133

A suspension of 3,5-dinitrobenzonitrile (2.75g) in methanol (15ml) and tetrahydrofuran (15ml) was hydrogenated over 10 % palladium on carbon (50% wet) under hydrogen atmosphere for 6 hours. The catalyst was filtered off. The filtrate was concentrated, purified with silica gel column chromatography and eluted with dichloromethane in 0-1% methanol to give 3-cyano-5-nitroaniline (951mg).
ESI-mass;m/z162.3(M-H⁺)
¹H-NMR(DMSO-d6): δ ;6.33(2H,s), 7.24(1H,s), 7.6-7.8(2H,m)

### Preparation 134

This was prepared in a manner similar to Preparation 132 to give N-formyl-3-cyano-5-nitroaniline.
APCI-mass;m/z190.20(M-H⁺)
¹H-NMR(DMSO-d6): δ ;8.32(1H,s), 8.44(2H,m), 8.76(1H,s), 10.94(1H,s)

### Preparation 135

To a suspension of N-formyl-3-cyano-5-nitroaniline (994mg) and potassium carbonate (862mg) in N,N-dimethylformamide (10ml) was added 2-bromo-3-butanone (864mg) and the mixture was stirred for 2 hours. The mixture was diluted with ethyl acetate, washed with water and brine, dried over magnesium sulfate and evaporated.

To the residue was added ammonium acetate (4.01g) and acetic acid (1ml) in xylene (20ml) was refluxed for 30 minutes. To the mixture was added ethyl acetate and an 1N aqueous solution of sodium hydroxide (100ml) and the mixture was refluxed for 10 minutes. The aqueous layer was separated and washed with water and brine, dried over magnesium sulfate and evaporated. The residue was purified with column chromatography with silica gel (25g), and eluted with 2% methanol/dichloromethane to give 4,5-dimethyl-1-(3-cyano-5-nitrophenyl)imidazole (0.81g).
APCI-mass;m/z243.13(M+H⁺)
¹H-NMR(DMSO-d6): δ ;2.16(3H,s), 7.87(1H,s), 8.50(1H,t,J=1.7Hz), 8.55(1H,m), 8.81 (1H,t,J=1.7Hz)

### Preparation 136

To a suspension of N-(3-chloro-5-formylamino-phenyl)formamide (1g) in N,N-dimethylformamide (10ml) was added 60% sodium hydride in mineral oil dispersion (201mg) and the mixture was stirred for 30 minutes. To the mixture was added 2-bromo-3-butanone (760mg) in N,N-dimethylformamide (5ml) and the mixture was stirred for 2 hours. The mixture was diluted with ethyl acetate, washed with water and brine, dried over magnesium sulfate and evaporated to give N-[3-chloro-5-[formyl-(1-methyl-2-oxo-propyl)amino]phenyl]formamide (560mg).
APCI-mass;m/z268.73(M+H⁺)
¹H-NMR(DMSO-d6): δ ;1.32(3H,d,J=7.1Hz), 2.14(3H,s), 4.66(1H,q,J=7.1Hz), 7.1-7.3 (2H,m), 7.42(1H,s), 7.73(1H,s), 8.3-8.5(2H,m), 10.51(1H,s)

### Preparation 137

A suspension of N-[3-chloro-5-[formyl-(1-methyl-2-oxo-propyl)amino] phenyl]formamide (443mg), ammonium acetate (1.27g) and acetic acid (0.5ml) in xylene (10ml) was stirred at 130°C for 30 minutes. To the mixture was added ethyl acetate and 1N aqueous solution of sodium hydroxide (100ml) and the mixture was stirred for 10 minutes. The aqueous layer was separated and washed with water and brine, dried over magnesium sulfate and evaporated. The residue was dissolved in 6N hydrochloric acid (1ml) and methanol (1ml) and the solution was stirred for 1 hour. To the mixture was added aqueous sodium hydroxide (1N, 10ml) and ethyl acetate (20ml). The separated organic layer was washed with brine, dried over magnesium sulfate and evaporated. The residue was triturated with isopropyl ether, collected by filtration and dried to give 4,5-dimethyl-1-(3-chloro-5-aminophenyl)imidazole (287mg).
APCI-mass;m/z222.33,224.37(M+H⁺)
¹H-NMR(DMSO-d6): δ ;2.06(3H,s), 2.08(3H,s), 5.75(2H,s), 6.44(1H,t,J=1.9Hz), 6.52 (1H,t,J=1.9Hz), 6.64(1H,t,J=1.9Hz), 7.58(1H,s)

### Preparation 138

To a suspension of 4,5-dimethyl-1-(3-cyano-5-nitrophenyl)imidazole (125mg), iron(III) chloride (8mg) and charcoal (120mg) in tetrahydrofuran (1ml) was added hydrazine hydrate (0.15ml) and the mixture was stirred at 80°C for 1 hour. The catalyst was filtered off, and the filtrate was diluted with ethyl acetate. The solution was washed with 1N aqueous sodium hydroxide and brine. The organic layer was dried over magnesium sulfate and evaporated to give 4,5-dimethyl-1-(3-cyano-5-aminophenyl)imidazole (106mg).
APCI-mass;m/z213.20(M+H⁺)
¹H-NMR(DMSO-d6): δ ;2.07(3H,s), 2.08(3H,s), 5.94(2H,s), 6.79(1H,t,J=2.0Hz), 6.9-7.2(2H,m), 7.62(1H,s)

### Preparation 139

A suspension of 3,5-dinitroanizole (5.0g) in methanol (25ml) and tetrahydrofuran (25ml) was hydrogenated over 10 % palladium on carbon (50% wet) under hydrogen atmosphere for 7 hours. The catalyst was filtered off and the filtrate was concentrated. To the residue was added formic acid (50ml) and the mixture was refluxed for 1 hour. After cooling, the mixture was poured into water. The precipitate was collected by filtration and dried. The precipitate was purified with column chromatography with silica gel (50g), and eluted with 30-50% ethyl acetate in hexane to give N-formyl-3-methoxy-5-nitroaniline (1.73g).
APCI-mass;m/z195.07(M-H⁺)
¹H-NMR(DMSO-d6): δ ;3.89(3H,s), 7.4-7.6(2H,m), 8.14(1H,t,J=1.9Hz), 8.37(1H,s), 10.63(1H,s)

### Preparation 140

This was prepared in a manner similar to Preparation 135 to give 4,5-dimethyl-1-(3-methoxy-5-nitrophenyl)imidazole.
APCI-mass;m/z248.20(M+H⁺)
¹H-NMR(DMSO-d6): δ ;2.12(3H,s), 2.14(3H,s), 3.94(3H,s), 7.50(1H,t,J=2.1Hz), 7.7-7.9(2H,m)

### Preparation 141

A suspension of 4,5-dimethyl-1-(3-methoxy-5-nitrophenyl)imidazole (500mg) in methanol (10ml) was hydrogenated over 10% palladium on carbon (50% wet, 100mg) under hydrogen atmosphere for 3 hours. The catalyst was filtered off and the filtrate was concentrated to give 4,5-dimethyl-1-(3-methoxy-5-aminophenyl) imidazole.
APCI-mass;m/z218.33(M+H⁺)
¹H-NMR(DMSO-d6): δ ;2.06(3H,s), 2.08(3H,s), 3.69(3H,s), 5.41(2H,s), 6.04(1H,t, J=2.0Hz), 6.09(1H,t,J=2.0Hz), 6.18(1H,t,J=2.0Hz), 7.52(1H,s)

### Preparation 142

To a suspension of 4-methyl-1-(3-nitrophenyl)-1H-imidazole (3.2g) in acetonitrile (2ml) was added N-chlorosuccinimide (2.73g) and the mixture was stirred at 70°C for 6 hours. The mixture was diluted with ethyl acetate, washed with 1N aqueous sodium hydroxide, water and brine. The separated organic layer was dried over magnesium sulfate and evaporated. The residue was purified with column chromatography with silica gel (50g), and eluted with 30% ethyl acetate in n-hexane to give 5-chloro-4-methyl-1-(3-nitrophenyl)-1H-imidazole (642mg).
APCI-mass;m/z238.27(M+H⁺)
¹H-NMR(DMSO-d6): δ ;2.18(3H,s), 7.8-8.1(2H,m), 8.08(1H,s), 8.3-8.5(2H,m)

### Preparation 143

This was prepared in a manner similar to Preparation 138 to give 3-(5-chloro-4-methyl-1H-imidazol-1-yl)aniline.
APCI-mass;m/z208.20,210.13(M+H⁺)
¹H-NMR(DMSO-d6): δ ;2.13(3H,s), 5.47(2H,s), 6.5-6.7(3H,m), 7.15(1H,t,J=7.9Hz), 7.80(1H,s)

### Preparation 144

A mixture of 2-chloro-3-methyl-5-nitropyridine (967mg) and 2-pyridylmethanamine (1.7ml) in tetrahydrofuran (10ml) was stirred for 18 hours. The mixture was poured into water and stirred for 1 hour. The precipitated solid was collected by filtration, washed with water, and dried to give 3-methyl-5-nitro-N-(2-pyridylmethyl)-2-pyridinamine (985mg).
APCI-mass;m/z245(M+1)
NMR(200MHz,DMSO-d6): δ ;2.25(3H,s), 4.79(2H,d,J=5.9Hz), 7.23(1H,d,J=7.4Hz), 7.26(1H,d,J=7.7Hz), 7.71(1H,td,J=7.7Hz,1.8Hz), 8.01(1H,t,J=5.9Hz), 8.08(1H,t, J=2.6Hz), 8.49-8.53(1H,m), 8.77(1H,d,J=2.6Hz)

### Preparation 145

A suspension of 3-methyl-5-nitro-N-(2-pyridylmethyl)-2-pyridinamine (970mg) in methanol (15ml) and tetrahydrofuran (15ml) was hydrogenated over palladium (10% w/w, 50% wet, 450mg) on carbon under hydrogen atmosphere for 5 hours. The catalyst was filtered off, and the filtrate was evaporated under reduced pressure to give 3-methyl-N²-(2-pyridylmethyl)-2,5-pyridyldiamine (851mg).
APCI-mass;m/z215(M+1)
NMR(200MHz,DMSO-d6): δ ;2.07(3H,s), 4.24(2H,s), 4.53(2H,d,J=5.8Hz), 5.75(1H,t, J=5.8Hz), 6.73(1H,d,J=2.6Hz), 7.16-7.28(3H,m), 7.66(1H,td,J=7.6Hz,1.8Hz), 8.47(1H, d,J=4.0Hz)

### Preparation 146

A mixture of 2-chloro-5-nitro-3-(trifluoromethyl)pyridine (226mg) and 2-pyridylmethanamine (0.2ml) in tetrahydrofuran (2.0ml) was stirred for 18 hours. The mixture was poured into water and stirred for 1 hour. The precipitated solid was collected by filtration, washed with water, and dried to give 5-nitro-N-(2-pyridylmethyl)-3-(trifluoromethyl)-2-pyridinamine (277mg).
APCI-mass;m/z299(M+1)
NMR(200MHz,DMSO-d6): δ ;4.86(2H,s), 7.24(1H,d,J=7.6Hz), 7.27(1H,d,J=5.1Hz). 7.75(1H,td,J=7.6Hz,1.8Hz), 8.46(1H,s), 8.47(1H,s), 8.51(1H,dd,J=6.3Hz,1.6Hz), 9.08(1H,d,J=2.6Hz)

### Preparation 147

A suspension of 5-nitro-N-(2-pyridylmethyl)-3-(trifluoromethyl)-2-pyridinamine (265mg) in methanol (3.0ml) and tetrahydrofuran (3.0ml) was hydrogenated over palladium on carbon (10% w/w, 50% wet, 82mg) under hydrogen atmosphere for 3 hours. The catalyst was filtered off, and the filtrate was evaporated under reduced pressure to give N²-(2-pyridylmethyl)-3-(trifluoromethyl)-2,5-pyridyldiamine (240mg).
APCI-mass;m/z269(M+1)
NMR(200MHz,DMSO-d6): δ ;4.59(2H,d,J=5.4Hz), 4.74(2H,s), 6.27(1H,t,J=5.4Hz), 7.19(1H,s), 7.20(1H,s), 7.25(1H,s), 7.70(2H,td,J=7.6Hz,1.7Hz), 8.49(1H,d,J=4.6Hz)

### Preparation 148

A mixture of 2-chloro-5-nitropyridine (650mg), N-methyl (2-pyridyl) methanamine (600mg) and triethylamine (0.7ml) in tetrahydrofuran (6ml) was stirred for 18 hours. The mixture was poured into water and stirred for 1 hour. The precipitated solid was collected by filtration, washed with water, and dried to give N-methyl-5-nitro-N-(2-pyridylmethyl)-2-pyridinamine (563mg).
APCI-mass;m/z245(M+1)
NMR(200MHz,DMSO-d6): δ ;3.27(3H,s), 5.01(2H,s), 6.83(1H,d,J=9.5Hz), 7.23(1H,d, J=7.4Hz), 7.28(1H,dd,J=7.5Hz,4.9Hz), 7.76(1H,td,J=7.7Hz,1.8Hz), 8.23(1H,dd, J=9.5Hz,2.8Hz), 8.51(1H,d,J=4.1Hz), 8.95(1H,d,J=2.8Hz)

### Preparation 149

A suspension of N-methyl-5-nitro-N-(2-pyridylmethyl)-2-pyridinamine (200mg) in methanol (3.0ml) and tetrahydrofuran (3.0ml) was hydrogenated over palladium on carbon (10% w/w, 50% wet, 93mg) under hydrogen atmosphere for 3 hours. The catalyst was filtered off, and the filtrate was evaporated under reduced pressure to give N²-methyl-N²-(2-pyridylmethyl)-2,5-pyridyldiamine (174mg).
APCI-mass;m/z215(M+1)
NMR(200MHz,DMSO-d6): δ ;2.97(3H,s), 4.41(2H,br), 4.70(2H,s), 6.47(1H,d, J=8.7Hz), 6.91(1H,dd,J=5.8Hz,2.8Hz), 7.07(1H,d,J=7.8Hz), 7.21(1H,dd,J=6.4Hz, 4.9Hz), 7.55(1H,d,J=2.4Hz), 7.67(1H,td,J=7.7Hz,1.8Hz), 8.48(1H,d,J=2.8Hz)

### Preparation 150

A mixture of N-methyl-5-nitro-N-(2-pyridylmethyl)-2-pyridinamine (194mg) and N-chlorosuccinimide (135mg) in acetonitrile (2.5ml) was stirred at 60°C under nitrogen atmosphere for 4 hours. The mixture was diluted with dichloromethane, washed with 1N aqueous solution of sodium hydroxide (30ml), water and brine, dried over magnesium sulfate and evaporated under reduced pressure to give 3-chloro-N-methyl-5-nitro-N-(2-pyridylmethyl)-2-pyridinamine (216mg).
APCI-mass;m/z279(M+1)
NMR(200MHz,DMSO-d6): δ ;3.32(3H,s), 5.03(2H,s), 7.26-7.35(2H,m), 7.77(1H,td, J=7.7Hz,1.8Hz), 8.40(1H,d,J=2.4Hz), 8.51(1H,dt,J=3.8Hz,0.8Hz), 8.91(1H,d,J=2.4Hz)

### Preparation 151

To a suspension of 3-chloro-N-methyl-5-nitro-N-(2-pyridylmethyl)-2-pyridinamine (200mg) and activated carbon (330mg) in tetrahydrofuran (4.0ml) was added iron(III) chloride hexahydrate (20mg) and hydrazine monohydrate (0.4ml) and refluxed for 2 hours. After cooling, the reaction mixture was evaporated under reduced pressure. The residue was diluted with ethyl acetate, washed with water and brine, then dried over magnesium sulfate and evaporated under reduced pressure to give 3-chloro-N²-methyl-N²-(2-pyridylmethyl)-2,5-pyridyldiamine (185mg).
APCI-mass;m/z249(M+1)
NMR(200MHz,DMSO-d6): δ ;2.66(3H,s), 4.29(2H,s), 5.15(2H,s), 7.07(1H,d,J=2.5Hz), 7.20-7.26(1H,m), 7.44(1H,d,J=7.8Hz), 7.59(1H,d,J=2.5Hz), 7.74(1H,td,J=7.7Hz, 1.8Hz), 8.45-8.48(1H,m)

### Preparation 152

A mixture of 2-chloro-5-nitropyridine (665mg), (4-methyl-2-pyridyl) methanamine (1.0g) and triethylamine (0.53ml) in tetrahydrofuran (8ml) was stirred for 18 hours. The mixture was diluted with ethyl acetate, washed with water, saturated aqueous solution of sodium hydrogen carbonate and brine, then dried over magnesium sulfate and evaporated under reduced pressure. The residue was purified by silica gel column chromatography (silica gel 100g, eluting with 1-4% methanol/dichloromethane) to give N-[(4-methyl-2-pyridyl)methyl]-5-nitro-pyridinamine (300mg).
APCI-mass;m/z245(M+1)
NMR(200MHz,DMSO-d6): δ ;2.29(3H,s), 4.80(2H,d,J=5.3Hz), 6.71(1H,d,J=8.8Hz), 7.11(1H,d,J=5.1Hz), 7.16(1H,s), 8.15(1H,dd,J=9.4Hz,2.8Hz), 8.38(1H,d,J=5.0Hz), 8.63(1H,t,J=5.3Hz), 8.90(1H,d,J=2.8Hz)

### Preparation 153

A suspension of N-[(4-methyl-2-pyridyl)methyl)-5-nitro-2-pyridinamine (150mg) in methanol (2.0ml) and tetrahydrofuran (2.0ml) was hydrogenated over palladium on carbon (10% w/w, 50% wet, 56mg) under hydrogen atmosphere for 6 hours. The catalyst was filtered off, and the filtrate was evaporated under reduced pressure to give N²-[(4-methyl-2-pyridyl)methyl]-2,5-pyridyldiamine (133mg).
APCI-mass;m/z215(M+1)
NMR(200MHz,DMSO-d6): δ ;2.25(3H,s), 4.29(2H,br), 4.38(2H,d,J=6.1Hz), 6.17(1H,t, J=6.1Hz), 6.36(1H,d,J=8.6Hz), 6.82(1H,dd,J=2.8Hz,8.6Hz), 7.04(1H,d,J=5.0Hz), 7.13 (1H,s), 7.42(1H,d,J=2.7Hz), 8.33(1H,d,J=5.0Hz)

### Preparation 154

A mixture of 2-chloro-5-nitropyridine (1.35g), (3-methyl-2-pyridyl) methanamine (4.2g) and triethylamine (4.7ml) in tetrahydrofuran (20ml) was stirred for 18 hours. The mixture was poured into water and stirred for 1 hour. The precipitated solid was collected by filtration, washed with water, triturated with diisopropyl ether and methanol, and dried to give N-[(3-methyl-2-pyridyl)methyl]-5-nitro-2-pyridinamine (776mg).
APCI-mass;m/z245(M+1)
NMR(200MHz,DMSO-d6): δ ;2.34(3H,s), 4.71(2H,d,J=4.9Hz), 6.79(1H,d,J=9.2Hz), 7.24(1H,dd,J=7.6Hz,4.8Hz), 7.61(1H,dd,J=7.6Hz,0.8Hz), 8.13(1H,dd,J=9.3Hz,2.7Hz), 8.37(1H,dd,J=4.8Hz,1.1Hz), 8.48(1H,br) 8.92(1H,d,J=2.7Hz)

### Preparation 155

A suspension of N-[(3-methyl-2-pyridyl)methyl]-5-nitro-2-pyridinamine (200mg) in methanol (3.0ml) and tetrahydrofuran (3.0ml) was hydrogenated over palladium on carbon (10% w/w, 50% wet, 90mg) under hydrogen atmosphere for 3 hours. The catalyst was filtered off, and the filtrate was evaporated under reduced pressure to give N²-[(3-methyl-2-pyridyl)methyl]-2,5-pyridyldiamine (147mg).
APCI-mass;m/z215(M+1)
NMR(200MHz,DMSO-d6): δ ;2.31(3H,s), 4.31(2H,br), 4.40(2H,d,J=5.1Hz), 5.98(1H,t, J=5.1Hz), 6.49(1H,d,J=8.6Hz), 6.85(1H,dd,J=8.6Hz,2.8Hz), 7.20(1H,dd,J=7.5Hz, 4.8Hz), 7.49(1H,d,J=2.6Hz), 7.57(1H,d,J=7.4Hz), 8.36(1H,d,J=4.8Hz)

### Preparation 156

A mixture of 2-chloro-5-nitropyridine (1.0g), [3-chloro-5-(trifluoromethyl)-2-pyridyl]methanamine hydrochloride (1.86g) and triethylamine (1.7ml) in tetrahydrofuran (10ml) was stirred for 18 hours. The mixture was poured into water and stirred for 1 hour. The precipitated solid was collected by filtration, washed with water, and dried to give N-[[3-chloro-(5-trifluoromethyl)-2-pyridyl]methyl]-5-nitro-2-pyridinamine (1.12g).
APCI-mass;m/z333(M+1)
NMR(200MHz,DMSO-d6): δ ;4.94(2H,d,J=5.6Hz), 6.79(2H,d,J=9.0Hz), 8.16(1H,dd J=9.3Hz,2.8Hz), 8.49(1H,s), 8.66(1H,br 8.87(1H,d,J=2.8Hz 8.89(1H,s)

### Preparation 157

A suspension of N-[[3-chloro-(5-trifluoromethyl)-2-pyridyl]methyl]-5-nitro-2-pyridinamine (300mg) in methanol (3.0ml) and tetrahydrofuran (3.0ml) was hydrogenated over palladium on carbon (10% w/w, 50% wet, 85mg) under hydrogen atmosphere for 6 hours. The catalyst was filtered off, and the filtrate was evaporated under reduced pressure to give N²-[[3-chloro-(5-trifluoromethyl)-2-pyridyl]methyl]-2,5-pyridyldiamine (315mg).
APCI-mass;m/z303(M+1)
NMR(200MHz,DMSO-d6): δ ;4.72(2H,s), 6.55(2H,br), 6.70(1H,d,J=8.9Hz), 7.03-7.15 (3H,m), 7.52(1H,d,J=8.3Hz), 8.88(1H,s)

### Preparation 158

A mixture of 2-chloro-5-nitropyridine (1.0g) and 3-pyridylmethanamine (1.28ml) in tetrahydrofuran (10ml) was stirred for 18 hours. The mixture was poured into water and stirred for 1 hour. The precipitated solid was collected by filtration, washed with water, and dried to give 5-nitro-N-(3-pyridylmethyl)-2-pyridinamine (1.08g).
APCI-mass;m/z231(M+1)
NMR(200MHz,DMSO-d6): δ ;4.65(2H,d,J=5.8Hz), 6.65(1H,d,J=9.3Hz), 7.36(1H,dd, J=7.7Hz,4.8Hz), 7.73(1H,dd,J=1.8Hz,7.8Hz), 8.15(1H,dd,J=9,3Hz,2.8Hz), 8.47(1H,dd, J=4.8Hz,1.5Hz), 8.57(1H,d,J=1.9Hz), 8.62(1H,t,J=5.7Hz), 8.92(1H,d,J=2.8Hz)

### Preparation 159

A suspension of 5-nitro-N-(3-pyridylmethyl)-2-pyridinamine (400mg) in methanol (5.0ml) and tetrahydrofuran (5.0ml) was hydrogenated over palladium on carbon (10% w/w, 50% wet, 160mg) under hydrogen atmosphere for 6 hours. The catalyst was filtered off, and the filtrate was evaporated under reduced pressure to give N²-(3-pyridylmethyl)-2,5-pyridyldiamine (330mg).
APCI-mass;m/z201(M+1)
NMR(200MHz,DMSO-d6): δ ;4.31(2H,br), 4.36(2H,d,J=6.2Hz), 6.21(1H,t,J=6.2Hz), 6.34(1H,d,J=8.6Hz), 6.82(1H,dd,J=8.6Hz,2.8Hz), 7.29(1H,dd,J=7.8Hz,4.8Hz), 7.43 (1H,d,J=2.7Hz), 7.69(1H,d,J=7.8Hz), 8.40(1H,dd,J=4.8Hz,1.5Hz), 8.52(1H,d,J=1.9Hz)

### Preparation 160

A mixture of 2-chloro-5-nitropyridine (1.0g) and 2-(2-pyridyl)ethylamine (1.66ml) in tetrahydrofuran (6.5ml) was stirred for 18 hours. The mixture was poured into water and stirred for 1 hour. The precipitated solid was collected by filtration, washed with water, and dried to give 5-nitro-N-[2-(2-pyridyl)ethyl]-2-pyridinamine (1.5g).
APCI-mass;m/z245(M+1)
NMR(200MHz,DMSO-d6): δ ;3.02(2H,t,J=7.3Hz), 3.78(2H,br), 6.55(1H,d,J=9.4Hz), 7.23(1H,dd,J=7.6Hz,4.8Hz), 7.28(1H,d,J=7.9Hz), 7.71(1H,td,J=7.7Hz,1.8Hz), 8.09 (1H,d,J=7.2Hz), 8.21(1H,br), 8.51(1H,d,J=4.8Hz), 8.92(1H,d,J=2.7Hz)

### Preparation 161

A suspension of 5-nitro-N-[2-(2-pyridyl)ethyl]-2-pyridinamine (200mg) in methanol (3.0ml) and tetrahydrofuran (3.0ml) was hydrogenated over palladium on carbon (10% w/w, 50% wet, 80mg) under hydrogen atmosphere for 3 hours. The catalyst was filtered off, and the filtrate was evaporated under reduced pressure to give N²-[2-(2-pyridyl)ethyl]-2,5-pyridyldiamine (184mg).
APCI-mass;m/z215(M+1)
NMR(200MHz,DMSO-d6): δ ;2.93(2H,t,J=7.4Hz), 3.47(2H,td,J=7.4Hz,5.9Hz), 4.27 (2H,s), 5.60(1H,t,J=5.9Hz), 6.29(1H,d,J=8.6Hz), 6.81(1H,dd,J=8.6Hz,2.8Hz), 7.20(1H, dd,J=7.5Hz,4.9Hz), 7.25(1H,d,J=8.6Hz), 7.47(1H,d,J=2.7Hz), 7.68(1H,td,J=7.6Hz, 1.9Hz), 8.48(1H,d,J=4.0Hz)

### Preparation 162

To a solution of 5-chloro-1,3-benzenediamine (1.43g) in tetrahydrofuran (10ml) at 0°C under nitrogen atmosphere was added n-butyl lithium (5.8ml) in hexane dropwise. A precipitate appeared. Stirred for 30 minutes and added 3-chloro-1,2-benzoisoxazole (0.77g) all at once. After 1 hour at 0°C, removed the cold bath and stirred at room temperature for 1 hour until all dissolved to give a clear, black solution. Added water (10ml) dropwise, ethyl acetateb (100ml), water (100ml), and separated. Washed with water (50ml) plus 1N hydrochloric acid (2ml, three times), saturated aqueous sodium hydrogen carbonate (twice), brine, dried over magnesium sulfate, filtered, and evaporated. Purified by silica gel column chromatography (dichloromethane/methanol) and then recrystallized from dichloromethane to give N-1-(1,2-benzoisoxazol-3-yl)-5-chloro-1,3-benzenediamine (0.63g) as green crystals.
mp:192-194°C
MS:260(M+1)
NMR(DMSO, δ ):5.49(2H,s), 6.23(1H,dd,J=1.8Hz,1.8Hz), 6.92(2H,s), 7.32-7.41 (1H,m), 7.58-7.70(2H,m), 8.13(1H,d,J=7.9Hz), 9.42(1H,s)
Elemental analysis:
Calculated+(H₂O)_{0.05}:C;59.92,H;3.91,N;16.12
Found:C;60.15,H;4.02,N;15.76

### Preparation 163

This was prepared in a manner similar to Preparation 13 to give methyl 4-(4-acetyl-1-piperazinyl)benzenecarbimidothioate hydroiodide.
MS:278(free+1)
NMR(DMSO, δ ):2.05(3H,s), 2.77(3H,s), 3.4-3.7(8H,m), 7.08(2H,d,J=9.1Hz), 7.84(2H, d,J=9.1Hz)

### Preparation 164

To a suspension of 7-chloro-2,3-dihydro-benzo[b]oxepin-4-carboxylic acid (157mg) in dichloromethane (3ml) was added oxalyl chloride (0.16ml) and N,N-dimethylformamide (1drop) and the mixture was stirred at ambient temperature for 2 hours. The solvents were evaporated under reduced pressure to give crude acid chloride. To a suspension of 3-aminopyridine (66mg) in dichloromethane (3ml) and pyridine (0.17ml) was added a solution of acid chloride (obtained above) in dichloromethane (3ml) dropwise and the mixture was stirred at ambient temparature for 2 hours. The mixture was diluted with dichloromethane, washed with water and brine. The separated organic layer was dried over magnesium sulfate and evaporated under reduced pressure. The residue was recrystallized from methanol-isopropyl ether and collected by filtration to give 7-chloro-2,3-dihydro-benzo[b]oxepin-4-carboxylic acid pyridine-3-ylamide (168mg).
APCI-mass;m/z301(M+H⁺)
¹H-NMR(DMSO-d6): δ ;2.98(2H,t,J=4.3Hz), 4.27(2H,t,J=4.6Hz), 7.01(1H,d,J=8.7Hz), 7.2-7.5(3H,m), 7.56(1H,d,J=2.6Hz), 8.11(1H,dq,J=8.3Hz,1.5Hz), 8.29(1H.dd,J=4.7Hz, 1.4Hz), 8.85(1H,d,J=2.1Hz), 10.16(1H,s)

### Preparation 165

To a suspension of 8-methoxy-2,3-dihydro-benzo[b]oxepin-4-carboxylic acid (103mg) in dichloromethane (1ml) was added oxalyl chloride (0.1ml) and N,N-dimethylformamide (1drop) and the mixture was stirred at ambient temperature for 2 hours. The solvents were evaporated under reduced pressure to give crude acid chloride. To a suspension of 3-aminopyridine (49mg) in dichloromethane (2ml) and pyridine (0.17ml) was added a solution of acid chloride (obtained above) in dichloromethane (2ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was triturated with methanol and isopropyl ether to give 8-methoxy-2,3-dihydro-benzo[b]oxepin-4-carboxylic acid pyridine-3-ylamide (103mg).
APCI-mass;m/z297(M+H⁺)
¹H-NMR(DMSO-d6): δ ;2.94(2H,t,J=4.6Hz), 3.77(3H,s), 4.26(2H,t,J=4.6Hz), 6.55(1H, d,J=2.5Hz), 6.68(1H,dd,J=2.3Hz,8.6Hz), 7.26(1H,s), 7.3-7.5(2H,m), 8.10(1H,d, J=8.4Hz), 8.27(1H,d,J=3.4Hz), 8.85(1H,d,J=2.3Hz), 10.06(1H,s)

### Preparation 166

To a suspension of 8-methoxy-2,3-dihydro-benzo[b]oxepin-4-carboxylic acid (103mg) in dichloromethane (1ml) was added oxalyl chloride (0.1ml) and N,N-dimethylformamide (1drop) and the mixture was stirred at ambient temperature for 2 hours. The solvents were evaporated under reduced pressure to give crude acid chloride. To a suspension of 3-aminopyridine (49mg) in dichloromethane (2ml) and pyridine (0.17ml) was added a solution of acid chloride (obtained above) in dichloromethane (2ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was triturated with methanol and isopropyl ether to give 8-methoxy-2,3-dihydro-benzo[b]oxepin-4-carboxylic acid pyridine-4-ylamide (62mg).
APCI-mass;m/z297(M+H⁺)
¹H-NMR(DMSO-d6): δ ;2.93(2H,t,J=4.5Hz), 3.77(3H,s), 4.26(2H,t,J=4.6Hz), 6.55(1H, d,J=2.5Hz), 6.69(1H,dd,J=2.6Hz,8.4Hz), 7.25(1H,s), 7.38(1H,d,J=8.6Hz), 7.68(2H,d, J=6.1Hz), 8.43(2H,d,J=6.1Hz), 10.21(1H,s)

### Preparation 167

This was prepared in a manner similar to Preparation 16 to give (2E)-N-(3-amino-5-chlorophenyl)-3-(1H-indole-3-yl)-2-propenamide.
MS:312(M+1)
NMR(DMSO, δ ):5.43(2H,s), 6.28(1H,dd,J=1.9Hz,1.9Hz), 6.77(1H,d,J=15.6Hz), 6.82(1H,dd,J=1.9Hz,1.9Hz), 7.02(1H,dd,J=1.9Hz,1.9Hz), 7.18-7.25(2H,m), 7.44-7.50 (1H,m), 7.74(1H,d,J=15.6Hz) 7.83(1H,s), 7.91-7.97(1H,m), 9.81(1H,s), 11.64(1H,s)

### Preparation 168

To a suspension of 5-bromonicotinic acid (1.01g) and triethylamine (0.73ml) in tert-butanol (15ml) was added diphenylphosphoryl azide (1.08ml). The mixture was refluxed for 6 hours and diluted with ethyl acetate. The solution was washed with water, aqueous solution of sodium hydrogen carbonate, and brine. The separated organic layer was dried over magnesium sulfate and evaporated under reduced pressure. The residue was purified with column chromatography with silica gel (50g), and eluted with 2% methanol in dichloromethane to give 3-bromo-5-tert-butoxycarbonylaminopyridine (1.12g).
ESI-mass;m/z295(M+Na⁺)
¹H-NMR(DMSO-d6): δ ;1.53(9H,s), 6.78(1H,broad s), 8.34(3H,s)

### Preparation 169

To a suspension of 3-bromo-5-tert-butoxycarbonylaminopyridine (410mg), diethyl(3-pyridyl)borane (287mg), tetrakis(triphenylphosphine)palladium (87mg), and tetra-n-butylammonium bromide (48mg) in tetrahydrofuran (10ml) was added powdered potassium carbonate (297mg) and the mixture was refluxed for 12 hours. After cooling, the mixture was diluted with ethyl acetate, washed with water and brine. The separated organic layer was dried over magnesium sulfate and evaporated under reduced pressure. The residue was purified with column chromatography with silica gel (25g), and eluted with 5% methanol in dichloromethane to give [3,3']bipyridyl-5-yl-carbamic acid tert-butyl ester (201mg).
ESI-mass;m/z294(M+Na⁺)
¹H-NMR(DMSO-d6): δ ;1.54(9H,s), 6.83(1H,broad s), 7.3-7.5(1H,m), 7.91(1H,dt, J=8.0Hz,J=2.0Hz), 8.30(1H,s), 8.43(1H,d,J=2.5Hz), 8.51(1H,d,J=2.0Hz), 8.65(1H,dd, J=4.9Hz,J=1.6Hz), 8.87(1H,d,J=1.7Hz)

### Preparation 170

To a solution of [3,3']bipyridyl-5-yl-carbamic acid tert-butyl ester (100mg) in methanol (1ml) was added 12N hydrochloric acid (0.5ml). The mixture was stirred for 18 hours and evaporated under reduced pressure. The residue was triturated with isopropyl ether, filtered and dried to give 3-amino-5-(pyridine-3-yl)pyridine dihydrochloride (84mg).
ESI-mass;m/z172(M(free)+H⁺)
¹H-NMR(DMSO-d6): δ ;7.9-8.1(3H,m), 8.14(1H,d,J=2.3Hz), 8.50(1H,d,J=1.4Hz), 8.55(1H,d,J=8.6Hz), 8.87(1H,dd,J=5.4Hz,1.4Hz), 9.16(1H,d,J=1.8Hz)

### Preparation 171

To a solution of 9H-fluorene-1-carboxylic acid (0.49g) in dichloromethane (2ml) were added in turn oxalyl chloride (0.4ml) and a catalytic amount of N,N-dimethylformamide (2drops) at ambient temperature. After stirring at ambient temperature for 1 hour, the reaction mixture was evaporated in vacuo, and the residue was added in dichloromethane (5ml) to give a solution of 9H-fluorene-1-carboxyl chloride. To a solution of 5-amino-methyl nicotinic acid (0.53g) in dichloromethane (5ml) were added pyridine (1.0ml) and the acid chloride solution at 0°C. After stirring at 0°C for 1 hour, the reaction mixture was diluted with dichloromethane, washed with aqueous potassium carbonate (10%) and brine, and dried over potassium carbonate. Evaporation gave a residue which was triturated with diisopropyl ether to give 5-[(9H-fluorene-1-carbonyl)-amino]-nicotinic acid methyl ester (0.492g).
APCI-mass;345(m/z,[M+H]⁺)
NMR(DMSO-d₆,d);3.93(3H,s), 4.24(2H,s), 7.35-7.44(2H,m), 7.55-7.66(2H,m), 7.82 (1H,d,J=7.6Hz), 7.99(1H,d,J=6.4Hz), 8.16(1H,d,J=7.2Hz), 8.80-8.89(2H,m), 9.17(1H, d,J=2.4Hz), 10.77(1H,s)

### Preparation 172

To a solution of 5-[(9H-fluorene-1-carbonyl)-amino]-nicotinic acid methyl ester (0.105g) in methanol (3ml) was added hydrazine monohydrate (0.56ml), and the mixture was heated at reflux for 6 hours. After cooling to ambient temperature, the resultant precipitate was collected by filtration, washed with methanole to give 9H-fluorene-1-carboxylic acid (5-hydrazinocarbonyl-pyridine-3-yl)-amide (85mg).
APCI-mass;345(m/z,[M+H]⁺)
NMR(DMSO-d₆,d);4.23(2H,s), 4.61(2H,brs), 7.35-7.50(2H,m), 7.55-7.67(2H,m), 7.80 (1H,d,J=7.6Hz), 7.98(1H,d,J=6.7Hz), 8.15(1H,d,J=6.8Hz), 8.63-8.66(1H,m), 8.72(1H, d,J=1.8Hz), 9.06(1H,d,J=2.4Hz), 10.00(1H,s) 10.68(1H,s)

### Example 1

A mixture of S-methyl benzothioimidate hydriodide and N-(3-aminophenyl) phenylacetamide in methanol was heated at reflux. The most amine was used in 4 hours. The mixture was cooled, diluted with water, added 1N sodium hydroxide, then, extracted with dichloromethane/methanol (10:1) (twice). The combined organic layer was dried over magnesium sulfate, filtered, and evaporated. The residue was purified by silica gel column chromatography (dichloromethane/methanol → dichloromethane/methanol/ammonia), and recrystallized with methanol to give N-[3-(iminobenzylamino)phenyl]phenylacetamide.
mp:213-214°C
IR(KBr):3483,3328,1683,1635cm⁻¹
MS:330(M+1)
NMR(DMSO, δ ):3.62(2H,s), 6.24(2H,br s), 6.52(1H,d,J=6.9Hz), 7.09-7.55(11H,m), 7.88-8.01(2H,m), 10.07(1H,s)

### Example 2

This was prepared in a manner similar to Example 1 to give N-[3-(iminobenzylamino)phenyl]cinnamamide.
mp:115-117°C
IR(KBr):1672,1630cm⁻¹
MS:342(M+1)
NMR(DMSO, δ ):6.45(2H,br s), 6.60(1H,d,J=7.2Hz), 6.85(1H,d,J=15.7Hz), 7.21-7.71 (12H,m), 7.93-8.00(2H,m), 10.14(1H,br s)
Elemental analysis:
Calculated+(H₂O)_{1/3}(EtOAc)_{1/3}:C;74.38,H;5.97,N;11.15
Found:C;74.44,H;6.00,N;10.92

### Example 3

To a solution of 3-(1,2-dimethylimidazol-5-yl)aniline (94mg), 2-chlorocinnamic acid (91mg), 4-dimethylaminopyridine (24mg) and dichloromethane (2.5ml) was added at room temperature 1-[3-(dimethylamino)propyl]-3-ethylcarbodiimide hydrochloride (153mg). Stirred for two days. Diluted with ethyl acetate (50ml), washed with water (three times, 25ml each time), saturated aqueous sodium bicarbonate (three times, 25ml each time), brine (50ml), dried over magnesium sulfate, filtered, and evaporated. Purified by silica gel column chromatography (dichloromethane/methanol), recrystallized from ethyl acetate/diethyl ether to give (2E)-N-3-(1,2-dimethylimidazol-5-yl)phenyl-3-(2-chlorophenyl)-2-propenamide (151mg).
mp:105-109°C
MS:352(M+1)
NMR(DMSO, δ ):2.36(3H,s), 3.56(3H,s), 6.86(1H,m), 6.90(1H,d,J=15.7Hz), 7.15(1H, d,J=7.7Hz), 7.34-7.70(5H,m), 7.74-7.85(2H,m), 7.90(1H,d,J=15.7Hz), 10.42(1H,s)

### Example 4

This was prepared in a manner similar to Example 3 to give (2E)-N-3-(1,2,4-triazol-1-yl)phenyl-3-(2-chlorophenyl)-2-propenamide from 3-(1,2,4-triazol-1-yl) aniline.
mp:201-202°C
MS:325(M+1)
NMR(DMSO, δ ):6.91(1H,d,J=15.7Hz), 7.42-7.84(7H,m), 7.92(1H,d,J=15.7Hz), 8.26 (1H,s), 8.32(1H,dd,J=1.7Hz,1.7Hz), 9.28(1H,s), 10.61(1H,br s)

### Example 5

This was prepared in a manner similar to Example 3 to give (2E)-N-3-(5-pyrimidinyl)phenyl-3-(2-chlorophenyl)-2-propenamide from 3-(5-pyrimidinyl) aniline.
mp:193-194°C
MS:336(M+1)
NMR(DMSO, δ ):6.93(1H,d,J=15.7Hz), 7.41-7.62(4H,m), 7.75-7.85(2H,m), 7.92(1H,d, J=15.7Hz), 8.10(1H,s), 9.10(2H,s), 9.22(1H,s), 10.52(1H,s)

### Example 6

This was prepared in a manner similar to Example 3 to give (2E)-N-3-(1,2-dimethylimidazol-5-yl)phenyl-3-[2-(trifluoromethyl)phenyl]-2-propenamide hydrochloride from 3-(1,2-dimethylimidazol-5-yl)aniline.
mp:280-281°C
MS:386(free+1)
NMR(DMSO, δ ):2.66(3H,s), 3.69(3H,s), 7.02(1H,d,J=15.4Hz), 7.26(1H,d,J=7.8Hz), 7.55(1H,t,J=7.8Hz), 7.60-7.98(7H,m), 8.08(1H,br s), 10.88(1H,s), 13.5(1h,br s)

### Example 7

This was prepared in a manner similar to Example 3 to give (2E)-N-3-(1,2,4-triazol-1-yl)phenyl-3-[2-(trifluoromethyl)phenyl]-2-propenamide from 3-(1,2,4-triazol-1-yl)aniline.
mp:190-191°C
MS:381(M+Na)
NMR(DMSO, δ ):6.92(1H,d,J=15.4Hz), 7.48-7.75(4H,m), 7.76-8.00(4H,m), 8.26 (1H,s), 8.34(1H,br s), 9.29(1H,s), 10.66(1H,s)

### Example 8

This was prepared in a manner similar to Example 3 to give (2E)-N-3-(5-pyrimidinyl)phenyl-3-[2-(trifluoromethyl)phenyl]-2-propenamide from 3-(5-pyrimidinyl)aniline.
mp:181-182°C
MS:392(M+Na)
NMR(DMSO, δ ):6.94(1H,d,J=15.3Hz), 7.50-7.96(8H,m), 8.12(1H,s), 9.10(2H,s), 9.22 (1H,s), 10.57(1H,s)

### Example 9

This was prepared in a manner similar to Example 3 to give (2E)-N-[6-(2-methyl-3-pyridyloxy)pyridin-3-yl]-3-[2-(trifluoromethyl)phenyl]-2-propenamide from 3-amino-6-(2-methyl-3-pyridyloxy)pyridine.
mp:214-216°C
MS:400(M+1)
NMR(DMSO, δ):2.31(3H,s), 6.87(1H,d,J=15.4Hz), 7.15(1H,d,J=8.8Hz), 7.30(1H,dd, J=8.1Hz,4.7Hz), 7.52(1H,dd,J=8.1Hz,1.4Hz), 7.64(1H,t,J=7.6Hz), 7.74-7.94(4H,m), 8.22(1H,dd,J=8.8Hz,2.7Hz), 8.33(1H,dd,J=4.7Hz,1.4Hz), 8.38(1H,d,J=2.6Hz), 10.55 (1H,br s)

### Example 10

To a solution of 4-phenyl-3-E-butenoic acid in benzene was added thionyl chloride at room temperature, and heated at reflux for 1 hour. After cooled and evaporated, dichloromethane, 3-(imidazol-1-yl)aniline and triethylamine was added and stirred for 1 hour at room temperature. Washed with saturated aqueous sodium bicarbonate, dried over sodium sulfate, filtered and evaporated. Recrystallized from isopropyl ether/ethyl acetate to give (3E)-N-[3-(imidazol-1-yl)phenyl]-4-phenyl-3-butenamide.
mp:130-133°C
IR(Nujol):1680cm⁻¹
MS:304(M+1)
NMR(DMSO, δ ):3.20-3.40(2H,m), 6.35-6.55(1H,m), 6.58(1H,d,J=16Hz), 7.12(1H,s), 7.20-7.60(8H,m), 7.63(1H,s), 7.88(1H,br s), 8.15(1H,s), 10.27(1H,br s)

### Example 11

To a mixture of 3-(1,2-dimethyl-1H-imidazol-5-yl)-5-methoxyaniline (95mg), (2E)-3-(2-chlorophenyl)-2-propenoic acid (96mg), 1-hydroxybenzotriazole (71mg), 4-dimethylaminopyridine (64mg) and dichloromethane (5ml) was added 1-ethyl-3-(3-dimethylaminopropyl)carbodiimide hydrochloride (101mg) with stirring at ambient temperature. The stirring was continued for overnight. The reaction mixture was evaporated to give a residue which was purified by silica gel chromatography (dichloromethane-methyl alcohol) and triturated with ethyl acetate to give (2E)-3-(2-chlorophenyl)-N-[3-(1,2-dimethyl-1H-imidazol-5-yl)-5-methoxyphenyl]-2-propenamide (114mg) as colorless crystals.
MS:382(M+1)
NMR(DMSO, δ ):2.36(3H,s), 3.56(3H,s), 3.80(3H,s), 6.71(1H,br s), 6.88(1H,d, J=15.6Hz), 6.89(1H,s), 7.31(1H,br s), 7.39(1H,br s), 7.4-7.5(2H,m), 7.5-7.6(1H,m), 7.7-7.8(1H,m), 7.89(1H,d,J=15.6Hz), 10.40(1H,br s)

### Example 12

This was prepared in a manner similar to Example 11 to give (2E)-N-[3-chloro-5-(1,2-dimethyl-1H-imidazol-5-yl)phenyl]-3-(2-chlorophenyl)-2-propenamide.
MS:387(M+1)
NMR(DMSO, δ ):2.36(3H,s), 3.57(3H,s), 6.86(1H,d,J=15.7Hz), 6.96(1H,s), 7.22(1H, br s), 7.4-7.5(2H,m), 7.5-7.7(1H,m), 7.63(1H,br s), 7.7-7.9(1H,br s), 7.88(1H,br s), 7.91(1H,d,J=15.7Hz), 10.60(1H,br s)

### Example 13

This was prepared in a manner similar to Example 11 to give (2E)-N-[3-[[(2-chloro-4-pyridyl)amino]methyl]phenyl]-3-[2-(trifluoromethyl)phenyl]-2-propenamide.
MS:432(M+1)
NMR(DMSO, δ ):4.35(2H,d,J=5.9Hz), 6.52(1H,br s), 6.5-6.6(1H,m), 6.90(1H,d, J=15.4Hz), 7.06(1H,d,J=7.6Hz), 7.2-7.4(1H,m), 7.5-7.7(4H,m), 7.7-8.0(5H,m), 10.37 (1H,br s)

### Example 14

A solution of S-methyl benzothioimidate hydriodide (279mg), (2E)-N-(3-aminophenyl)- z3-(2-methoxyphenyl)-2-propenamide (134mg), and methanol (2ml) was refluxed for two hours. Cooled at room temperature, added dichloromethane (50ml), water (50ml), 1N sodium hydroxide (2ml), and filtered. Extracted with dichloromethane (20ml, twice). Combined organics were dried over magnesium sulfate, filtered, and evaporated. Purified by silica gel column chromatography, eluted with aqueous dichloromethan-methanol-ammonia, followed by recrystallization from ethyl acetate to give (2E)-N-[3-(iminobenzylamino)phenyl]-3-(2-methoxyphenyl)-2-propenamide (150mg) as white crystals.
mp:219-221°C
MS:372(M+1)
NMR(DMSO, δ ):3.89(3H,s), 6.43(2H,br s), 6.59(1H,d,J=6.9Hz), 6.88(1H,d,J=15.8Hz), 7.02(1H,dd,J=7.5Hz,7.5Hz), 7.10(1H,d,J=8.1Hz), 7.20-7.33(2H,m), 7.35-7.54(5H,m), 7.57(1H,d,J=7.6Hz), 7.79(1H,d,J=15.8Hz), 7.92-8.04(2H,m), 10.10(1H,s)
Elemental analysis:
Calculated+(H₂O)_{0.4}:C;72.96,H;5.80,N;11.10
Found:C;72.81,H;5.93,N;10.98

### Example 15

This was prepared in a manner similar to Example 11 to give N-[[3-(1 isoquinolylamino)methyl]phenyl]cinnamamide.
mp:216-218°C
IR(Nujol):3360,1660cm⁻¹
MS:380(M+1)
NMR(DMSO, δ ):4.76(2H,d,J=5.8Hz), 6.80(1H,d,J=15.7Hz), 6.90(1H,d,J=5.6Hz), 7.26(1H,dd,J=7.7Hz,7.7Hz), 7.40-7.74(12H,m), 7.82(1H,d,J=5.8Hz), 8.04(1H,t, J=5.9Hz), 8.32(1H,d,J=8.1Hz), 10.14(1H,s)

### Example 16

This was prepared in a manner similar to Example 14 to give (2E)-N-[2-fluoro-5-[[imino(2-thienyl)methyl]amino]phenyl]-3-(2-chlorophenyl)-2 propenamide.
mp:215-216°C
MS:400(M+1)
NMR(DMSO, δ ):6.49(2H,s), 6.54-6.65(1H,s), 7.08-7.25(3H,m), 7.38-7.79(7H,m), 7.87(1H,d,J=15.7Hz), 9.98(1H,s)
Elemental analysis:
Calculated:C;60.07,H;3.78,N;10.51
Found:C;59.98,H;3.57,N;10.42

### Example 17

This was prepared in a manner similar to Example 14 to give (2E)-N-[2-fluoro-5-(iminobenzylamino)phenyl]-3-(2-chlorophenyl)-2-propenamide.
mp:189-191°C
MS:394(M+1)
NMR(CDCl₃, δ ):6.4-6.7(3H,m), 7.14-7.29(2H,m), 7.40-7.61(6H,m), 7.69-8.00(5H,m), 9.99(1H,s)
Elemental analysis:
Calculated+(H₂O)_{0.3}:C;66.18,H;4.44,N;10.52
Found:C;66.21,H;4.16,N;10.39

### Example 18

This was prepared in a manner similar to Example 14 to give (2E)-N-[5-[[(5-chloro-2-methoxyphenyl)(imino)methyl]amino]-2-fluorophenyl]-3-(2-chlorophenyl)-2-propenamide.
mp:229-230°C
MS:458(M+1)
NMR(CDCl₃, δ ):3.93(3H,s), 5.70(2H,s), 6.59(1H,d,J=15.5Hz), 6.67-6.75(1H,m), 6.92(1H,d,J=8.9Hz), 7.11(1H,dd,J=10.7Hz,8.6Hz), 7.26-7.47(4H,m), 7.53(1H,s), 7.61-7.69(1H,m), 8.05-8.21(3H,m)
Elemental analysis:
Calculated+(H₂O)_{0.2}:C;60.28,H;3.96,N;9.17
Found:C;60.37,H;3.76,N;9.14

### Example 19

This was prepared in a manner similar to Example 14 to give (2E)-N-[3-[N¹-(1,3-benzothiazol-6-yl)amidino]phenyl]-3-(2-chlorophenyl)-2-propenamide.
mp:140-142°C
MS:433(M+1)
NMR(DMSO, δ ):6.48(2H,br s), 6.91(1H,d,J=15.6Hz), 7.05(1H,d,J=8.6Hz), 7.41-8.03 (10H,m), 8.30(1H,s), 9.18(1H,s), 10.48(1H,s)
Elemental analysis:
Calculated+(H₂O)_{1/3}(CH₃OH)₁:C;61.20,H;4.64,N;11.90
Found:C;61.11,H;4.47,N;11.91

### Example 20

This was prepared in a manner similar to Example 14 to give (2E)-N-[3-[N¹-[4-(1,3-thiazol-4-yl)phenyl]amidino]phenyl]-3-(2-chlorophenyl)-2-propenamide.
mp:179-180°C
MS:459(M+1)
NMR(DMSO, δ ):6.40(2H,br s), 6.85-6.99(3H,m), 7.38-7.70(5H,m), 7.74-8.06(6H,m), 8.29(1H,s), 9.18(1H,d,J=1.9Hz), 10.47(1H,s)
Elemental analysis:
Calculated+(H₂O)_{1.25}:C;62.36,H;4.50,N;11.64
Found:C;62.36,H;4.52,N;11.36

### Example 21

This was prepared in a manner similar to Example 14 to give (2E)-N-[3-[N¹-[4-(1H-pyrazol-1-yl)phenyl]amidino]phenyl]-3-(2-chlorophenyl)-2-propenamide.
mp:117-119°C
MS:442(M+1)
NMR(DMSO, δ ):6.41(2H,br s), 6.51(1H,s), 6.85-7.00(3H,s), 7.35-7.95(11H,m) 8.29(1H,s), 8.41(1H,s 10.47(1H,
Elemental analysis:
Calculated+(H₂O)_{1.1}:C;65.03,H;4.85,N;15.17
Found:C;64.82,H;4.81,N;15.10

### Example 22

This was prepared in a manner similar to Example 14 to give (2E)-N-[3-[N¹-[4-(1,3-oxazol-5-yl)phenyl]amidino]phenyl]-3-(2-chlorophenyl)-2-propenamide.
mp:115-117°C
MS:443(M+1)
NMR(DMSO, δ ):6.46(2H,br s), 6.90(1H,d,J=15.6Hz), 6.94(2H,m), 7.41-7.95(11H,m), 8.28(1H,s)8.38(1H,s), 10.46(1H,s)
Elemental analysis:
Calculated+(H₂O)_{0.8}(CH₃OH)₁:C;63.81,H;5.07,N;11.45
Found:C;63.81,H;4.83,N;11.51

### Example 23

This was prepared in a manner similar to Example 14 to give (2E)-N-[3-chloro-5-[[(5-fluoro-2-methoxyphenyl)(imino)methyl]amino]phenyl]-3-(2-chlorophenyl)-2-propenamide.
mp:238-239°C
MS:458,460(M+1,C1 isotopes)
NMR(CDCl₃, δ ):3.92(3H,s), 5.78(2H,br s), 6.53(1H,d,J=15.5Hz), 6.78(1H,s), 6.93 (1H,dd,J=9.0Hz,4.2Hz), 7.02-7.19(2H,m), 7.24-7.55(5H,m), 7.55-7.65(1H,m), 7.92 (1H,dd,J=7.7Hz,3.5Hz), 8.13(1H,d,J=15.5Hz)
Elemental analysis:
Calculated+(H₂O)_{0.4}:C;59.34,H;4.07,N;9.03
Found:C;59.32,H;4.12,N;8.75

### Example 24

This was prepared in a manner similar to Example 14 to give (2E)-N-[3-chloro-5-[[(5-chloro-2-methoxyphenyl)(imino)methyl]amino]phenyl]-3-(2-chlorophenyl)-2-propenamide.
mp:226-228°C
MS:474,476(M+1,C1 isotopes)
NMR(CDCl₃, δ ):3.93(3H,s), 5.72(2H,br s), 6.53(1H,d,J=15.6Hz), 6.79(1H,s), 6.93 (1H,d,J=9.0Hz), 7.08(1H,s), 7.22-7.46(6H,m), 7.58-7.70(1H,m), 8.14(1H,d,J=15.6Hz), 8.17(1H,s)
Elemental analysis:
Calculated:C;58.19,H;3.82,N;8.85
Found:C;58.20,H;3.73,N;8.78

### Example 25

This was prepared in a manner similar to Example 14 to give (2E)-N-[3-[N¹-(4-methoxyphenyl)amidino]phenyl]-3-(2-chlorophenyl)-2-propenamide.
mp:129-131°C
MS:406(M+1)
NMR(DMSO, δ ):3.74(3H,s), 6.19(2H,s), 6.75-6.96(5H,m), 7.35-7.50(3H,m), 7.53-7.65(2H,m), 7.74-7.80(1H,m), 7.84-7.95(2H,m), 8.26(1H,s), 10.45(1H,s)
Elemental analysis:
Calculated+(H₂O)_{0.25}:C;67.32,H;5.03,N;10.24
Found:C;67.00,H;4.97,N;10.24

### Example 26

This was prepared in a manner similar to Example 14 to give (2E)-N-(3-[N¹-[4-(1-methyl-1H-imidazol-4-yl)phenyl]amidino]phenyl]-3-(2-chlorophenyl)-2-propenamide.
mp:142-146°C
MS:456(M+1)
NMR(DMSO, δ ):3.68(3H,s), 6.30(2H,br s), 6.80-7.01(3H,m), 7.35-7.81(10H,m), 7.83-7.99(2H,m), 8.26(1H,s), 10.47(1H,s)
Elemental analysis:
Calculated+(H₂O)_{1.75}:C;64.06,H;5.27,N;14.37
Found:C;63.99,H;5.28,N;14.16

### Example 27

This was prepared in a manner similar to Example 14 to give N-[3-[imino(4-isopropylanilino)methyl]phenyl]-2-chlorocinnamamide.
mp:112-118°C
MS:418(M+1)
NMR(DMSO, δ ):1.21(6H,d,J=6.9Hz), 2.87(1H,septet,J=6.9Hz), 6.40(2H,br s), 6.81-6.98(3H,m), 7.20(2H,d,J=8.1Hz), 7.38-7.49(3H,m), 7.52-7.66(2H,m), 7.73-7.80(1H,m), 7.82-7.99(2H,m), 8.25(1H,s), 10.47(1H,s)
Elemental analysis:
Calculated+(H₂O)_{0.7}:C;69.74,H;5.95,N;9.76
Found:C;69.76,H;6.01,N;9.66

### Example 28

This was prepared in a manner similar to Example 14 to give (2E)-N-[3-[N¹-(4-oxo-4H-chromenyl-6-yl)amidino]phenyl]-3-(2-chlorophenyl)-2-propenamide.
mp:151-156°C
MS:444(M+1)
NMR(DMSO, δ ):6.31(1H,d,J=6.0Hz), 6.60(2H,br s), 6.81-6.99(2H,m). 7.20-8.00 (10H,m), 8.20-8.39(2H,m), 10.48(1H,s)
Elemental analysis:
Calculated+(H₂O)_{1.3}(Et₂O)_{0.1}:C;64.26,H;4.58,N;8.85
Found:C;64.25,H;4.47,N;8.58

### Example 29

This was prepared in a manner similar to Example 14 to give (2E)-N-[3-chloro-5-[[(5-chloro-2-methoxyphenyl)(imino)methyl]amino]phenyl]-3-[2-(trifluoromethyl)phenyl]-2-promenamide.
mp:227-229°C
MS:508,510(M+1,C1 isotopes)
NMR(CDCl₃, δ ):3.93(3H,s), 5.69(2H,s), 6.48(1H,d,J=14.8Hz), 6.79(1H,s), 6.93(1H,d, J=8.8Hz), 7.09(1H,s), 7.26-7.78(7H,m), 8.07-8.18(2H,m)
Elemental analysis:
Calculated:C;56.71,H;3.57,N;8.27
Found:C;56.36,H;3.58,N;8.18

### Example 30

This was prepared in a manner similar to Example 14 to give (2E)-N-[3-chloro-5-[[(5-fluoro-2-methoxyphenyl)(imino)methyl]amino]phenyl]-3-[2-(trifluoromethyl)phenyl]-2-propenamide.
mp:213-214°C
MS:492,494(M+1,C1 isotopes)
NMR(CDCl₃, δ ):3.92(3H,s), 5.79(2H,br s), 6.48(1H,d,J=15.3Hz), 6.78(1H,s), 6.93 (1H,dd,J=9.1Hz,4.3Hz), 7.02-7.19(2H,m), 7.39-7.63(4H,m), 7.69-7.77(2H,m), 7.89-7.99(1H,m), 8.05-8.19(1H,m)
Elemental analysis:
Calculated+(H₂O)_{1/2}(CH₃OH)₁:C;56.34,H;4.35,N;7.88
Found:C;56.26,H;4.28,N;7.85

### Example 31

This was prepared in a manner similar to Example 14 to give (2E)-N-[3-chloro-5-(iminobenzylamino)phenyl]-3-(2-chlorophenyl)-2-propenamide.
mp:105-115°C
MS:410(M+1)
NMR(DMSO, δ ):6.58(3H,br s), 6.86(1H,d,J=15.7Hz), 7.02(1H,s), 7.37-7.64(7H,m), 7.71-8.05(4H,m), 10.38(1H,br s)
Elemental analysis:
Calculated+(H₂O)_{0.2}:C;63.84,H;4.24,N;10.15
Found:C;63.84,H;4.06,N;10.08

### Example 32

This was prepared in a manner similar to Example 14 to give (2E)-N-[3-trifluoromethyl-5-[[imino(2-thienyl)methyl]amino]phenyl]-3-(2-chlorophenyl)-2-propenamide.
mp:206-208°C
MS:450(M+1)
NMR(DMSO, δ ):6.74(2H,br s), 6.83-6.92(2H,m), 7.12(1H,dd,J=3.7Hz,3.7Hz), 7.34(1H,s), 7.41-7.51(2H,m), 7.52-7.63(1H,m), 7.65(1H,d,J=4.7Hz), 7.75-7.99(4H,m), 10.54(1H,s)
Elemental analysis:
Calculated+(CH₃OH)_{0.5}(H₂O)_{0.5}:C;54.37,H;3.82,N;8.85
Found:C;54.08,H;3.71,N;8.56

### Example 33

This was prepared in a manner similar to Example 14 to give (2E)-N-[3-chloro-5-[[imino(2-thienyl)methyl]amino]phenyl]-3-(2-chlorophenyl)-2-propenamide.
mp:123-124°C
MS:416(M+1)
NMR(DMSO, δ ):6.59(1H,dd,J=1.8Hz,1.8Hz), 6.67(2H,br s), 6.86(1H,d,J=15.6Hz), 7.03(1H,s), 7.11(1H,dd,J=5.0Hz,3.7Hz), 7.41-7.49(2H,m), 7.52-7.67(3H,m), 7.72-7.81 (2H,m), 7.89(1H,d,J =15.7Hz), 10.38(1H,s)
Elemental analysis:
Calculated+(H₂O)_{0.9}:C;55.54,H;3.91,N;9.71
Found:C;55.54,H;4.02,N;9.50

### Example 34

This was prepared in a manner similar to Example 14 to give (2E)-N-[3-chloro-5-[[imino(2-thienyl)methyl]amino]phenyl]-3-[2-(trifluoromethyl)phenyl]-2-propenamide.
mp:178-179°C
MS:450(M+1)
NMR(DMSO, δ ):6.60(1H,dd,J=1.7Hz,1.7Hz), 6.68(2H,br s), 6.87(1H;d,J=15.4Hz), 7.03(1H,s), 7.11(1H,dd,J=4.9Hz,J=3.8Hz), 7.75-7.70(3H,m), 7.71-7.95(5H,m), 10.44 (1H,s)
Elemental analysis:
Calculated:C;56.07,H;3.36,N;9.34
Found:C;55.76,H;3.52,N;9.20

### Example 35

This was prepared in a manner similar to Example 14 to give (2E)-N-[3-chloro-5-(iminobenzylamino)phenyl]-3-[2-(trifluoromethyl)phenyl]-2-propenamide.
mp:137-139°C
MS:444(M+1)
NMR(DMSO, δ ):6.76(3H,br s), 6.87(1H,d,J=15.3Hz), 7.02(1H,s), 7.41-7.69(5H,m), 7.72-7.95(6H,m), 10.42(1H,s)
Elemental analysis:
Calculated+(H₂O)₁:C59.81,H;4.15,N;9.10
Found:C59.92,H;3.99,N;8.89

### Example 36

This was prepared in a manner similar to Example 14 to give (2E)-N-[3-cyano-5-(iminobenzylamino)phenyl]-3-[2-(trifluoromethyl)phenyl]-2-propenamide.
mp:181-182°C
MS:435(M+1)
NMR(DMSO, δ ):6.69(2H,br s), 6.88(1H,d,J=15.3Hz), 6.96(1H,s), 7.34-7.58(4H,m), 7.64(1H,t,J=7.6Hz), 7.76-8.03(7H,m), 10.59(1H,s).

### Example 37

This was prepared in a manner similar to Example 14 to give N-[3-(iminobenzylamino)phenyl]-3-(phenyl)-2-propenamide.
mp:156-158°C
MS:356(M+1)
NMR(DMSO, δ ):2.11(3H,s), 6.27(2H,s), 6.57(1H,d,J=7.7Hz), 7.21-7.58(12H,m), 7.91-8.03(2H,m), 9.84(1H,s)
Elemental analysis:
Calculated+(H₂O)_{1/4}:C;76,H;6.02,N;11.67
Found:C;76.75,H;5.88,N;11.52

### Example 38

This was prepared in a manner similar to Example 14 to give (2E)-N-[3-(iminobenzylamino)phenyl]-3-(4-methylphenyl)-2-propenamide.
mp:147-149°C
MS:356(M+1)
NMR(DMSO, δ ):2.34(3H,s), 6.36(2H,s), 6.58(1H,d,J=7.7Hz), 6.79(1H,d,J=15.7Hz), 7.20-7.60(11H,m), 7.89-8.02(2H,m), 10.08(1H,s)
Elemental analysis:
Calculated+(H₂O)_{1/4}:C;76.75,H;6.02,N;11.67
Found:C;76.79,H;5.80,N;11.71

### Example 39

This was prepared in a manner similar to Example 14 to give (2E)-N-[3-(iminobenzylamino)phenyl]-3-(3-methylphenyl)-2-propenamide.
mp:179-180°C
MS:356(M+1)
NMR(DMSO, δ ):2.35(3H,s), 6.29(2H,s), 6.56(1H,d,J=7.4Hz), 6.83(1H,d,J=15.6Hz), 7.21-7.59(11H,m), 7.93-8.01(2H,m), 10.09(1H,s)
Elemental analysis:
Calculated+(H₂O)_{4/5}:C;67.52,H;5.38,N;9.92
Found:C;67.61,H;5.27,N;10.08

### Example 40

This was prepared in a manner similar to Example 14 to give (2E)-N-[3-(iminobenzylamino)phenyl]-3-(2-methylphenyl)-2-propenamide.
mp:109-114°C
MS:356(M+1)
NMR(DMSO, δ ):2.41(3H,s), 6.29(2H,s), 6.57(1H,d,J=7.5Hz), 6.75(1H,d,J=15.6Hz), 7.19-7.50(9H,m), 7.56-7.61(1H,m 7.80(1H,d,J=15.6Hz), 7.93-8.01(2H,m), 10.13 (1H,s)
Elemental analysis:
Calculated+(H₂O)_{5/4}:C;73.09,H;6.27,N;11.12
Found:C;73.17,H;6.30,N;10.80

### Example 41

This was prepared in a manner similar to Example 14 to give (2E)-N-[5-[[(5-fluoro-2-methoxyphenyl)(imino)methyl]amino]phenyl]-3-(2-trifluoromethyl)-2-propenamide.
mp:220-221°C
MS:458(M+1)
NMR(DMSO, δ ):3.86(3H,s), 6.26(2H,s), 6.58(1H,d,J=7.2Hz), 6.79-7.42(6H,m), 7.58-7.70(2H,m), 7.76-7.98(4H,m), 10.27(1H,s)
Elemental analysis:
Calculated:C;63.02,H;4.19,N;9.19
Found:C;62.80,H;4.07,N;9.09

### Example 42

This was prepared in a manner similar to Example 14 to give (2E)-N-[3-[N¹-(phenyl)amidino]phenyl]-3-(2-chlorophenyl)-2-propenamide.
mp:82-84°C
MS:376(M+1)
NMR(DMSO, δ ):6.23(2H,s), 6.83-7.03(4H,m), 7.27-7.49(5H,m), 7.53-7.66(2H,m), 7.74-7.95(3H,m), 8.27(1H,s), 10.46(1H,s).
Elemental analysis:
Calculated+(H₂O)_{1.75}:C;64.86,H;5.32,N;10.31
Found:C;64.78,H;5.31,N;10.29

### Example 43

This was prepared in a manner similar to Example 11 to give N-[3-[[(1-methyl-1H-benzimidazol-2-yl)amino]methyl]phenyl]-3-(2-chlorophenyl)-2-propenamide.
mp:188-191°C
MS:417(M+1)
NMR(DMSO, δ ):3.56(3H,s), 4.59(2H,d,J=5.9Hz), 6.88(1H,d,J=15.7Hz), 6.90-7.00 (2H,m), 7.08-7.21(3H,m), 7.24-7.39(2H,m), 7.40-7.48(2H,m), 7.50-7.60(1H,m), 7.62-7.79(3H,m), 7.86(1H,d,J=15.7Hz), 10.29(1H,s)
Elemental analysis:
Calculated+(H₂O)_{0.33}:C;68.18,H;5.34,N;12.55
Found:C;68.04,H;5.26,N;12.53

### Example 44

This was prepared in a manner similar to Example 11 to give N-[6-[(2-pyridylmethyl)amino]-3-pyridyl]-3-(2-chlorophenyl)-2-propenamide.
mp:164-165°C
MS:365(M+1)
NMR(DMSO, δ ):4.55(2H,d,J=6.0Hz), 6.59(1H,d,J=8.9Hz), 6.82(1H,d,J=15.6Hz), 7.08(1H,t,J=5.8Hz), 7.19-7.35(2H,m), 7.39-7.46(2H,m), 7.51-7.59(1H,m), 7.65-7.76 (3H,m), 7.82(1H,d,J=15.6Hz), 8.23(1H,d,J=2.5Hz), 8.51(1H,d,J=4.4Hz), 10.06(1H,s)
Elemental analysis:
Calculated:C;65.84,H;4.70,N;15.36
Found:C;65.92,H;4.68,N;15.27

### Example 45

This was prepared in a manner similar to Example 11 to give N-[6-[(2-methyl-3-pyridyl)oxy]-3-pyridyl]-3-(2-chlorophenyl)-2-propenamide.
mp:187-189°C
MS:366(M+1)
NMR(DMSO, δ ):2.31(3H,s), 6.86(1H,d,J=15.7Hz), 7.15(1H,d,J=8.8Hz), 7.30(1H,dd, J=8.1Hz,4.7Hz), 7.41-7.60(4H,m), 7.74-7.81(1H,m), 7.89(1H,d,J=15.7Hz), 8.22(1H,dd, J=8.8Hz,2.7Hz), 8.33(1H,dd,J=4.6Hz,J=1.3Hz), 8.38(1H,d,J=2.6Hz), 10.50(1H,s)
Elemental analysis:
Calculated:C;65.67,H;4.41,N;11.49
Found:C;65.43,H;4.42,N;11.37

### Example 46

This was prepared in a manner similar to Example 14 to give (2E)-N-[3-(iminobenzylamino)phenyl]-3-[2-(trifluoromethyl)phenyl]-2-propenamide.
mp:82-84°C
MS:410(M+1)
NMR(DMSO, δ ):6.30(2H,br s), 6.59(1H,d,J=7.6Hz), 6.91(1H,d,J=15.3Hz), 7.18-7.50 (6H,m), 7.63(1H,dd,J=7.4Hz,7.4Hz), 7.71-8.03(6H,m), 10.26(1H,s)
Elemental analysis:
Calculated+(H₂O)_{0.7}:C;65.46,H;4.63,N;9.96
Found:C;65.47,H;4.60,N;9.86

### Example 47

This was prepared in a manner similar to Example 14 to give (2E)-N-[3-(iminobenzylamino)phenyl]-3-(4-chlorophenyl)-2-propenamide.
mp:109-110°C
MS:376(M+1)
NMR(DMSO, δ ):6.29(2H,br s), 6.56(1H,d,J=7.5Hz), 6.84(1H,d,J=15.7Hz), 7.19-7.57 (8H,m), 7.59-7.71(3H,m), 7.90-8.03(2H,m), 10.14(1H,s)
Elemental analysis:
Calculated(CH₃OH)₁:C;67.62,H;5.44,N;10.30
Found:C;67.54,H;5.39,N;10.24

### Example 48

This was prepared in a manner similar to Example 14 to give (2E)-N-[3-(iminobenzylamino)phenyl]-3-(3-methoxyphenyl)-2-propenamide.
mp:148-149°C
MS:372(M+1)
NMR(DMSO, δ ):3.81(3H,s), 6.44(2H,br s), 6.60(1H,d,J=7.1Hz), 6.84(1H,d,J=15.7Hz), 6.98(1H,d,J=9.1Hz), 7.16-7.49(9H,m), 7.54(1H,d,J=15.7Hz), 7.90-8.02(2H,m), 10.13 (1H,s)
Elemental analysis:
Calculated+(Et₂O)_{0.5}(H₂O)_{0.2}:C;72.86,H;6.46,N;10.20
Found:C;72.66,H;6.12,N;10.55

### Example 49

This was prepared in a manner similar to Example 14 to give N-[3-[[imino(1-methyl-1H-indole-5-yl)methyl]amino]phenyl]cinnamamide.
mp:143-148°C
MS:395(M+1)
NMR(DMSO, δ ):3.83(3H,s), 6.53(2H,br s), 6.64(2H,br s), 6.85(1H,d,J=15.5Hz), 7.22-7.59(9H,m), 7.60-7.70(2H,m), 7.85(1H,br s), 8.19(1H,s), 10.15(1H,s)
Elemental analysis:
Calculated+(H₂O)_{0.9}:C;73.11,H;5.84,N;13.64
Found:C;73.09,H;5.73,N;13.37

### Example 50

This was prepared in a manner similar to Example 11 to give N-[3-[(2-quinolylamino)methyl]phenyl]-3-(2-chlorophenyl)-2-propenamide.
mp:170-171°C
MS:414,416(M+1,C1 isotopes)
NMR(DMSO, δ ):4.65(2H,d,J=5.7Hz), 6.84(1H,d,J=8.9Hz), 6.88(1H,d,J=15.7Hz), 7.05-7.21(2H,m), 7.30(1H,dd,J=8.0Hz,8.0Hz), 7.35-7.93(12H,m), 10.28(1H,s)
Elemental analysis:
Calculated+(H₂O)_{0.2}:C;71.92,H;4.92,N;10.06
Found:C;71.91,H;4.82,N;9.99

### Example 51

This was prepared in a manner similar to Example 14 to give N-[3-[[[4-(dimethylamino)phenyl](imino)methyl]amino]phenyl]cinnamamide.
mp:225-227°C
MS:385(M+1)
NMR(DMSO, δ ):2.98(6H,s), 6.39(2H,br s), 6.60(1H,d,J=7Hz), 6.73(2H,d,J=8.9Hz), 6.85(1H,d,J=15.6Hz), 7.20-7.70(9H,m), 7.81(2H,d,J=8.4Hz), 10.13(1H,s)
Elemental analysis:
Calculated+(H₂O)_{3/4}:C;72.43,H;6.46,N;14.08
Found:C;72.54,6.24,N;14.00

### Example 52

This was prepared in a manner similar to Example 14 to give (2E)-N-[5-[[(3-methylphenyl)](imino)methyl]amino]-2-fluorophenyl]cinnamamide.
mp:105-110°C
MS:356(M+1)
NMR(DMSO, δ ):2.38(3H,s), 6.65(2H,m), 6.85(1H,d,J=15.7Hz), 7.22-7.84(14H,m), 10.18(1H,s)
Elemental analysis:
Calculated+(H₂O)_{1.25}:C;73.09,H;6.27,N;11.12
Found:C;73.14,5.97,N;10.84

### Example 53

This was prepared in a manner similar to Example 14 to give (2E)-N-[3-(iminobenzylamino)phenyl]-3-(3-chlorophenyl)-2-propenamide.
mp:260-262°C
MS:376(free+1)
NMR(DMSO, δ ):6.98(1H,d,J=15.8Hz), 7.18(1H,d,J=7.1Hz), 7.45-7.84(10H,m), 7.90-8.01(3H,m), 9.11(1H,br s), 9.81(1H,br s), 10.70(1H,s), 11.51(1H s)
Elemental analysis:
Calculated:C;64.09,H;4.64,N;10.19
Found:C;63.74,4.56,N;10.06

### Example 54

This was prepared in a manner similar to Example 14 to give (2E)-N-[3-(iminobenzylamino)phenyl]-3-(2-chlorophenyl)-2-propenamide.
mp:262-264°C
MS:376(free+1)
NMR(DMSO, δ ):7.01(1H,d,J=15.7Hz), 7.22(1H,d,J=7.1Hz), 7.39-8.01(13H,m), 9.13 (1H,br s), 9.82(1H,br s), 10.81(1H,s), 11.55(1H,br s)
Elemental analysis:
Calculated+(H₂O)_{0.1}:C;63.81,H;4.67,N;10.15
Found:C;63.64,4.57,N;10.05

### Example 55

This was prepared in a manner similar to Example 14 to give (2E)-N-[3-(N¹-(2-fluorophenyl)amidino]phenyl]-3-(2-chlorophenyl)-2-propenamide hydrochloride.
mp:178-180°C
MS:394(free+1)
NMR(DMSO, δ ):7.02(1H,d,J=15.6Hz), 7.31-7.71(9H,m), 7.73-7.81(1H,m 7.95-8.03 (2H,m), 8.43(1H,s), 9.21(1H,s 10.05(1H,s), 10.95(1H,s), 11.60(1H,s)

### Example 56

This was prepared in a manner similar to Example 14 to give (2E)-N-[3-chloro-5-[[imino(3-thienyl)methyl]amino]phenyl]-3-[2-(trifluoromethyl)phenyl]-2-propenamide.
mp:121-123°C
MS;450(M+1)
NMR(DMSO, δ ):6.48(2H,s), 6.57(1H,s), 6.87(1H,d,J=15.4Hz), 6.99(1H,s), 7.50-7.70 (4H,m), 7.73-7.96(4H,m), 8.14(1H,s), 10.41(1H,s).

### Example 57

This was prepared in a manner similar to Example 14 to give (2E)-N-[3-[N¹-(phenyl)amidino]phenyl]-3-(2-chlorophenyl)-2-propenamide.
mp:218-220°C
MS:376(M+1)
NMR(DMSO, δ ):6.19(2H,s), 6.81-7.05(4H,m), 7.31(2H,t,J=7.6Hz), 7.41-7.63(3H,m), 7.70-8.04(6H,m), 10.50(1H,s)

### Example 58

This was prepared in a manner similar to Example 14 to give N-[3-(1H-benzimidazol-2-yl)phenyl]-3-(2-chlorophenyl)-2-propenamide.
mp:152-154°C
MS:374(M+1)
NMR(DMSO, δ ):6.94(1H,d,J=15.7Hz), 7.18-7.27(2H,m), 7.42-7.63(6H,m), 7.76-7.90 (3H,m), 7.94(1H,d,J=15.7Hz), 8.63(1H,s), 10.54(1H,s), 12.94(1H,s)

### Example 59

This was prepared in a manner similar to Example 14 to give N-[3-chloro-5-[[imino[4-[(2-methyl-3-pyridyl)oxy]phenyl]methyl]amino]phenyl]-3-(2-chlorophenyl)-2-propenamide.
mp:105-107°C
MS:517(M+1)
NMR(DMSO, δ ):2.39(3H,s), 6.57(3H,br s), 6.86(1H,d,J=15.6Hz), 7.00-7.09(3H,m), 7.28-7.62(6H,m), 7.75-7.81(1H,m), 7.88(1H,d,J=15.6Hz), 7.95-8.04(2H,m), 8.32-8.36 (1H,m), 10.37(1H,s)

### Example 60

This was prepared in a manner similar to Example 14 to give N-[3-chloro-5-[[imino[4-[(2-pyridylmethyl)amino]phenyl]methyl]amino]phenyl]-3-(2-chlorophenyl)-2-propenamide.
mp:119-122°C
MS:516(M+1)
NMR(DMSO, δ ):4.42(2H,d,J=5.9Hz), 6.20(2H,s), 6.50-6.62(3H,m), 6.78(1H,t, J=6.2Hz), 6.86(1H,d,J=15.6Hz), 6.96(1H,s), 7.22-7.80(10H,m), 7.88(1H,d,J=15.6Hz), 8.54(1H,d,J=4.2Hz), 10.33(1H,s)

### Example 61

This was prepared in a manner similar to Example 14 to give (2E)-N-[3-(iminobenzylamino)-2-fluorophenyl]-3-[2-(trifluoromethyl)phenyl]-2-propenamide.
mp:185-187°C
MS:428(M+1)
NMR(CDCl₃, δ ):4.73(2H,br s), 6.56(1H,d,J=15.3Hz 6.70-6.82(1H,m 7.10(1H,d J=10.7Hz,8.7Hz), 7.38-7.96(10H,m), 8.02-8.12(2H,m)

### Example 62

This was prepared in a manner similar to Example 14 to give (2E)-N-[2-fluoro-5-[[imino(2-thienyl)methyl]amino]phenyl]-3-[2-(trifluoromethyl)phenyl]-2-propenamide.
mp:208-209°C
MS:434(M+1)
NMR(CDCl₃, δ ):4.96(2H,s), 6.54(1H,d,J=15.3Hz), 6.70-6.81(1H,m), 7.07-7.19 (2H,m), 7.38-7.66(5H,m), 7.70-7.81(2H,m), 8.08-8.24(2H,m)

### Example 63

This was prepared in a manner similar to Example 14 to give N-[3-chloro-5-[[imino[(4-(1H-pyrazol-1-yl)phenyl]methyl]amino]phenyl]-3-[2-(trifluoromethyl) phenyl]-2-propenamide.
mp:146-153°C
MS:510(M+1)
NMR(DMSO, δ ):6.58-6.70(4H,m), 6.87(1H,d,J=15.5Hz), 7.05(1H,s), 7.58-7.70 (2H,m), 7.78-8.17(9H,m), 8.60(1H,d,J=2.5Hz), 10.43(1H,s)

### Example 64

This was prepared in a manner similar to Example 14 to give N-[3-chloro-5-[[imino[4-[(2-methyl-3-pyridyl)oxy]phenyl]methyl]amino]phenyl]-3-[2-(trifluoromethyl)phenyl]-2-propenamide.
mp:114-119°C
MS:551(M+1)
NMR(DMSO, δ ):2.39(3H,s), 6.49-6.63(3H,m), 6.86(1H,d,J=15.3Hz) 6.96-7.03 (3H,m), 7.31(1H,dd,J=8.1Hz,4.5Hz), 7.42(1H,d,J=7.9Hz), 7.56-7.70(2H,m), 7.76-8.08 (6H,m), 8.34(1H,d,J=4.5Hz), 10.42(1H,s)

### Example 65

This was prepared in a manner similar to Example 14 to give N-[3-chloro-5-[[imino[4-[(1-oxido-3-pyridyl)oxy]phenyl]methyl]amino]phenyl]-3-(2-chlorophenyl)-2-propenamide.
mp:214-216°C
MS:519(M+1)
NMR(DMSO, δ ):6.58(3H,s), 6.86(1H,d,J=15.6Hz), 7.00-7.09(2H,m), 7.21(2H, d,J=8.7Hz), 7.39-7.54(3H,m), 7.56-7.64(2H,m), 7.76-7.81(1H,m), 7.89(1H,d, J=15.6Hz), 7.98-8.20(4H,m), 10.38(1H,s)

### Example 66

This was prepared in a manner similar to Example 14 to give N-[3-[[[4-(4-acetyl-1-piperazinyl)phenylimino]methyl]amino]-5-chlorophenyl-3-(2-chlorophenyl)-2-propenamide.
mp:259-260°C
MS:536(M+1)
NMR(DMSO, δ ):2.05(3H,s), 3.15-3.38(4H,m), 3.50-3.66(4H,m), 6.36(2H,br s), 6.554 (1H,s), 6.86(1H,d,J=15.6Hz), 6.93-7.08(3H,m), 7.40-7.51(2H,m), 7.52-7.62(2H,m), 7.75-7.94(4H,m), 10.35(1H,s)

### Example 67

This was prepared in a manner similar to Example 14 to give (2E)-N-[3-chloro-5-(iminobenzylamino)phenyl]-3-(2-cyanophenyl)-2-propenamide.
mp:220-221°C
MS:401(M+1)
NMR(DMSO, δ ):6.59(3H,s), 7.02(1H,d,J=15.6Hz), 7.03(1H,s), 7.39-7.57(3H,m), 7.58-7.68 z(2H,m), 7.76-8.08(6H,m), 10.48(1H,s)

### Example 68

This was prepared in a manner similar to Example 14 to give (2E)-N-[3-fluoro-5-(iminobenzyl)phenyl]-3-(2-pyridyl)-2-propenamide.
mp:205-207°C
MS:377(M+1)
NMR(DMSO, δ ):6.57(3H,br s), 7.05(1H,s), 7.31(1H,d,J=15.3Hz), 7.37-7.49(4H,m), 7.57-7.70(3H,m), 7.80-8.05(3H,m), 8.65(1H,d,J=4.4Hz), 10.41(1H,s).

### Example 69

To a solution of 3-(3-chlorophenyl) acrylic acid (18mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give (2E)-3-(3-chlorophenyl)-N-[3-(1,2-dimethyl-1H-imidazol-5-yl) phenyl]-2-propenamide (27mg).
APCl-mass;m/z352(M+H⁺)

### Example 70

To a solution of 3-(4-chlorophenyl)acrylic acid (18mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric (0.1N) and brine. The separated organic layer was dried over magnesium sulfate and evaporated to give (2E)-3-(4-chlorophenyl)-N-[3-(1,2-dimethyl-1H-imidazol-5-yl) phenyl]-2-propenamide (27mg).
APCl-mass;m/z352(M+H⁺)

### Example 71

To a solution of 3-(2,4-dichlorophenyl)acrylic acid (22mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give (2E)-3-(2,4-dichlorophenyl)-N-[3-(1,2-dimethyl-1H-imidazol-5-yl) phenyl]-2-propenamide (32mg).
APCl-mass;m/z386(M+H⁺)

### Example 72

To a solution of 3-biphenyl-4-yl-acrylic acid (22mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give 3-biphenyl-4-yl-N-[3-(2,3-dimethyl-3H-imidazol-4-yl)phenyl] acrylamide (26mg).
APCl-mass;m/z394(M+H⁺)

### Example 73

To a solution of 3-(3,5-difluorophenyl)acrylic acid (18mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give (2E)-3-(3,5-difluorophenyl)-N-[3-(1,2-dimethyl-1H-imidazol-5-yl) phenyl]-2-propenamide (30mg).
APCl-mass;m/z354(M+H⁺)

### Example 74

To a solution of 3-(2,5-difluorophenyl)acrylic acid (18mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give (2E)-3-(2,5-difluorophenyl)-N-[3-(1,2-dimethyl-1H-imidazol-5-yl) phenyl]-2-propenamide (28mg).
APCl-mass;m/z354(M+H⁺)

### Example 75

To a solution of 3-(4-trifluoromethylphenyl)acrylic acid (22mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give (2E)-3-(4-trifluoromethylphenyl)-N-[3-(1,2-dimethyl-1H-imidazol-5-yl)phenyl]-2-propenamide (27mg) .
APCl-mass;m/z386(M+H⁺)

### Example 76

To a solution of 3-[2,5-bis(trifluoromethyl)phenyl]acrylic acid (28mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give (2E)-3-[2,5-bis(trifluoromethyl)phenyl]-N-[3-(1,2-dimethyl-1H-imidazol-5-yl)phenyl]-2-propenamide (33mg).
APCl-mass;m/z454(M+H⁺)

### Example 77

To a solution of 3-phenylsulfanyl-acrylic acid (18mg) in N,N-dimethylacetamide (3ml) was added O-(7-azabenzotriazol-1-yl)-N,N,N',N'-tetramethyluronium hexafluorophosphonate (57mg) and the mixture was stirred for 30 minutes. To the mixture was added 3-(2,3-dimethyl-3H-imidazol-4-yl)aniline (19mg) and the mixture was stirred for 1 hour. The mixture was diluted with ethyl acetate and washed with aqueous solution of sodium hydroxide (1N) and hydrochloric acid (0.1N) and brine. The separated organic layer was dried over magnesium sulfate and evaporated to give N-[3-(2,3-dimethyl-3H-imidazol-4-yl)phenyl]-3-phenylsulfanyl-acrylamide (23mg).
APCl-mass;m/z350(M+H⁺)

### Example 78

To a suspension of 3-(4,5-dimethylimidazol-1-yl)aniline (94mg) and 3-(2-chloro-4-fluorophenyl)acrylic acid in dichloromethane (5ml) was added 1-ethyl-3-(3-dimethylaminopropyl)carbodiimide hydrochloride (144mg) and N,N-dimethylaminopyridine (31mg) and the mixture was stirred for 24 hours. The mixture was diluted with 10% methanol in dichloromethane and washed with water, aqueous sodium hydroxide (1N) and brine. The separated organic layer was dried over magnesium sulfate and evaporated. The residue was triturated with methanol, collected by filtration and dried to give (2E)-3-(2-chloro-4-fluorophenyl)-N-[3-(4,5-dimethyl-1H-imidazol-1-yl)phenyl]-2-propenamide (95mg).
APCl-mass;m/z370(M+H⁺)
NMR(DMSO, δ ):2.11(3H,s), 6.85(1H,d,J=15.6Hz), 7.12(1H,d,J=8.1Hz), 7.36(1H,dt, J=2.6Hz,8.5Hz), 7.50(1H,t,J=8.0Hz), 7.6-7.9(3H,m), 7.8-8.0(3H,m), 10.56(1H,s)

### Example 79

To a suspension of 3-(4,5-dimethylimidazol-1-yl)aniline (78mg) and 3-(2,3-dichlorophenyl)acrylic acid (90mg) in dichloromethane (5ml) was added 1-ethyl-3-(3-dimethylaminopropyl)carbodiimide hydrochloride (120mg) and N,N-dimethylaminopyridine (25mg) and the mixture was stirred for 24 hours. The mixture was diluted with 15% methanol in dichloromethane and washed with water, aqueous sodium hydroxide (1N) and brine. The separated organic layer was dried over magnesium sulfate and evaporated. The residue was triturated with methanol, collected by filtration and dried to give (2E)-3-(2,3-dichlorophenyl)-N-[3-(4,5-dimethyl-1H-imidazol-1-yl)phenyl]-2-propenamide (93mg).
APCl-mass;m/z386,388(M+H⁺)
NMR(DMSO, δ ):2.11(6H,s), 6.93(1H,d,J=15.6Hz), 7.13(1H,d,J=8.7Hz), 7.4-7.6 (2H,m), 7.6-7.9(5H,m), 7.90(1H,d,J=15.6Hz), 10.60(1H,s)

### Example 80

To a suspension of 3-(4,5-dimethylimidazol-1-yl)aniline (94mg) and 3-[2-(trifluoromethyl)phenyl]acrylic acid (109mg) in dichloromethane (5ml) was added 1-ethyl-3-(3-dimethylaminopropyl)carbodiimide hydrochloride (144mg) and N,N-dimethylaminopyridine (31mg) and the mixture was stirred for 24 hours. The mixture was diluted with 15% methanol in dichloromethane and washed with water, aqueous sodium hydroxide (1N) and brine. The separated organic layer was dried over magnesium sulfate and evaporated. The residue was triturated with methanol, collected by filtration and dried to give (2E)-3-[2-(trifluoromethyl)phenyl]-N-[3-(4,5-dimethyl-1H-imidazol-1-yl)phenyl]-2-propenamide (120mg).
APCl-mass;m/z386(M+H⁺)
NMR(DMSO, δ ):2.11(6H,s), 6.90(1H,d,J=15.2Hz), 7.14(1H,d,J=7.0Hz), 7.51(1H,t, J=8.0Hz), 7.6-8.0(8H,m), 10.61(1H,s)

### Example 81

To a suspension of 3-(2,3-dimethyl-3H-imidazol-4-yl)phenylamine (78mg) and 3-(2,3-dichlorophenyl)acrylic acid (90mg) in dichloromethane (4ml) was added 1-ethyl-3-(3-dimethylaminopropyl)carbodiimide hydrochloride (120mg) and N,N-dimethylaminopyridine (25mg) and the mixture was stirred for 24 hours. The mixture was diluted with 15% methanol in dichloromethane and washed with water, aqueous sodium hydroxide (1N) and brine. The separated organic layer was dried over magnesium sulfate and evaporated. The residue was triturated with methanol, collected by filtration and dried to give (2E)-3-(2,3-dichlorophenyl)-N-[3-(1,2-dimethyl-1H-imidazol-5-yl)phenyl]-2-propenamide (44mg).
APCl-mass;m/z386,388(M+H⁺)
NMR(DMSO, δ ):2.36(3H,s), 3.56(3H,s), 6.87(1H,s), 6.91(1H,d,J=15.6Hz), 7.15(1H,d, J=7.6Hz), 7.4-7.6(2H,m), 7.6-7.9(5H,m), 7.89(1H,d,J=15.6Hz), 10.46(1H,s)

### Example 82

To a suspension of 3-(2-chlorophenyl)acrylic acid (92mg) and O-(7-azabenzotriazol-1-yl)-N,N,N',N'-tetramethyluronium hexafluorophosphonate (286mg) in N,N-dimethylformamide (5ml) was added 3-(4,5-dimethylimidazol-1-yl) aniline (94mg) and the mixture was stirred for 6 hours. The mixture was diluted with ethyl acetate and washed with water, aqueous sodium hydroxide (1N) and brine. The separated organic layer was dried over magnesium sulfate and evaporated. The residue was triturated with ethyl acetate, collected by filtration and dried to give (2E)-3-(2-chlorophenyl)-N-[3-(4,5-dimethyl-1H-imidazol-1-yl)phenyl]-2-propenamide (138mg).
APCl-mass;m/z352,254(M+H⁺)
NMR(DMSO, δ ):2.11(3H,s), 2.12(3H,s), 6.89(1H,d,J=15.6Hz), 7.12(1H,d,J=8.7Hz), 7.4-7.9(8H,m), 7.90(1H,d,J=15.6Hz), 10.57(1H,s)

### Example 83

To a mixture of 4-chlorocinnamic acid (142mg) and oxalyl chloride (0.136ml) in dichloromethane (3ml) was added small amount of N,N-dimethylformamide. The mixture was stirred for 2 hours under nitrogen atmosphere and concentrated in vacuo, and diluted with dichloromethane (3ml) to give an acid chloride solution. To a solution of 5-(4,5-dimethyl-1H-imidazol-1-yl)-2-fluoroaniline (160mg) in dichloromethane (2ml) was added the acid chloride solution and 4-(dimethylamino) pyridine (one potion). The mixture was stirred for 2 hours under nitrogen atmosphere. The mixture was diluted with dichloromethane. The separated organic layer was washed with water and brine, and concentrated in vacuo. The crude solid was triturated with dichloromethane to give (2E)-3-(2-chlorophenyl)-N-[5-(4,5-dimethyl-1H-imidazol-1-yl)-2-fluorophenyl]-2-propenamide (137mg).
APCl-mass;m/z370(M+1)
NMR(DMSO, δ ):2.15(3H,s), 2.37(3H,s), 7.23(1H,d,J=15.5Hz), 7.40-7.67(5H,m), 7.76-7.81(1H,m), 7.92(1H,d,J=15.5Hz), 8.43(1H,dd,J=6.8Hz,2.5Hz), 9.27(1H,s), 10.5 (1H,s)

### Example 84

N-[5-chloro-6-[(2-pyridylmethyl)amino]-3-pyridyl]-2,3-dimethyl-1H-indole-7-carboxamide was obtained according to the similar manner to that of Example 161.
APCI-mass;m/z406(M+H+)
¹H-NMR(DMSO-d6): δ ;2.18(3H,s), 2.35(3H,s), 4.68(2H,d,J=5.7HZ), 6.9-7.1(2H,m), 7.2-7.4(2H,m), 7.5-7.8(3H,m), 8.21(1H,d,J=2.2Hz), 8.28(1H,d,J=2.2Hz), 8.52(1H,d, J=4.0Hz), 10.15(1H,s 10.73(1H,s)

### Example 85

N-[5-chloro-6-[(2-pyridylmethyl)amino]-3-pyridyl]-4'-fluoro[1,1'-biphenyl]-3-carboxamide was obtained according to the similar manner to that of Example 161.
APCI-mass;m/z433(M+H⁺)
¹H-NMR(DMSO-d6): δ ;4.68(2H,d,J=5.7Hz), 7.0-7.5(5H,m), 7.6-8.0(6H,m), 8.11(1H, d,J=2.4Hz), 8.19(1H,s), 8.26(1H,d,J=2.4Hz), 8.52(1H,d,J=4.8Hz), 10.27(1H,s)

### Example 86

N-[5-chloro-b-[(2-pyridylmethyl)amino]-3-pyridyl]-3-(2-thienyl)benzamide was obtained according to the similar manner to that of Example 161.
APCI-mass;m/z421.40(M+H⁺)
¹H-NMR(DMSO-d6): δ ;4.68(2H,d,J=5.7Hz), 7.08(1H,t,J=5.7Hz), 7.1-7.3(3H,m), 7.5-7.9(5H,m), 8.10(1H,d,J=2.2Hz), 8.17(1H,s) 8.26(1H,d,J=2.2Hz), 8.52(1H,d,J=4.8Hz), 10.28(1H,s)

### Example 87

N-[5-chloro-6-[(2-pyridylmethyl)amino]-3-pyridyl]-5-phenyl-2-thiophenecarboxamide was obtained according to the similar manner to that of Example 161.
APCI-mass;m/z420.93(M+H⁺)
¹H-NMR(DMSO-d6): δ ;4.68(2H,d,J=5.7Hz), 7.09(1H,t,J=5.7Hz), 7.2-7.6(5H,m), 7.62(1H,d,J=3.9Hz), 7.7-7.8(3H,m), 7.93(1H,d,J=3.9Hz), 8.05(1H,d,J=2.2Hz), 8.21 (1H,d,J=2.2Hz), 8.52(1H,d,J=4.4Hz), 10.21(1H,s)

### Example 88

N-[5-chloro-6-[(2-pyridylmethyl)amino]-3-pyridyl]-5-(4-fluorophenyl)-2-thiophenecarboxamide was obtained according to the similar manner to that of Example 161.
APCI-mass;m/z439(M+H⁺)
¹H-NMR(DMSO-d6): δ ;4.68(2H,d,J=5.7Hz), 7.09(1H,t,J=5.7Hz), 7.2-7.4(4H,m), 7.58 (1H,d,J=4.0Hz), 7.7-7.9(3H,m), 7.92(1H,d,J=4.0Hz), 8.04(1H,d,J=2.3Hz), 8.20(1H,d, J=2.3Hz), 8.52(1H,d,J=4.0Hz), 10.21(1H,s)

### Example 89

N-[5-chloro-6-[(2-pyridylmethyl)amino]-3-pyridyl]-9H-carbazol-1-carboxamide was obtained according to the similar manner to that of Example 161.
APCI-mass;m/z428(M+H⁺)
¹H-NMR(DMSO-d6): δ ;4.70(2H,d,J=5.7Hz), 7.08(1H,t,J=5.7Hz), 7.2-7.5(5H,m), 7.6-7.8(2H,m), 8.06(1H,d,J=7.7Hz), 8.16(1H,d,J=7.7Hz), 8.2-8.6(4H,m), 10.33(1H,s)

### Example 90

N-[5-chloro-6-[(2-pyridylmethyl)amino]-3-pyridyl]-6-fluoro-9H-carbazol-1-carboxamide was obtained according to the similar manner to that of Example 161.
APCI-mass;m/z446(M+H⁺)
¹H-NMR(DMSO-d6): δ ;4.70(2H,d,J=5.7Hz), 7.08(2H,d,J=5.7Hz), 7.2-7.4(4H,m), 7.6-7.8(2H,m), 8.0-8.1(1H,m), 8.08(1H,d,J=7.6Hz), 8.25(1H,d,J=2.2Hz), 8.31(1H,d, J=2.1Hz), 8.38(1H,d,J=7.6Hz), 8.53(1H,d,J=4.9Hz), 10.34(1H,s), 11.54(1H,s)

### Example 91

To a suspension of 9H-fluorene-1-carboxylic acid (147mg) in dichloromethane (3ml) was added oxalyl chloride (0.2ml) and N,N-dimethylformamide (0.01ml). The mixture was stirred for 2 hours and evaporated to give crude 9H-fluorene-1-carboxylic acid chloride. To a suspension of 4-(pyridine-2-ylmethyloxy)aniline dihydrochloride (191mg) and pyridine (0.283ml) in dichloromethane (3ml) was added a solution of crude 9H-fluorene-1-carboxylic acid chloride (obtained above) in dichloromethane (3ml) and stirred for 6 hours. The mixture was diluted with dichloromethane and washed with water and brine. The separated organic layer was dried over potassium carbonate and evaporated. The residue was triturated with methanol, collected by filtration, washed with methanol and isopropyl ether and dried to give N-[4-(2-pyridylmethoxy)phenyl]-9H-fluorene-1-carboxamide (151mg).
APCI-mass;m/z393(M+H⁺)
¹H-NMR(DMSO-d6): δ ;4.18(2H,s), 5.19(2H,s), 7.04(2H,d,J=9.1Hz), 7.3-7.8(9H,m), 7.85(1H,t,J=7.8Hz), 7.97(1H,d,J=6.7Hz), 8.09(1H,dd,J=7.5,0.9Hz), 8.59(1H,d,
J=7.5Hz), 10.22(1H,s)

### Example 92

To a suspension of N-(4-aminophenyl)-9H-fluorene-1-carboxamide (150mg) and 2-formylpyridine (54mg) and acetic acid (0.03ml) in 1,2-dichloroethane (5ml) was added sodium tri(acetoxy)borohydride (148mg) and the mixture was stirred for 2 hours. The mixture was diluted with dichloromethane and washed with aqueous sodium hydrogencarbonate (saturated), water and brine. The separated organic layer was dried over magnesium sulfate and evaporated. The residue was purified with column chromatography (silica gel, 25g, 2% methanol in dichloromethane) to give N-[4-[(2-pyridylmethyl)amino]phenyl]-9H-fluorene-1-carboxamide (61mg).
APCI-mass;m/z392(M+H⁺)
¹H-NMR(DMSO-d6): δ ;4.16(2H,s), 4.37(1H,d,J=6.1Hz), 6.25(1H,t,J=6.1Hz), 6.58 (2H,d,J=8.8Hz), 7.2-7.8(10H,m), 7.95(1H,d,J=6.7Hz), 8.06(1H,d,J=6.7Hz), 8.53(1H,d, J=4.0Hz), 9.95(1H,s)

### Example 93

N-[6-(2-pyridylmethoxy)-3-pyridyl]-9H-fluorene-1-carboxamide was obtained according to the similar manner to that of Example 91.
APCI-mass;m/z394(M+H⁺)
¹H-NMR(DMSO-d6): δ ;4.20(2H,s), 5.43(2H,s), 7.00(2H,d,J=8.9Hz), 7.3-7.9(8H,m), 7.98(1H,d,J=6.6Hz), 8.1-8.2(2H,m), 8.53(1H,d,J=2.5Hz), 8.57(1H,d,J=4.4Hz), 10.39 (1H,s)

### Example 94

N-[6-(2-pyridylmethoxy)-3-pyridyl)-9H-carbazol-1-carboxamide was obtained according to the similar manner to that of Example 161.
APCI-mass;m/z395(M+H⁺)
¹H-NMR(DMSO-d6): δ ;5.44(2H,s), 7.03(1H,d,J=8.9Hz), 7.2-7.6(5H,m), 7.69(1H,t, J=7.2Hz), 7.83(1H,t,J=7.2Hz), 8.09(1H,d,J=7.5Hz), 8.2-8.3(2H,m), 8.38(1H,d, J=7.5Hz), 8.5-8.7(2H,m)10.45(1H,s), 11.49(1H,s)

### Example 95

N-[6-[(2-pyridylmethyl)amino]-3-pyridyl]-9H-fluorene-1-carboxamide was obtained according to the similar manner to that of Example 161.
APCI-mass;m/z393(M+H⁺)
¹H-NMR(DMSO-d6): δ ;4.18(2H,s), 4.57(2H,d,J=6.0Hz), 6.61(1H,d,J=8.9Hz), 7.08 (1H,t,J=6.1Hz), 7.2-7.8(9H,m), 7.96(1H,d,J=6.5Hz), 8.09(1H,d,J=7.4Hz), 8.28(1H,d, J=2.4Hz), 8.51(1H,d,J=4.7Hz), 10.06(1H,s)

### Example 96

N-[6-[(2-pyridylmethyl)amino]-3-pyridyl]-9H-carbazol-1-carboxamide was obtained according to the similar manner to that of Example 161.
APCI-mass;m/z394(M+H⁺)
¹H-NMR(DMSO-d6): δ ;4.58(2H,d,J=6.0Hz), 6.63(1H,d,J=8.9Hz), 7.08(1H,t,J=6.0Hz), 7.1-7.5(5H,m), 7.6-7.9(3H,m), 8.05(1H,d,J=7.6Hz), 8.16(1H,d,J=7.6Hz), 8.2-8.3 (2H,m), 8.52(1H,d,J=4.8Hz), 10.16(1H,s), 11.44(1H,s)

### Example 97

N-[6-[(2-methyl-3-pyridyl)oxy]-3-pyridyl]-9H-carbazol-1-carboxamide was obtained according to the similar manner to that of Example 161.
APCI-mass;m/z395(M+H⁺)
¹H-NMR(DMSO-d6): δ ;2.34(3H,s), 7.1-7.3(4H,m), 7.42(1H,t,J=7.6Hz), 7.53(1H,d, J=8.0Hz), 7.71(1H,d,J=8.0Hz), 8.10(1H,d,J=7.5Hz), 8.17(1H,d,J=7.7Hz), 8.3-8.5 (3H,m), 8.56(1H,s), 10.55(1H,s), 11.49(1H,s)

### Example 98

3-methyl-N-[6-[(2-pyridylmethyl)amino]-3-pyridyl]-2-(trifluoromethyl)-1H-indole-7-carboxamide was obtained according to the similar manner to that of Example 161.
APCI-mass;m/z426(M+H⁺)
¹H-NMR(DMSO-d6):d;2.43(3H,s), 4.57(2H,d,J=6.0Hz), 6.61(1H,d,J=8.9Hz), 7.07 (1H,t,J=6.0Hz), 7.2-7.4(3H,m), 7.7-8.0(4H,m), 8.28(1H,d,J=2.4Hz), 8.51(1H,dt, J=4.9,0.8Hz), 10.18(1H,s) 11.33(1H,s

### Example 99

A suspension of N-(2-chloro-4-pyridyl)-9H-fluorene-1-carboxamide (160mg) in 1-benzylpiperazine (2ml) was stirred at 140°C for 4 hours and 160°C for 6 hours. After cooling, the mixture was poured into water and the mixture was stirred for 10 minutes. The precipitate was collected by filtration, washed with water and dried to give N-[2-(4-benzyl-1-piperazinyl)-4-pyridyl]-9H-fluorene-1-carboxamide.
APCI-mass;m/z461(M+H⁺)
¹H-NMR(DMSO-d6):d;3.5-3.7(10H,m), 4.19(2H,s), 7.09(1H,dd,J=1.7,5.7Hz), 7.2-7.8 (11H,m), 7.97(1H,d,J=6.4Hz), 8.04(1H,d,J=5.6Hz), 8.13(1H,d,J=6.7Hz), 10.43(1H,s)

### Example 100

To a solution of N-[2-(4-benzyl-1-piperazinyl)-4-pyridyl]-9H-fluorene-1-carboxamide (100mg) in methanol (2ml) and tetrahydrofuran was added hydrochloric acid (6N, 0.1ml) and the mixture was hydrogenated over 10 % palladium hydroxide on carbon (100mg, 50%wet) under hydrogen atmosphere for 8 hours. The catalyst was filtered off and the filtrate was evaporated. To the residue was added hydrochloric acid (1N, 0.5ml) and evaporated. The residue was triturated with ethanol, collected by filtration, washed with ethanol and dried to give N-[2-(1-piperazinyl)-4-pyridyl]-9H-fluorene-1-carboxamide dihydrochloride (64mg).
APCI-mass;m/z371(M+H⁺)
¹H-NMR(DMSO-d6): δ ;3.29(4H,s), 3.89(4H,s), 4.23(2H,s), 7.3-7.7(5H,m), 7.8-7.9 (2H,m), 8.00(1H,d,J=6.4Hz), 8.10(1H,d,J=6.7Hz), 8.19(1H,d,J=7.5Hz), 9.4-9.8(2H,m), 11.22(1H,s)

### Example 101

To a suspension of 2-(3-pyridylmethyl)-5-isoindolylamine (248mg) and pyridine (0.242ml) in dichloromethane (5ml) was added a solution of 9H-fluorene-1-carbonyl chloride (229mg) in dichloromethane (5ml) and stirred for 1 hour. The mixture was diluted with dichloromethane and washed with water and brine. The separated organic layer was dried over magnesium sulfate and evaporated. The residue was triturated with methanol and isopropyl ether, collected by filtration, washed with isopropyl ether and dried to give N-[2-(3-pyridylmethyl)-2,3-dihydro-1H-isoindol-5-yl]-9H-fluorene-1-carboxamide (151mg).
APCI-mass;m/z418(M+H⁺)
¹H-NMR(DMSO-d6): δ ;3.83(2H,s), 3.89(2H,s), 3.91(2H,s), 4.18(2H,s), 7.20(1H,d, J=8.0Hz), 7.3-7.5(3H,m), 7.5-7.7(5H,m), 7.80(1H,d,J=7.8Hz), 7.97(1H,d,J=6.7Hz), 8.10(1H,d,J=7.2Hz), 8.50(1H,d,J=3.6Hz), 8.59(1H,s), 10.30(1H,s)

### Example 102

N-benzyl-5-[(9H-fluorene-1-ylcarbonyl)amino]nicotinamide was obtained according to the similar manner to that of Example 161.
APCI-mass;m/z420(M+H⁺)
¹H-NMR(DMSO-d6): δ ;4.23(2H,s), 4.53(1H,d,J=5.9Hz), 7.2-7.5(7H,m), 7.5-7.7 (2H,m), 7.81(1H,d,J=7.6Hz), 7.99(1H,d,J=6.4Hz), 8.15(1H,d,J=7.3Hz), 8.71(1H,s), 8.82(1H,d,J=1.8Hz), 9.08(1H,d,J=2.4Hz), 9.30(1H,t,J=5.9Hz), 10.70(1H,s)

### Example 103

N-(4-fluorobenzyl)-5-[(9H-fluorene-1-ylcarbonyl)amino]nicotinamide was obtained according to the similar manner to that of Example 161.
APCI-mass;m/z438(M+H⁺)
¹H-NMR(DMSO-d6): δ ;4.23(2H,s), 4.53(1H,d,J=5.9Hz), 7.2-7.5(7H,m), 7.5-7.7 (2H,m), 7.81(1H,d,J=7.6Hz), 7.99(1H,d,J=6.4Hz), 8.15(1H,d,J=7.3Hz) 8.71(1H,s), 8.82(1H,d,J=1.8Hz), 9.08(1H,d,J=2.4Hz), 9.30(1H,t,J=5.9Hz), 10.70(1H,s)

### Example 104

N-[6-[(2-pyridylmethyl)amino]-3-pyridyl]-3-(2-thienyl)benzamide was obtained according to the similar manner to that of Example 161.
APCI-mass;m/z426(M+H⁺)
¹H-NMR(DMSO-d6):d;4.57(2H,d,J=6.0Hz), 6.61(1H,d,J=8.9Hz), 7.1-7.5(5H,m), 7.6-7.9(6H,m), 8.17(1H,s), 8.26(1H,d,J=2.5Hz), 8.51(1H,d,J=4.8Hz), 10.12(1H,s)

### Example 105

3-(2-thienyl)-N-[3-(1H-1,2,4-triazol-1-yl)phenyl]benzamide was obtained according to the similar manner to that of Example 161.
APCI-mass;m/z347(M+H⁺)
¹H-NMR(DMSO-d6):d;7.20(1H,dd,J=3.7,5.0Hz), 7.5-7.7(5H,m), 7.8-8.0(3H,m), 8.22 (1H,s), 8.26(1H,s), 8.39(1H,s), 9.29(1H,s), 10.62(1H,s)

### Example 106

N-[3-(5-pyrimidinyl)phenyl]-3-(2-thienyl)benzamide was obtained according to the similar manner to that of Example 161.
APCI-mass;m/z358(M+H⁺)
¹H-NMR(DMSO-d6):d;7.20(1H,dd,J=3.8,4.8Hz), 7.5-7.7(5H,m), 7.8-8.0(3H,m), 8.17 (1H,s), 8.22(1H,s), 9.12(2H,s), 9.23(1H,s), 10.53(1H,s)

### Example 107

N-[3-(1,2-dimethyl-1H-imidazol-5-yl)phenyl]-2-[4-(1-methyl-1-phenylethyl) phenyl]acetamide was obtained according to the similar manner to that of Example 161.
APCI-mass;m/z424(M+H⁺)
¹H-NMR(DMSO-d6):d;1.62(6H,s), 2.34(3H,s), 3.51(3H,s), 3.60(2H,s), 6.83(1H,s), 7.0-7.5(10H,m), 7.55(1H,d,J=8.7Hz), 7.68(1H,s), 10.25(1H,s)

### Example 108

A suspension of (2E)-N-[3-(1,2-dimethyl-lH-imidazol-5-yl)phenyl]-3-(1-naphthyl)-2-propenamide (81mg) in methanol (2ml) was hydrogenated over 10 % palladium on carbon (50%wet) under hydrogen atmosphere for 10 hours. The catalyst was filtered off. The filtrate was concentrated, purified with column chromatography (silica gel, 20g, 3% methanol in dichloromethane) to give N-[3-(1,2-dimethyl-1H-imidazol-5-yl)phenyl]-3-(1-naphthyl)propanamide (57mg).
APCI-mass;m/z370(M+H⁺)
¹H-NMR(DMSO-d6): δ ;2.34(3H,s), 2.75(2H,t,J=7.7Hz), 3.44(2H,t,J=7.7Hz), 3.52 (3H,s), 6.83(1H,s), 7.08(1H,d,J=7.7Hz), 7.34(1H,d,J=8.0Hz), 7.4-8.0(8H,m), 8.14(1H, d,J=8.0Hz), 10.05(1H,s)

### Example 109

N-[2-[(2-pyridylmethyl)amino]-5-pyrimidinyl]-9H-carbazol-1-carboxamide was obtained according to the similar manner to that of Example 161.
APCI-mass;m/z395(M+H⁺)
¹H-NMR(DMSO-d6): δ ;4.62(2H,d,J=6.2Hz), 7.1-7.4(4H,m), 7.41(1H,t,J=7.6Hz), 7.6-7.8(3H,m), 8.07(1H,d,J=7.6Hz), 8.17(1H,d,J=7.7Hz), 8.51(1H,d,J=4.8Hz), 8.67(2H,s), 10.29(1H,s), 11.47(1H,s)

### Example 110

N-[2-[(2-pyridylmethyl)amino]-5-pyrimidinyl]-9H-fluorene-1-carboxamide was obtained according to the similar manner to that of Example 161.
APCI-mass;m/z394(M+H⁺)
¹H-NMR(DMSO-d6): δ ;4.20(2H,s), 4.61(2H,d,J=6.3Hz), 7.2-7.5(4H,m), 7.5-7.9 (5H,m), 7.97(1H,d,J=6.7Hz), 8.11(1H,d,J=7.4Hz), 8.50(1H,d,J=4.8Hz), 8.61(1H,s), 10.18(1H,s)

### Example 111

To a suspension of 3-chloro-5-nitro-N-(2-pyridylmethyl)-2-pyridylamine (132mg) and iron powder (139mg) in ethanol (2m1) was added hydrochloric acid (1N, 1ml) and the mixture was refluxed for 3 hours. After cooling, the mixture was filtered and the filtrate was evaporated. To the residue was added 9H-fluorene-1-carboxylic acid (105mg) and dichloromethane (5ml). To a suspension was added 1-ethyl-3-(3-dimethylaminopropyl)carbodiimide hydrochloride (144mg) and N,N-dimethylaminopyridine (183mg) and the mixture was stirred for 16 hours. To the mixture was added aqueous sodium hydroxide (1N, 5ml) and dichloromethane and stirred for 15 minutes and filtered through celite. The organic layer was separated and washed with water and brine, dried over magnesium sulfate and evaporated. The residue was purified with column chromatography (silica gel, 25g, 2% methanol in dichloromethane) to give N-[5-chloro-6-[(2-pyridylmethyl)amino]-3-pyridyl]-9H-fluorene-1-carboxamide (55mg).
APCI-mass;m/z427(M+H⁺)
¹H-NMR(DMSO-d6): δ ;4.19(2H,s), 4.68(2H,d,J=5.7Hz), 7.08(1H,t,J=5.7Hz), 7.2-7.8 (8H,m), 7.97(1H,d,J=6.5Hz), 8.10(1H,d,J=6.9Hz), 8.16(1H,dd,J=2.2Hz), 8.26(1H,d, J=2.2Hz), 8.52(1H,d,J=4.8Hz), 10.24(1H,s)

### Example 112

N-[5-chloro-6-[(2-pyrazinylmethyl)amino]-3-pyridyl]-9H-fluorene-1-carboxamide was obtained according to the similar manner to that of Example 161.
APCI-mass;m/z428(M+H⁺)
¹H-NMR(DMSO-d6): δ ;4.18(2H,s), 4.73(2H,d,J=5.8Hz), 7.16(1H,t,J=5.8Hz) 7.3-7.7 (4H,m), 7.72(1H,d,J=6.9Hz), 7.97(1H,d,J=6.3Hz), 8.11(1H,d,J=6.8H 8.16(1H,d, J=2.2Hz), 8.26(1H,d,J=2.2Hz), 8.5-8.7(3H,m), 10.25(1H,s)

### Example 113

N-[3-(4-methoxy-3-pyridyl)phenyl]-9H-carbazol-1-carboxamide was obtained according to the similar manner to that of Example 161.
APCI-mass;m/z394(M+H⁺)
¹H-NMR(DMSO-d6): δ ;3.88(3H,s), 7.1-7.7(7H,m), 7.70(1H,d,J=8.0Hz), 7.80(1H,d, J=8.1Hz), 8.08(1H,s), 8.1-8.3(2H,m), 8.3-8.5(2H,m), 8.48(1H,d,J=5.7Hz), 10.45(1H,s), 11.51(1H,s)

### Example 114

N-[3-(1-ethyl-2-methyl-1H-imidazol-5-yl)phenyl]-9H-carbazol-1-carboxamide was obtained according to the similar manner to that of Example 161.
APCI-mass;m/z395(M+H⁺)
¹H-NMR(DMSO-d6): δ ;1.19(3H,t,J=7.1Hz), 2.39(3H,s), 4.01(2H,q,J=7.1Hz), 6.85 (1H,s), 7.1-7.5(5H,m), 7.71(1H,d,J=8.1Hz), 7.88(1H,d,J=8.1Hz), 7.96(1H,s), 8.11(1H, d,J=7.7Hz), 8.18(1H,J=7.7Hz), 8.38(1H,d,J=7.5Hz), 10.48(1H,s), 11.48(1H,s)

### Example 115

N-[3-(1-ethyl-2-methyl-1H-imidazol-5-yl)phenyl]-3-(2-thienyl)benzamide was obtained according to the similar manner to that of Example 161.
APCI-mass;m/z388(M+H⁺)
¹H-NMR(DMSO-d6): δ ;1.18(3H,t,J=7.2Hz), 3.99(2H,q,J=7.2Hz), 6.84(1H,s), 7.1-7.3 (2H,m), 7.45(1H,t,J=7.9Hz), 7.5-7.7(3H,m), 7.78(1H,d,J=8.1Hz), 7.8-8.0(3H,m), 8.19 (1H,s), 10.46(1H,s)

### Example 116

3-(5-chloro-2-thienyl)-N-[3-(1,2-dimethyl-lH-imidazol-5-yl)phenyl] benzamide was obtained according to the similar manner to that of Example 161.
APCI-mass;m/z408(M+H⁺)
¹H-NMR(DMSO-d6): δ ;2.36(3H,s), 3.55(3H,s), 6.88(1H,s), 7.1-7.3(2H,m), 7.45(1H,t, J=7.9Hz), 7.5-7.7(2H,m), 7.7-8.0(4H,m), 8.13(1H,s), 10.44(1H,s)

### Example 117

N-[3-[2-(difluoromethyl)-1-methyl-1H-imidazol-5-yl]phenyl]-3-(2-thienyl) benzamide was obtained according to the similar manner to that of Example 161.
APCI-mass;m/z410(M+H⁺)
¹H-NMR(DMSO-d6): δ ;3.77(3H,s), 7.0-7.3(3H,m), 7.28(1H,d,J=7.8Hz), 7.5-7.7 (4H,m), 7.9-8.1(4H,m), 8.19(1H,s), 10.51(1H,s)

### Example 118

N-[3-[2-(difluoromethyl)-1-methyl-1H-imidazol-5-yl]phenyl]-9H-carbazol-1-carboxamide was obtained according to the similar manner to that of Example 161.
APCI-mass;m/z417(M+H⁺)
¹H-NMR(DMSO-d6): δ ;3.79(3H,s), 7.2-7.5(6H,m), 7.56(1H,d,J=7.8Hz), 7.71(1H,d, J=8.0Hz), 7.96(1H,d,J=8.1Hz), 8.06(1H,s), 8.12(1H,d,J=7.5Hz), 8.18(1H,d,J=7.7Hz), 8.39(1H,d,J=7.7Hz), 10.53(1H,s), 11.50(1H,s)

### Example 119

N-[6-[(2-pyrazinylmethyl)amino]-3-pyridyl]-9H-fluorene-1-carboxamide was obtained according to the similar manner to that of Example 161.
APCI-mass;m/z394(M+H⁺)
¹H-NMR(DMSO-d6): δ ;4.18(2H,s), 4.63(2H,d,J=6.0Hz), 6.65(1H,d,J=8.9Hz), 7.17 (1H,t,J=6.0Hz), 7.3-7.6(4H,m), 7.63(1H,d,J=6.5Hz), 7.71(1H,d,J=6.9Hz), 7.82(1H,dd, J=8.9Hz,2.6Hz), 7.96(1H,dd,J=8.0Hz,1.5Hz), 8.09(1H,d,J=6.7Hz), 8.28(1H,d, J=2.4Hz), 8.51(1H,d,J=2.3Hz), 8.5-8.6(2H,m), 10.08(1H,s)

### Example 120

N-[3-(3-methyl-4H-1,2,4-triazol-4-yl)phenyl)-9H-carbazol-1-carboxamide was obtained according to the similar manner to that of Example 161.
APCI-mass;m/z368.47(M+H⁺)
¹H-NMR(DMSO-d6): δ ;2.40(3H,s), 7.2-7.5(4H,m), 7.61(1H,t,J=8.0Hz). 7.71(1H,d, J=8.0Hz), 7.98(1H,d,J=8.0Hz), 8.0-8,3(3H,m),8.40(1H,d,J=7.1Hz), 8.73(1H,s), 10.65 (1H,s), 11.51(1H,s)

### Example 121

N-[4-methoxy-3-(5-pyrimidinyl)phenyl]-9H-carbazol-1-carboxamide was obtained according to the similar manner to that of Example 161.
APCI-mass;m/z395(M+H⁺)
¹H-NMR(DMSO-d6): δ ;3.84(3H,s), 7.2-7.5(4H,m), 7.71(1H,d,J=8.0Hz), 7.93(1H,dd, J=8.9Hz,2.6Hz), 8.03(1H,d,J=2.4Hz), 8.13(1H,d,J=8.0Hz), 8.17(1H,d,J=8.0Hz), 8.37 (1H,d,J=7.5Hz), 8.98(2H,s), 9.18(1H,s), 10.41(1H,s), 11.51(1H,s)

### Example 122

To a mixture of N-(3-formylphenyl)-9H-fluorene-1-carboxamide (94mg), 4-aminopyrimidine (57mg), and N,N-dimethylformamide (1ml was added titanium (IV) isopropoxide (146mg) at ambient temperature. After stirring for 1 hour, to the reaction mixture was added sodium triacetoxyborohydride (254mg) and stirred for 14 hours. The reaction mixture was diluted with ethyl acetate (30ml) and washed with water (3 times, 30ml) and brine (10ml). The organic layer was dried over magnesium sulfate and filtered. Evaporation gave a residue which was purified by silica gel chromatography (ethyl acetate) to give N-[3-[(4-pyrimidinylamino)methyl]phenyl]-9H-fluorene-1-carboxamide (42mg) as white crystals.
MS:393(M+1)
NMR(DMSO, δ ):4.18(2H,br s), 4.54(2H,d,J=5.7Hz), 6.4-6.6(1H,m), 7.07(1H,d, J=7.5Hz), 7.2-8.2(12H,m), 8.41(1H,s), 10.35(1H,br s)

### Example 123

This was prepared in a manner similar to Example 122 to give N-[3-((4-pyridylamino)methyl]phenyl]-9H-fluorene-1-carboxamide.
MS:392(M+1)
NMR(DMSO, δ ):4.18(2H,br s), 4.49(2H,d,J=5.7Hz), 6.6-7.0(2H,m), 7.10(1H,d, J=8.1Hz), 7.3-8.2(12H,m), 8.61(1H, s), 10.39(1H,br s)

### Example 124

This was prepared in a manner similar to Example 122 to give N-[3-[[(5-bromo-1,3-thiazol-2-yl)amino]methyl]phenyl]-9H-fluorene-1-carboxamide.
MS:476,478(1:1,Br isotopes,M+1)
NMR(DMSO, δ ):4.18(2H,br s), 4.44(2H,d,J=5.8Hz), 7.0-7.2(2H,m), 7.2-7.9(8H,m), 7.97(1H,d,J=6.6Hz), 8.10(1H,d,J=7.2Hz), 8.37(1H,t,J=5.8Hz), 10.37(1H,br s)

### Example 125

This was prepared in a manner similar to Example 122 to give N-[3-[[(3-methyl-5-isoxazolyl)amino]methyl]phenyl]-9H-fluorene-1-carboxamide.
MS:396(M+1)
NMR(DMSO, δ ):2.00(3H,s), 4.18(2H,br s), 4.26(2H,d,J=6.2Hz), 7.08(1H,d,J=7.6Hz), 7.2-8.2(12H,m), 10.36(1H,br s)

### Example 126

This was prepared in a manner similar to Example 122 to give N-[3-[[(4,6-dimethyl-2-pyridyl)amino]methyl]phenyl]-9H-fluorene-1-carboxamide.
MS:420(M+1)
NMR(DMSO, δ ):2.14(3H,s), 2.28(3H,s), 4.20(2H,br s), 4.52(2H,d,J=5.9Hz), 6.2-6.4 (2H,m), 7.09(1H,d,J=7.6Hz), 7.2-7.8(8H,m), 7.84(1H,br s), 7.97(1H,d,J=6.5Hz), 8.10(1H,d,J=7.3Hz), 10.36(1H,br s)

### Example 127

This was prepared in a manner similar to Example 122 to give N-[3-[[(5,6-dimethyl-1,2,4-triazin-3-yl)amino]methyl]phenyl]-9H-fluorene-1-carboxamide.
MS:422(M+1)
NMR(DMSO, δ ):2.31(3H,s), 2.38(3H,s), 4.17(2H,br s), 4.55(2H,d,J=6.5Hz), 7.07(1H, d,J=7.3Hz), 7.2-7.9(9H,m), 7.97(1H,d,J=7.7Hz), 8.10(1H,d,J=7.3Hz), 10.32(1H,br s)

### Example 128

This was prepared in a manner similar to Example 122 to give N-[3-[[(6-chloro-3-pyridazinyl)amino]methyl]phenyl]-9H-fluorene-1-carboxamide.
MS:427(M+1)
NMR(DMSO, δ ):4.18(2H,br s), 4.56(2H,d,J=5.8Hz), 6.97(1H,d,J=9.3Hz), 7.10(1H,d, J=7.6Hz), 7.2-7.8(9H,m), 7.83(1H,br s), 7.97(1H,d,J=6.6Hz), 8.10(1H,d,J=7.2Hz), 10.35 (1H,br s)

### Example 129

This was prepared in a manner similar to Example 122 to give N-[3-[[(5-pyridine-3-yl-4H-1,2,4-triazol-3-yl)amino]methyl]phenyl]-9H-fluorene-1-carboxamide (11.2mg).
MS:459(M+1)
NMR(DMSO, δ ):4.18(2H,br s), 4.45(2H,d,J=6.6Hz), 7.0-8.4(15H,m), 8.54(1H,d, J=4.5Hz), 9.08(1H,br s), 10.37(1H,br s)

### Example 130

This was prepared in a manner similar to Example 122 to give N-[3-[(1,3,4-thiadiazol-2-ylamino)methyl)phenyl]-9H-fluorene-1-carboxamide.
MS:399(M+1)
NMR(DMSO, δ ):4.19(2H,br s), 4.51(2H,d,J=5.7Hz), 7.11(1H,d,J=7.8Hz), 7.2-7.8 (7H,m), 7.86(1H,br s), 7.97(1H,d,J=6.5Hz), 8.11(1H,d,J=7.0Hz), 8.33(1H,t,J=5.7Hz), 8.64(1H,s), 10.36(1H,br s)

### Example 131

This was prepared in a manner similar to Example 122 to give N-[3-[(1H-1,2,4-triazol-3-ylamino)methyl]phenyl]-9H-fluorene-1-carboxamide.
MS:382(M+1)
NMR(DMSO, δ ):4.18(2H,br s), 4.31(2H,d,J=5.8Hz), 6.77(1H,t,J=5.8Hz), 6.9-7.8 (10H,m), 7.85(1H,br s), 7.97(1H,d,J=6.6Hz), 8.10(1H,d,J=7.0Hz), 10.35(1H,br s)

### Example 132

This was prepared in a manner similar to Example 122 to give N-[3-[[(2-chloro-4-pyridyl)amino]methyl]phenyl]-9H-fluorene-1-carboxamide.
MS:426(M+1)
NMR(DMSO, δ ):4.18(2H,br s), 4.36(2H,d,J=5.8Hz), 6.4-6.6(2H,m), 7.08(1H,d, J=7.5Hz), 7.3-7.9(10H,m), 7.97(1H,d,J=6.6Hz), 8.10(1H,d,J=7.4Hz), 10.37(1H,br s)

### Example 133

This was prepared in a manner similar to Example 158 to give N-[3-[[(2-chloro-4-pyridyl)amino]methyl]phenyl]-9H-carbazol-1-carboxamide.
MS:427(M+1)
NMR(DMSO, δ ):4.37(2H,d,J=5.8Hz), 6.5-6.6(2H,m), 7.0-7.5(5H,m), 7.53(1H,t, J=5.8Hz), 7.6-8.0(4H,m), 8.0-8.3(2H,m), 8.37(1H,d,J=7.1Hz), 10.41(1H,br s), 11.49 (1H,br s)

### Example 134

This was prepared in a manner similar to Example 158 to give N-[3-[(4-pyridylamino)methyl]phenyl]-9H-carbazol-1-carboxamide.
MS:393(M+1)
NMR(DMSO, δ ):4.34(2H,d,J=5.8Hz), 6.4-6.6(2H,m), 7.0-7.5(6H,m), 7.6-8.3(7H,m), 8.36(1H,d,J=7.5Hz), 10.40(1H,br s), 11.50(1H,br s)

### Example 135

This was prepared in a manner similar to Example 156 to give N-[3-[[(5-fluoro-1H-benzimidazol-2-yl)amino]methyl]phenyl]-9H-fluorene-1-carboxamide.
MS:449(M+1)
NMR(DMSO, δ ):4.18(2H,br s), 4.54(2H,d,J=6.0Hz), 6.5-6.8(1H,m), 6.8-7.8(11H,m), 7.86(1H,br s), 7.96(1H,d,J=6.6Hz), 8.10(1H,d,3=7.2Hz), 10.36(1H,br s)

### Example 136

This was prepared in a manner similar to Example 158 to give N-[3-[[(5-methoxy-1H-benzimidazol-2-yl)amino]methyl]phenyl]-9H-carbazol-1-carboxamide.
MS:462(M+1)
NMR(DMSO, δ ):3.70(3H,s), 4.54(2H,d,J=5.8Hz), 6.48(1H,dd,J=2.3Hz,8.4Hz), 6.75 (1H,d,J=2.3Hz), 6.9-7.5(7H,m), 7.6-7.8(2H,m), 7.95(1H,br s), 8.0-8.3(2H,m), 8.36(1H, d,J=7.6Hz), 10.40(1H,br s), 10.7(1H,br s), 11.51(1H,br s)

### Example 137

This was prepared in a manner similar to Example 14 to give (2E)-N-[3-fluoro-5-(iminobenzylamino)phenyl]-3-(2-trifluoromethylphenyl)-2-propenamide.
mp:103-105°C
MS:428(M+1)
NMR(DMSO, δ ):6.37(1H,d,J=10.0Hz), 6.53(2H,s), 6.81-6.93(2H,m), 7.31-7.49 (4H,m), 7.64(1H,dd,J=7.5Hz,7.5Hz), 7.73-7.95(6H,m), 10.44(1H,s)

### Example 138

This was prepared in a manner similar to Example 14 to give (2E)-N-[3-chloro-5-(iminobenzylamino)phenyl]-3-(2-fluorophenyl)-2-propenamide.
mp:186-187°C
MS:394(M+1)
NMR(DMSO, δ ):6.57(3H,s), 6.92(1H,d,J=15.8Hz), 7.04(1H,s), 7.24-7.37(2H,m), 7.39-7.56(4H,m). 7.55-7.78(3H,m), 7.94(2H,s)10.37(1H,s)

### Example 139

This was prepared in a manner similar to Example 14 to give (2E)-N-[5-(iminobenzylamino)-2-methylphenyl]-3-(2-trifluoromethylphenyl)-2-propenamide.
mp:220-222°C
MS:424(M+1)
NMR(DMSO, δ ):2.24(3H,s), 6.54(2H,s), 6.67(1H,d,J=7.6Hz), 7.07-7.21(2H,m), 7.30 (1H,s), 7.39-7.56(3H,m), 7.63(1H,dd,J=7.5Hz,7.5Hz), 7.75-8.03(6H,m), 9.52 (1H,s)

### Example 140

This was prepared in a manner similar to Example 14 to give (2E)-N-[5-(iminobenzylamino)-2-methoxyphenyl]-3-(2-trifluoromethylphenyl)-2-propenamide.
mp:208-210°C
MS:440(M+1)
NMR(DMSO, δ ):3.87(3H,s), 6.33(2H,s), 6.62(1H,d,J=7.0Hz), 7.04(1H,d.J=8.7Hz), 7.34(1H,d,J=15.6Hz), 7.40-7.50(3H,m), 7.62(1H,dd,J=7.6Hz,7.6Hz), 7.71-8.00(7H,m), 9.48(1H,s)

### Example 141

This was prepared in a manner similar to Example 14 to give (2E)-N-[2-chloro-5-(iminobenzylamino)phenyl]-3-(2-trifluoromethylphenyl)-2-propenamide.
mp:198-199°C
MS:444(M+1)
NMR(DMSO, δ ):6.47(2H,s), 6.68(1H,d,J=8.3Hz), 7.26(1H,d,J=15.4Hz), 7.35-7.69 (6H,m), 7.73-8.03(6H,m), 9.68(1H,s)

### Example 142

This was prepared in a manner similar to Example 14 to give (2E)-N-[3-chloro-5-(iminobenzylamino)phenyl]-3-(2-bromophenyl)-2-propenamide.
mp:99-101°C
MS:454,456(M+1,Br isomer)
NMR(DMSO, δ ):6.58(3H,br s), 6.81(1H,d,J=15.5Hz), 7.02(1H,s), 7.31-7.62(6H,m), 7.70-7.80(2H,m), 7.85(1H,d,J=15.5Hz), 7.97(2H,m), 10.37(1H,s

### Example 143

This was prepared in a manner similar to Example 14 to give (2E)-N-[3-(iminobenzylamino)-4-methylphenyl)-3-(2-trifluoromethylphenyl)-2-propenamide.
mp:112-116°C
MS:424(M+1)
NMR(DMSO, δ ):2.04(3H,s), 6.16(2H,s), 6.90(1H,d,J=15.3Hz), 7.11-7.19(2H,m), 7.29(1H,d,J=8.3Hz), 7.40-7.51(3H,m), 7.62(1H,dd,J=7.4Hz,7.4Hz), 7.71-8.03(6H,m), 10.19(1H,s)

### Example 144

This was prepared in a manner similar to Example 14 to give (2E)-N-[3 -(iminobenzylamino)-4-methoxyphenyl]-3-(2-trifluoromethylphenyl)-2-propenamide.
mp:213-214°C
MS:440(M+1)
NMR(DMSO, δ ):3.71(3H,s), 6.28(2H,s), 6.88(1H,d,J=15.3Hz), 6.97(1H,d,J=8.9Hz), 7.17(1H,s), 7.33-7.50(4H,m), 7.62(1H,dd,J=7.5Hz,7.5Hz), 7.71-8.00(6H,m), 10.16 (1H,s)

### Example 145

This was prepared in a manner similar to Example 14 to give (2E)-N-[4-chloro-3-(iminobenzylamino)phenyl]-3-(2-trifluoromethylphenyl)-2-propenamide.
mp:188-189°C
MS:444(M+1)
NMR(DMSO, δ ):6.49(2H,s), 6.88(1H,d,J=15.4Hz), 7.27-7.50(6H,m), 7.63(1H,dd, J=7.5Hz,7.5Hz), 7.72-7.97(6H,m), 10.37(1H,s)

### Example 146

This was prepared in a manner similar to Example 14 to give (2E)-N-[3-(iminobenzylamino)-5-(methylsulfonylamino)phenyl]-3-(2-trifluoromethylphenyl)-2-propenamide.
mp:150-155°C
MS:503(M+1)
NMR(DMSO, δ ):3.02(3H,s), 6.47(3H,br s), 6.92(1H,d,J=15.4Hz), 7.10(1H,s), 7.28 (1H,s), 7.40-7.51(3H,m), 7.63(1H,dd,J=7.4Hz,7.4Hz), 7.70-7.99(6H,m), 9.68(1H,s), 10.34(1H,s)

### Example 147

This was prepared in a manner similar to Example 14 to give (2E)-N-[3-chloro-5-[imino(4-methylsulfonylaminobenzyl)amino]phenyl]-3-(2-chlorophenyl)-2-propenamide.
mp: 158-160°C
MS:503(M+1)
NMR(DMSO, δ ):6.50(2H,br s), 6.57(1H,s), 6.86(1H,d,J=15.6Hz), 7.01(1H,s), 7.23 (2H,d,J=8.7Hz), 7.41-7.50(2H,m), 7.50-7.59(2H,m), 7.73-7.95(4H,m), 10.01(1H,s) 10.37(1H,s)

### Example 148

This was prepared in a manner similar to Example 14 to give (2E)-N-[3-chloro-5-[imino(2-fluorobenzyl)amino]phenyl]-3-(2-trifluoromethylphenyl)-2-propenamide.
mp:209-210°C
MS:462(M+1)
NMR(CDCl₃, δ ):5.29(2H,br s), 6.49(1H,d,J=15.3Hz), 6.80(1H,s), 7.08-7.31(3H,m), 7.41-7.76(7H,m), 8.04-8.18(2H,m)

### Example 149

This was prepared in a manner similar to Example 14 to give (2E)-N-[3-[imino(2-fluorobenzyl)amino]phenyl]-3-(2-trifluoromethylphenyl)-2-propenamide hydrochloride.
MS:428(free+1)
NMR(DMSO, δ ):7.05(1H,d,J=15.4Hz), 7.37-7.74(7H,m), 7.77-8.03(5H,m), 8.48(1H,s), 9.23(1H,s), 10.07(1H,s). 11.05(1H,s), 11.61(1H,s)

### Example 150

This was prepared in a manner similar to Example 14 to give (2E)-N-[3-chloro-5-(iminobenzylamino)-2-methoxyphenyl]-3-(2-trifluoromethylphenyl)-2-propenamide.
MS:474(M+1)
NMR(DMSO, δ ):3.80(3H,s), 6.50(2H,s), 6.64(1H,s), 7.30-7.55(4H,m), 7.63(1H,t, J=7.4Hz), 7.75-8.05(7H,m), 9.71(1H,s)

### Example 151

This was prepared in a manner similar to Example 14 to give (2E)-N-[3-chloro-5-(iminobenzylamino)-4-methoxyphenyl]-3-(2-trifluoromethylphenyl)-2-propenamide.
MS:474(M+1)
NMR(DMSO, δ ):3.68(3H,s), 6.53(2H,br s), 6.84(1H,d,J=15.3Hz), 7.00(1H,s), 7.40-7.46(3H,m), 7.55-7.69(2H,m), 7.71-8.06(6H,m), 10.23(1H,s)

### Example 152

This was prepared in a manner similar to Example 14 to give (2E)-N-[2,4-difluoro-5-[imino(4-acetylaminobenzyl)amino]phenyl]-3-(2-trifluoromethylphenyl)-2-propenamide.
mp:180-185°C
MS:503(M+1)
NMR(DMSO, δ ):2.07(3H,s), 6.54(2H,s), 7.14(1H,d,J=15.3Hz), 7.29(1H,t,J=10.8Hz), 7.60-7.95(10H,m), 10.03(1H,s) 10.12(1H,s)

### Example 153

This was prepared in a manner similar to Example 14 to give 1:1 complex of phosphoric acid with (2E)-N-[3-chloro-5-[imino(4-methylsulfonylaminobenzyl)amino] phenyl]-(2-trifluoromethylphenyl)-2-propenamide.
mp:180-185°C
MS:537(M+1)
NMR(DMSO, δ ):3.17(3H,s), 6.89(1H,d,J=15.4Hz) 7.29(1H,s), 7.36-7.43(2H,m), 7.60-7.96(9H,m), 9.40(2H, br s), 10.51(1H,s), 10.76(1H,s), 11.36(1H,s)

### Example 154

This was prepared in a manner similar to Example 14 to give (2E)-N-[2,3-dichloro-5-(iminobenzylamino)phenyl]-3-(2-trifluoromethylphenyl)-2-propenamide.
mp:213-214°C
MS:478(M+1)
NMR(DMSO, δ ):6.67(2H,s), 6.89(1H,s), 7.25(1H,d,J=15.3Hz), 7.40-7.50(4H,m), 7.64(1H,t,J=7.5Hz), 7.75-7.98(6H,m), 9.82(1H,s)

### Example 155

To (2E)-N-[2,3-dichloro-5-(iminobenzylamino)phenyl]-3-(2-trifluoromethylphenyl)-2-propenamide (50mg) in ethyl acetate (1ml) was added 4N hydrochloric acid (1ml) in ethyl acetate. After one hour at room temperature, evaporated, slurried in hexane to give (2E)-N-[2,3-dichloro-5-(iminobenzylamino) phenyl]-3-(2-trifluoromethylphenyl)-2-propenamide hydrochloride (14mg) as a brown amorphous solid.
MS:478(free+1)
NMR(DMSO, δ ):7.12-8.22(13H,m), 9.24(1H,s), 9.98(1H,s), 10.21(1H,s), 11.68(1H,s)

### Example 156

To a mixture of 3-(1,2-dimethyl-1H-imidazol-5-yl)-5-fluoroaniline (62mg) and triethylamine (73mg) in dichloromethane (1ml) was added fluorenecarboxylic acid chloride (145mg) at ambient temperature. After stirring for 6 hours, the reaction mixture was diluted with ethyl acetate (5ml) and washed with water (3 times, 30ml). The organic layer was dried over magnesium sulfate and filtered. Evaporation gave a residue which was purified by silica gel chromatography (dichloromethane-methyl alcohol=10/1) and triturated with a mixture of ethyl acetate-isopropyl ether to give N-[3-(1,2-dimethyl-1H-imidazol-5-yl)-5-fluorophenyl]-9H-fluorene-1-carboxamide (55mg) as pale yellow crystals.
MS:398(M+1)
NMR(DMSO, δ ):2.37(3H,s), 3.60(3H,s), 4.21(2H, br s), 6.97(1H,s), 6.9-7.2(1H,m), 7.3-7.5(2H,m), 7.5-7.9(5H,m), 7.99(1H,d,J=6.5Hz), 8.14(1H,d,J=7.2Hz), 10.62(1H,s)

### Example 157

This was prepared in a manner similar to Example 156 to give N-[3-chloro-5-(1,2-dimethyl-1H-imidazol-5-yl)phenyl]-9H-fluorene-1-carboxamide.
MS:414(M+1)
NMR(DMSO, δ ):2.37(3H,s), 3.59(3H,s), 4.21(2H,br s), 6.98(1H,s), 7.2-7.9(7H,m), 7.9-8.1(2H,m), 8.14(1H,d,J=7.3Hz), 10.60(1H,s)

### Example 158

To a mixture of 3-(1,2-dimethyl-1H-imidazol-5-yl)-5-methoxyaniline (1.07g), 1-fluorenecarboxylic acid (1.24g), 1-hydroxybenzotriazole (799mg), 4-dimethylaminopyridine (722mg), and dichloromethane (50ml) was added 1-ethyl-3-(3-dimethylaminopropyl)carbodiimide hydrochloride (1.13g) at ambient temperature under stirring. After stirring for 2 days, the reaction mixture was poured into 1N-hydrochloric acid (200ml). The separated aqueous layer was washed with dichloromethane (200ml), neutralized with 1N-sodium hydroxide, and extracted with dichloromethane (3 times, 200ml). The combined organic extracts were dried over magnesium sulfate and filtered. Evaporation gave a residue which was purified by silica gel chromatography (dichloromethane-methyl alcohol;1→2→3%) and triturated with ethyl acetate-n-hexane (1:2) to give N-[3-(1,2-dimethyl-1H-imidazol-5-yl)-5-methoxyphenyl]-9H-fluorene-1-carboxamide (1.16g) as pale yellow crystals.
MS:410(M+1)
NMR(DMSO, δ ):2.36(3H,s), 3.57(3H,s), 3.81(3H,s), 4.20(2H,br s), 6.7-6.8(1H,m), 6.89(1H,s), 7.3-7.5(3H,m), 7.5-7.7(3H,m), 7.74(1H,d,J=7.7Hz), 7.9-8.1(1H,m), 8.13 (1H,d,J=7.5Hz), 10.40(1H,br s)

### Example 159

This was prepared in a manner similar to Example 156 to give N-[3-(1,2-dimethyl-1H-imidazol-5-yl)-5-(4-morpholinyl)phenyl]-9H-fluorene-1-carboxamide.
MS:465(M+1)
NMR(DMSO, δ ):2.35(3H,s), 3.0-3.3(4H,m), 3.56(3H,s), 3.6-3.9(4H,m), 4.20(2H,br s), 6.72(1H,s), 6.86(1H,s), 7.2-7.8(7H,m), 7.9-8.1(1H,m) 8.11(1H,d,J=7.2Hz), 10.29 (1H,s)

### Example 160

This was prepared in a manner similar to Example 158 to give (2E)-N-[3-chloro-5-(1,2-dimethyl-1H-imidazol-5-yl)phenyl]-3-(2-(trifluoromethyl)phenyl]-2-propenamide.
MS:420(M+1)
NMR(DMSO, δ ):2.36(3H,s), 3.57(3H,s), 6.87(1H,d,J=15.3Hz), 6.97(1H,s), 7.2-7.3(1H,m), 7.5-8.0(7H,m), 10.65(1H,br s)

### Example 161

To a suspension of 3-(1,2-dimethyl-1H-imidazol-5-yl)-5-methoxyaniline (52mg) and 6-fluoro-9H-carbazol-1-carboxylic acid (55mg) in dichloromethane (2ml) was added 1-ethyl-3-(3-dimethylaminopropyl)carbodiimide hydrochloride (69mg) and N,N-dimethylaminopyridine (15mg) and the mixture was stirred for 18 hours. The mixture was diluted with dichloromethane and washed with water, aqueous sodium hydroxide (1N) and brine. The separated organic layer was dried over magnesium sulfate and evaporated. The residue was triturated with methanol, collected by filtration and dried to give N-[3-(1,2-dimethyl-1H-imidazol-5-yl)-5-methoxyphenyl]-6-fluoro-9H-carbazol-1-carboxamide (40mg).
APCI-mass;m/z429.40(M+H⁺)
¹H-NMR(DMSO-d6): δ ;2.37(3H,s), 3,59(3H,s), 3.83(3H,s), 6.74(1H,s), 6.91(1H,s), 7.2-7.4(2H,m), 7.53(1H,t,J=1.4Hz), 7.62(1H,t,J=2.0Hz), 7.71(1H,dd,J=4.6Hz,8.9Hz), 8.03(1H,dd,J=2.5Hz,9.3Hz), 8.13(1H,d,J=7.2Hz), 8.40(1H,d,J=7.6Hz), 10.44(1H,s), 11.56(1H,s)

### Example 162

N-[3-(1,2-dimethyl-1H-imidazol-5-yl)-5-methoxyphenyl]-9H-carbazol-1-carboxamide was obtained according to the similar manner to that of Example 161.
APCI-mass;m/z411.40(M+H⁺)
¹H-NMR(DMSO-d6): δ ;2.37(3H,s), 3.59(3H,s), 3.83(3H,s), 6.74(1H,t,J=1.8Hz), 6.91 (1H,s), 7.21(1H,t,J=7.5Hz), 7.31(1H,t,J=7.7Hz), 7.42(1H,t,J=7.6Hz), 7.54(1H,s), 7.63 (1H,t,J=2.0Hz), 8.10(1H,d,J=7.7Hz), 8.18(1H,d,J=7.7Hz) 8.38(1H,d,J=7.6Hz), 10.43 (1H,s), 11.50(1H,s)

### Example 163

N-[3-chloro-5-(1,2-dimethyl-1H-imidazol-5-yl)phenyl]-9H-carbazol-1-carboxamide was obtained according to the similar manner to that of Example 161.
APCI-mass;m/z415.27(M+H⁺)
¹H-NMR(DMSO-d6): δ ;2.38(3H,s), 3.61(3H,s), 6.99(1H,s), 7.2-7.5(4H,m), 7.72(1H,d, J=8.0Hz), 7.84(1H,t,J=1.6Hz), 8.1-8.3(3H,m), 8.40(1H,d,J=7.5Hz), 10.61(1H,s), 11.53 (1H,s)

### Example 164

N-[3-chloro-5-(1,2-dimethyl-1H-imidazol-5-yl)phenyl]-6-fluoro-9H-carbazol-1-carboxamide was obtained according to the similar manner to that of Example 161.
APCI-mass;m/z433.20(M+H⁺)
¹H-NMR(DMSO-d6): δ ;2.37(3H,s), 3.61(3H,s), 6.98(1H,s), 7.2-7.4(3H,m), 7.70(1H,dd,J=9.3Hz,4.6Hz), 7.83(1H,s), 8.04(1H,dd,J=9.3Hz,2.5Hz), 8.1-8.3(2H,m), 8.42(1H,d,J=7.6Hz), 10.61(1H,s), 11.50(1H,s)

### Example 165

N-[3-chloro-5-(1,2-dimethyl-1H-imidazol-5-yl)phenyl]-2,3-dimethyl-1H-indole-7-carboxamide was obtained according to the similar manner to that of Example 161.
APCI-mass;m/z393.33(M+H⁺)
¹H-NMR(DMSO-d6): δ ;2.18(3H,s), 2.37(6H,s), 3.59(3H,s), 6.96(1H,s), 7.09(1H,t, J=7.7Hz), 7.23(1H,t,J=1.7Hz), 7.63(1H,d,J=7.7Hz), 7.71(1H,d,J=7.7Hz), 7.81(1H,s), 8.11(1H,t,J=1.9Hz), 10.44(1H,s) 10.80(1H,s)

### Example 166

N-[3-chloro-5-(4,5-dimethyl-1H-imidazol-1-yl)phenyl]-9H-fluorene-1-carboxamide was obtained according to the similar manner to that of Example 161.
APCI-mass;m/z414.33(M+H⁺)
¹H-NMR(DMSO-d6): δ ;2.12(3H,s), 2.15(3H,s), 4.21(2H,s), 7.3-7.5(3H,m), 7.5-7.9 (5H,m), 7.98(1H,d,J=6.4Hz), 8.05(1H,t,J=1.8Hz), 8.15(1H,d,J=6.8Hz), 10.70(1H,s)

### Example 167

N-[3-chloro-5-(4,5-dimethyl-1H-imidazol-1-yl)phenyl]-9H-carbazol-1-carboxamide was obtained according to the similar manner to that of Example 161.
APCI-mass;m/z415.20(M+H⁺)
¹H-NMR(DMSO-d6): δ ;2.14(3H,s), 2.17(3H,s), 7.21(1H,t,J=7.5Hz), 7.3-7.5(3H,m), 7.6-7.8(2H,m), 7.87(1H,t,J=1.9Hz), 8.1-8.3(3H,m), 8.41(1H,d,J=7.5Hz), 10.71(1H,s), 11.54 z(1H,s)

### Example 168

N-[3-chloro-5-(4,5-dimethyl-1H-imidazol-1-yl)phenyl]-2,3-dimethyl-1H-indole-7-carboxamide was obtained according to the similar manner to that of Example 161.
APCI-mass;m/z393.33(M+H⁺)
¹H-NMR(DMSO-d6): δ ;2.12(3H,s), 2.15(3H,s), 2.19(3H,s), 2.36(3H,s), 7.10(1H,t, J=7.7Hz), 7.30(1H,t,J=1.9Hz), 7.64(1H,d,J=7.7Hz), 7.7-7.9(2H,m), 7.84(1H,t, J=1.7Hz), 8.15(1H,t,J=1.9Hz) 10.55(1H,s), 10.82(1H,s)

### Example 169

N-[3-(4,5-dimethyl-1H-imidazol-1-yl)-5-methoxyphenyl]-9H-fluorene-1-carboxamide was obtained according to the similar manner to that of Example 161.
APCI-mass;m/z410.47(M+H⁺)
¹H-NMR(DMSO-d6): δ ;2.12(3H,s), 2.14(3H,s), 3.82(3H,s), 4.20(2H,s), 6.75(1H,t, J=2.0Hz), 7.3-7.5(3H,m), 7.5-7.7(4H,m), 7.74(1H,d,J=7.7Hz), 7.98(1H,d,J=6.2Hz), 8.13(1H,d,J=6.6Hz) 10.52(1H,s)

### Example 170

N-[3-(4,5-dimethyl-1H-imidazol-1-yl)-5-methoxyphenyl]-9H-carbazol-1-carboxamide was obtained according to the similar manner to that of Example 161.
APCI-mass;m/z411.33(M+H⁺)
¹H-NMR(DMSO-d6): δ ;2.13(3H,s), 2.16(3H,s), 3.85(3H,s), 6.76(1H,t,J=2.0Hz), 7.20 (1H,t,J=7.1Hz), 7.31(1H,t,J=7.7Hz 7.42(1H,t,J=7.1Hz), 7.56(1H,t,J=1.7Hz), 7.6-7.8 (3H,m), 8.10(1H,d,J=7.7Hz), 8.17(1H,d,J=7.6Hz), 8.39(1H,d,J=7.7Hz), 10.53(1H,s), 11.50(1H,s)

### Example 171

N-[3-(4,5-dimethyl-1H-imidazol-1-yl)-5-methoxyphenyl]-2,3-dimethyl-1H-indole-7-carboxamide was obtained according to the similar manner to that of Example 161.
APCI-mass;m/z389.27(M+H⁺)
¹H-NMR(DMSO-d6): δ ;2.12(3H,s), 2.13(3H,s), 2.18(3H,s), 2.36(3H,s), 3.83(3H,s), 6.73(1H,t,J=2.0Hz), 7.09(1H,t,J=7.6Hz), 7.53(1H,s), 7.6-7.7(2H,s), 7.71(1H,d, J=7.1Hz), 10.36(1H,s), 10.77(1H,s)

### Example 172

N-[3-cyano-5-(4,5-dimethyl-1H-imidazol-1-yl)phenyl]-9H-fluorene-1-carboxamide was obtained according to the similar manner to that of Example 161.
APCI-mass;m/z405.40(M+H⁺)
¹H-NMR(DMSO-d6): δ ;2.13(3H,s), 2.16(3H,s), 4.22(2H,s), 7.3-7.9(7H,m), 7.99(1H,d, J=6.7Hz), 8.11(1H,t,J=2.0Hz), 8.16(1H,d,J=6.8Hz), 8.31(1H,t,J=1.6Hz), 10.85(1H,s)

### Example 173

N-[3-(5-chloro-4-methyl-1H-imidazol-1-yl)phenyl]-9H-carbazol-1-carboxamide was obtained according to the similar manner to that of Example 161.
APCI-mass;m/z401(M+H⁺)
¹H-NMR(DMSO-d6): δ ;2.19(3H,s), 7.22(1H,d,J=8.0Hz), 7.32(1H,t,J=7.7Hz), 7.42(1H,t,J=7.6Hz), 7.59(1H,t,J=8.0Hz), 7.70(1H,d,J=8.0Hz), 7.9-8.3(5H,m), 8.39(1H, d,J=7.8Hz), 10.65(1H,s), 11.51(1H,s)

### Example 174

N-[3-(5-chloro-4-methyl-1H-imidazol-1-yl)phenyl]-9H-fluorene-1-carboxamide was obtained according to the similar manner to that of Example 161.
APCI-mass;m/z400.27(M+H⁺)
¹H-NMR(DMSO-d6): δ ;2.17(3H,s), 4.20(2H,s), 7.21(1H,d,J=7.7Hz), 7.3-7.7(5H,m), 7.75(1H,d,J=6.7Hz), 7.9-8.1(4H,m), 8.14(1H,d,J=6.7Hz) 10.63(1H,s)

### Example 175

To a suspension of 9H-fluorene-1-carboxylic acid (835mg) and 3-methyl-N²-(2-pyridylmethyl)-2,5-pyridyldiamine (845mg) in dichloromethane (30ml) was added 1-ethyl-3-(3-dimethylaminopropyl)carbodiimide hydrochloride (1.14g) and 4-dimethylaminopyridine (242mg) and stirred for 18 hours. The mixture was diluted with dichloromethane, washed with water and brine, dried over magnesium sulfate and evaporated under reduced pressure. The residue was purified by silica gel column chromatography (silica gel 150g, eluting with 1-4% methanol/dichloromethane) to give N-[5-methyl-6-[methyl(2-pyridylmethyl)amino]-3-pyridyl]-9H-fluorene-1-carboxamide (1.14g).
APCI-mass;m/z407(M+1)
NMR(200MHz,DMSO-d6): δ ;2.20(3H,s), 4.17(2H,s), 4.66(2H,d,J=5.8Hz), 6.55(1H,t, J=5.8Hz), 7.19-7.74(9H,m), 7.96(1H,d,J=7.1Hz), 8.08(1H,d,J=7.1Hz), 8.12(1H,d, J=2.3Hz), 8.50(1H,d,J=4.5Hz), 10.02(1H,s)

### Example 176

To a suspension of 9H-fluorene-1-carboxylic acid (178mg) and N²-(2-pyridylmethyl)-3-(trifluoromethyl)-2,5-pyridyldiamine (230mg) in dichloromethane (5.0ml) was added 1-ethyl-3-(3-dimethylaminopropyl)carbodiimide hydrochloride (244mg) and 4-dimethylaminopyridine (52mg) and stirred for 18 hours. The mixture was diluted with dichloromethane, washed with water and brine, dried over magnesium sulfate and evaporated under reduced pressure. The residue was triturated with methanol, collected by filtration and dried to give N-[6-[(2-pyridylmethyl)amino]-5-(trifluoromethyl)-3-pyridyl]-9H-fluorene-1-carboxamide (143mg).
APCI-mass;m/z461(M+1)
NMR(200MHz,DMSO-d6): δ ;4.20(2H,s), 4.73(2H,d,J=5.4Hz), 7.11(1H,t,J=5.4Hz), 7.22-7.78(8H,m), 7.97(1H,d,J=6.5Hz), 8.12(1H,d,J=7.0Hz), 8.36(1H,d,J=2.3Hz), 8.52(2H,d,J=4.2Hz), 10.33(1H,s)

### Example 177

To a suspension of 9H-fluorene-1-carboxylic acid (167mg) and N²-methyl-N²-(2-pyridylmethyl)-2,5-pyridyldiamine (170mg) in dichloromethane (6.0ml) was added 1-ethyl-3-(3-dimethylaminopropyl)carbodiimide hydrochloride (228mg) and 4-dimethylaminopyridine (48mg) and stirred for 18 hours. The mixture was diluted with dichloromethane, washed with water and brine, dried over magnesium sulfate and evaporated under reduced pressure. The residue was triturated with methanol, collected by filtration and dried to give N-[6-[methyl(2-pyridylmethyl)amino]-3-pyridyl]-9H-fluorene-1-carboxamide (121mg).
APCI-mass;m/z407(M+1)
NMR(200MHz,DMSO-d6): δ ;3.13(3H,s), 4.19(2H,s), 4.86(2H,s), 6.71(1H,d,J=9.1Hz), 7.12(1H,d,J=7.8Hz), 7.25-7.76(7H,m), 7.91(1H,dd,J=9.1Hz,2.6Hz), 7.96(1H,d, J=7.5Hz), 8.09(1H,d,J=6.7Hz), 8.41(1H,d,J=2.4Hz), 8.52(1H,d,J=4.8Hz), 10.13(1H,s)

### Example 178

To a suspension of 9H-fluorene-1-carboxylic acid (151mg) and 3-chloro-N²-methyl-N²-(2-pyridylmethyl)-2,5-pyridyldiamine (185mg) in dichloromethane (6.0ml) was added 1-ethyl-3-(3-dimethylaminopropyl)carbodiimide hydrochloride (207mg) and 4-dimethylaminopyridine (44mg) and stirred for 18 hours. The mixture was diluted with dichloromethane, washed with water and brine, dried over magnesium sulfate and evaporated under reduced pressure. The residue was purified by silica gel column chromatography (silica gel 20g, eluting with 3-5% methanol/dichloromethane) to give N-[5-chloro-6-[methyl(2-pyridylmethyl)amino]-3-pyridyl]-9H-fluorene-1-carboxamide (129mg).
APCI-mass;m/z441(M+1)
NMR(200MHz,DMSO-d6): δ ;2.91(3H,s), 4.21(2H,s), 4.60(2H,s), 7.24-7.45(4H,m), 7.59(1H,d,J=7.6Hz), 7.63(1H,d,J=7.6Hz), 7.74-7.79(2H,m), 7.98(1H,d,J=6.9Hz), 8.13 (1H,d,J=6.9Hz), 8.31(1H,d,J=2.3Hz), 8.50(1H,s), 8.52(1H,d,J=2.3Hz), 10.47(1H,s)

### Example 179

To a suspension of 9H-fluorene-1-carboxylic acid (128mg) and N²-[(4-methyl-2-pyridyl)methyl]-2,5-pyridyldiamine (133mg) in dichloromethane (4.0ml) was added 1-ethyl-3-(3-dimethylaminopropyl)carbodiimide hydrochloride (174mg) and 4-dimethylaminopyridine (37mg) and stirred for 18 hours. The mixture was diluted with dichloromethane, washed with water and brine, dried over magnesium sulfate and evaporated under reduced pressure. The residue was triturated with methanol, collected by filtration and dried to give N-[6-[[(4-methyl-2-pyridyl)methyl] amino]-3-pyridyl]-9H-fluorene-1-carboxamide (95mg).
APCI-mass;m/z407(M+1)
NMR(200MHz,DMSO-d6): δ ;2.28(3H,s), 4.18(2H,s), 4.52(2H,d,J=6.0Hz), 6.60(1H,d, J=8.9Hz), 7.00-7.73(7H,m), 7.79(1H,dd,J=8.9Hz,2.5Hz), 7.92(1H,d,J=6.8Hz), 8.09(1H, d,J=6.8Hz), 8.29(1H,d,J=2.5Hz), 8.36(1H,d,J=5.0Hz), 10.06(1H,s)

### Example 180

To a suspension of 9H-fluorene-1-carboxylic acid (160mg) and N²-[(3-methyl-2-pyridyl)methyl]-2,5-pyridyldiamine (164mg) in dichloromethane (6.0ml) was added 1-ethyl-3-(3-dimethylaminopropyl)carbodiimide hydrochloride (220mg) and 4-dimethylaminopyridine (47mg) and stirred for 18 hours. The mixture was diluted with dichloromethane, washed with water and brine, dried over magnesium sulfate and evaporated under reduced pressure. The residue was triturated with methanol, collected by filtration and dried to give N-[6-[[(3-methyl-2-pyridyl)methyl) amino]-3-pyridyl]-9H-fluorene-1-carboxamide (161mg).
APCI-mass;m/z407(M+1)
NMR(200MHz,DMSO-d6): δ ;2.35(3H,s), 4.19(2H,s), 4.54(2H,d,J=5.0Hz), 6.72(1H,d, J=8.9Hz), 6.80(1H,t,J=5.0H 7.23(1H,dd,J=7.5Hz,4.8Hz), 7.32-7.82(7H,m), 7.97(1H, d,J=6.9Hz), 8.09(1H,d,J=6.9Hz), 8.35(1H,s), 8.38(1H,d,J=8.9Hz), 10.06(1H,s)

### Example 181

To a suspension of 9H-fluorene-1-carboxylic acid (190mg) and N2-[[3-chloro-(5-trifluoromethyl)-2-pyridyl]methyl]-2,5-pyridyldiamine (320mg) in dichloromethane (5.5ml) was added 1-ethyl-3-(3-dimethylaminopropyl)carbodiimide hydrochloride (259mg) and 4-dimethylaminopyridine (55mg) and stirred for 18 hours. The mixture was diluted with dichloromethane, washed with water and brine, dried over magnesium sulfate and evaporated under reduced pressure. The residue was purified by silica gel column chromatography (silica gel 35g, eluting with 3-5% methanol/dichloromethane) to give N-[6-[[[3-chloro-5-(trifluoromethyl)-2-pyridyl] methyl]amino]-3-pyridyl]-9H-fluorene-1-carboxamide (98mg).
APCI-mass;m/z495(M+1)
NMR(200MHz,DMSO-d6): δ ;4.17(2H,s), 4.66(2H,d,J=5.8Hz), 6.64(1H,d,J=8.9Hz), 7.24(1H,t,J=6.1Hz), 7.31-7.72(6H,m), 7.82(1H,dd,J=8.9Hz,2.5Hz), 7.96(1H,d, J=6.5Hz), 8.07-8.16(2H,m), 8.26(1H,d,J=2.4Hz), 8.90(1H,s), 10.06(1H,s)

### Example 182

To a suspension of 9H-fluorene-1-carboxylic acid (365mg) and N²-(3-pyridylmethyl)-2,5-pyridyldiamine (388mg) in dichloromethane (8.0ml) was added 1-ethyl-3-(3-dimethylaminopropyl)carbodiimide hydrochloride (500mg) and 4-dimethylaminopyridine (105mg) and stirred for 18 hours. The mixture was diluted with dichloromethane, washed with water and brine, dried over magnesium sulfate and evaporated under reduced pressure. The residue was triturated with diisopropyl ether and methanol, collected by filtration and dried to give N-[6-[(3-pyridylmethyl)amino]-3-pyridyl]-9H-fluorene-1-carboxamide (215mg).
APCI-mass;m/z393(M+1)
NMR(200MHz,DMSO-d6): δ ;4.18(2H,s), 4.50(2H,d,J=6.0Hz), 6.58(1H,d,J=8.9Hz), 7.06(1H,t,J=6.0Hz), 7.30-7.82(8H,m), 7.96(1H,d,J=6.5Hz), 8.09(1H,d,J=7.1Hz), 8.32 (1H,d,J=2.4Hz), 8.43(1H,dd,J=1.5Hz,4.7Hz), 8.56(1H,d,J=1.7Hz), 10.06(1H,s)

### Example 183

To a suspension of 9H-fluorene-1-carboxylic acid (172mg) and N²-[2-(2-pyridyl)ethyl]-2,5-pyridyldiamine (180mg) in dichloromethane (4.0ml) was added 1-ethyl-3-(3-dimethylaminopropyl)carbodiimide hydrochloride (235mg) and 4-dimethylaminopyridine (50mg) and stirred for 18 hours. The mixture was diluted with dichloromethane, washed with water and brine, dried over magnesium sulfate and evaporated under reduced pressure. The residue was triturated with methanol, collected by filtration and dried to give N-[6-[[2-(2-pyridyl)ethyl]amino]-3-pyridyl]-9H-fluorene-1-carboxamide (45mg).
APCI-mass;m/z407(M+1)
NMR(200MHz,DMSO-d6): δ ;3.00(1H,t,J=6.7Hz), 3.59(2H,td,J=6.7Hz,6.2Hz), 4.19 (2H,s), 6.48-6.52(2H,m), 7.19-7.75(9H,m), 7.97(1H,d,J=6.5Hz), 8.09(1H,d,J=7.3Hz), 8.33(1H,d,J=2.3Hz), 8.51(1H,d,J=4.0Hz), 10.05(1H,s)

### Example 184

To a suspension of 9H-fluorene-1-carboxylic acid (366mg) and N²-benzyl-2,5-pyridyldiamine (440mg) in dichloromethane (8.0ml) was added 1-ethyl-3-(3-dimethylaminopropyl)carbodiimide hydrochloride (500mg) and 4-dimethylaminopyridine (106mg) and stirred for 18 hours. The mixture was diluted with dichloromethane, washed with water and brine, dried over magnesium sulfate and evaporated under reduced pressure. The residue was triturated with diisopropyl ether and methanol, collected by filtration and dried to give N-[6-(benzylamino)-2-pyridyl]-9H-fluorene-1-carboxamide (205mg).
APCI-mass;m/z392(M+1)
NMR(200MHz,DMSO-d6): δ ;4.18(2H,s), 4.48(2H,d,J=6.1Hz), 6.55(1H,d,J=8.9Hz), 6.98(1H,t,J=6.1Hz), 7.20-7.80(11H,m), 7.96(1H,d,J=6.6Hz), 8.08(1H,d,J=7.1Hz), 8.30 (1H,d,J=2.4Hz), 10.05(1H,s)

### Example 185

To a suspension of 9H-fluorene-1-carboxylic acid (73mg) and 3-(4-pyridylmethyl)aniline (64mg) in dichloromethane (3.5ml) was added 1-ethyl-3-(3-dimethylaminopropyl)carbodiimide hydrochloride (100mg) and 4-dimethylaminopyridine (21mg) and stirred for 18 hours. The mixture was diluted with dichloromethane, washed with water and brine, dried over magnesium sulfate and evaporated under reduced pressure. The residue was purified by silica gel column chromatography (silica gel 15g, eluting with 1-4% methanol/dichloromethane) to give N-[3-(4-pyridylmethyl)phenyl]-9H-fluorene-1-carboxamide (96mg).
APCI-mass;m/z377(M+1)
NMR(200MHz,DMSO-d6): δ ;3.99(2H,s), 4.17(2H,s), 7.02(1H,d,J=7.5Hz), 7.25-7.72 (10H,m), 7.97(1H,d,J=7.9Hz), 8.10(1H,d,J=7.2Hz), 8.48(2H,dd,J=1.4Hz,4.5Hz), 10.30 (1H,s)

### Example 186

This was prepared in a manner similar to Example 14 to give N-[3-(1,2-benzisoxazol-3-ylamino)-5-chlorophenyl]-2-chlorocinnamamide.
mp:133-135°C
MS:424(M+1)
NMR(DMSO, δ ):6.93(1H,d,J=15.6Hz), 7.35-7.67(8H,m), 7.76-7.85(1H,m), 7.92(1H,d, J=15.6Hz), 8.05(1H,s), 8.17(1H,d,J=7.9Hz), 9.88(1H,s), 10.59(1H,s)
Elemental analysis:
Calculated+(H₂O)_{1.0}:C;59.74,H;3.87,N;9.50
Found:C;59.69,H;3.89,N;9.51

### Example 187

To a suspension of 7-fluoro-2,3-dihydro-benzo[b]oxepin-4-carboxylic acid (146mg) in dichloromethane (3ml) was added oxalyl chloride (0.15ml) and N,N-dimethylformamide (1 drop) and the mixture was stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was triturated with methanol to give 7-fluoro-2,3-dihydro-benzo[b]oxepin-4-carboxylic acid(3-imidazol-1-yl-phenyl)-amide (105mg).
APCI-mass;m/z350(M+H⁺)
¹H-NMR(DMSO-d6): δ ;2.98(2H,t,J=4.5Hz), 4.25(2H,t,J=4.7Hz), 7.07(1H,dd, J=15.4Hz,5.1Hz), 7.1-7.3(3H,m), 7.3-7.4(2H,m), 7.49(1H,t,J=8.0Hz), 7.6-7.7(2H,m), 7.97(1H,t,J=1.9Hz), 8.16(1H,s), 10.19(1H,s)

### Example 188

To a suspension of 9-methanesulfonyl-2,3-dihydro-benzo[b]oxepin-4-carboxylic acid (188mg) in dichloromethane (2ml) was added oxalyl chloride (0.2ml) and N,N-dimethylformamide (1 drop) and the mixture was stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 3 hours. The mixture was diluted with dichloromethane, washed with water and brine. The separated organic layer was dried over magnesium sulfate and evaporated under reduced pressure. The residue was triturated with methanol to give 9-methanesulfonyl-2,3-dihydro-benzo[b]oxepin-4-carboxylic acid(3-imidazol-1-yl-phenyl)-amide (248mg).
APCI-mass;m/z410(M+H⁺)
¹H-NMR(DMSO-d6): δ ;3.01(2H,t,J=4.4Hz), 3.32(3H,s), 4.45(2H,t,J=4.6Hz), 7.14 (1H,s), 7.2-7.4(3H,m), 7.50(1H,t,J=8.0H 7.6-8.0(4H,m), 7.98(1H,s), 8.17(1H,s) 10.26(1H,s)

### Example 189

To a suspension of 9-chloro-2,3-dihydro-benzo[b]oxepin-4-carboxylic acid (157mg) in dichloromethane (3ml) was added oxalyl chloride (0.16ml) and N,N-dimethylformamide (1 drop) and the mixture was stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was triturated with methanol to give 9-chloro-2,3-dihydro-benzo[b]oxepin-4-carboxylic acid(3-imidazol-1-yl-phenyl)-amide (195mg).
APCI-mass;m/z366(M+H⁺)
¹H-NMR(DMSO-d6): δ ;3.02(2H,t,J=4.3Hz), 4.37(2H,t,J=4.7Hz), 7.07(1H,d,J=7.8Hz), 7.12(1H,s), 7.27(1H,s), 7.3-7.6(4H,m), 7.6-7.8(2H,m), 7.97(1H,t,J=2.0Hz), 8.17(1H,d, J=1.0Hz), 10.22(1H,s)

### Example 190

To a suspension of 8-methoxy-2,3-dihydro-benzo[b]oxepin-4-carboxylic acid (154mg) in dichloromethane (3ml) was added oxalyl chloride (0.15ml) and N,N-dimethylformamide (1 drop) and the mixture was stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was triturated with methanol to give 8-methoxy-2,3-dihydro-benzo[b]oxepin-4-carboxylic acid(3-imidazol-1-yl-phenyl)-amide (222mg).
APCI-mass;m/z362(M+H⁺)
¹H-NMR(DMSO-d6): δ ;2.94(2H,t,J=4.7Hz), 3.77(3H,s), 4.26(2H,t,J=4.7Hz), 6.56(1H, d,J=2.4Hz), 6.68(1H,d,J=8.6Hz), 7.12(1H,s), 7.25(1H,s), 7.3-7.4(2H,m), 7.46(1H,d, J=8.0Hz), 7.6-7.7(2H,m), 7.96(1H,s), 8.16(1H,s), 10.07(1H,s)

### Example 191

To a suspension of 7-methoxy-2,3-dihydro-benzo[b]oxepin-4-carboxylic acid (154mg) in dichloromethane (3ml) was added oxalyl chloride (0.15ml) and N,N-dimethylformamide (1 drop) and the mixture was stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was purified with column chromatography (silica gel 25g, 2% methanol in dichloromethane) to give 7-methoxy-2,3-dihydro-benzo[b]oxepin-4-carboxylic acid(3-imidazol-1-yl-phenyl)-amide (189mg).
APCI-mass;m/z362(M+H⁺)
¹H-NMR(DMSO-d6): δ ;2.96(2H,t,J=4.7Hz), 3.74(3H,s), 4.20(2H,t,J=4.7Hz), 6.8-7.0 (2H,m), 7.02(1H,d,J=2.7Hz), 7.13(1H,s), 7.24(1H,s), 7.34(1H,d,J=8.4Hz), 7.48(1H,d, J=8.0Hz), 7.6-7.8(2H,m), 7.97(1H,s), 8.17(1H,s), 10.17(1H,s)

### Example 192

To a suspension of 7-chloro-2,3-dihydro-benzo[b]oxepin-4-carboxylic acid (157mg) in dichloromethane (3ml) was added oxalyl chloride (0.16ml) and N,N-dimethylformamide (1 drop) and the mixture stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was crystalized from methanol and collected by filtration to give 7-chloro-2,3-dihydro-benzo[b]oxepin-4-carboxylic acid(3-imidazol-1-yl-phenyl)-amide (197mg).
APCI-mass;m/z366(M+H⁺)
¹H-NMR(DMSO-d6): δ ;2.98(2H,t,J=4.6Hz), 4.27(2H,t,J=4.6Hz), 7.01(1H,d,J=8.7Hz), 7.13(1H,s), 7.23(1H,s), 7.3-7.7(6H,m), 7.96(1H,t,J=1.9Hz), 8.16(1H,s), 10.17(1H,s)

### Example 193

To a suspension of 2,3-dihydro-benzo[b]oxepin-4-carboxylic acid (154mg) and 1-hydroxybenzotriazole (131mg) in dichloromethane (10ml) was added 1-ethyl-3-(3-dimethylaminopropyl)carbodiimide hydrochloride (233mg) and the mixture was stirred for 1 hour. To the mixture 3-(imidazol-1-yl)aniline (129mg) and N,N-dimethylaminopyridine (109mg) was added and the mixture was stirred for 24 hours. The mixture was diluted with dichloromethane, washed with water and brine, dried over potassium carbonate and evaporated. The residue was purified with column chromatography (silica gel, 25g, 3% methanol in dichloromethane) to give 2,3-dihydro-benzo[b]oxepin-4-carboxylic acid(3-imidazol-1-yl-phenyl)-amide (196mg).
ESI-mass;m/z332(M+H⁺)
¹H-NMR(DMSO-d6): δ ;2.98(2H,t,J=4.3Hz), 4.27(2H,t,J=4.3Hz), 7.0-7.6(8H,m), 7.6-7.8(2H,m), 7.98(1H,t,J=2.0Hz), 8.16(1H,s), 10.17(1H,s)

### Example 194

A suspension of 2,3-dihydro-benzo[b]oxepin-4-carboxylic acid(3-imidazol-1-yl-phenyl)-amide (110mg) in ethanol (5ml) was hydrogenated over palladium on carbon (10% w/w, 50% wet, 50mg) under hydrogen atmosphere for 10 hours. The catalyst was filtered off, the filtrate was evaporated under reduced pressure. The residue was triturate with dichloromethane and isopropyl ether to give 2,3,4,5-tetrahydro-benzo[b]oxepin-4-carboxylic acid(3-imidazol-1-yl-phenyl)-amide (59mg).
APCI-mass;m/z334(M+H⁺)
NMR(DMSO-d6): δ ;2.0-2.3(2H,m), 2.6-3.2(3H,m), 3.6-3.8(1H,m), 4.3-4.5(1H,m), 6.9-7.6(8H,m), 7.63(1H,s), 7.92(1H,s), 8.14(1H,s), 10.23(1H,s)

### Example 195

To a suspension of 8-chloro-2,3-dihydro-benzo[b]oxepin-4-carboxylic acid (106mg) and 1-hydroxybenzotriazole (77mg) in dichloromethane (5ml) was added 1-ethyl-3-(3-dimethylaminopropyl)carbodiimide hydrochloride (136mg) and the mixture was stirred for 30 minutes. To the mixture 3-(imidazol-1-yl)aniline (75mg) and N,N-dimethylaminopyridine (63mg) was added and the mixture was stirred for 24 hours. The mixture was diluted with dichloromethane, washed with water and brine, dried over potassium carbonate and evaporated. The residue was triturated with methanol, collected by filtration and dried to give 8-chloro-2,3-dihydro-benzo[b]oxepin-4-carboxylic acid(3-imidazol-1-yl-phenyl)-amide (102mg).
ESI-mass;m/z366(M+H⁺)
¹H-NMR(DMSO-d6): δ ;2.98(2H,t,J=4.6Hz), 4.29(2H,t,J=4.6Hz), 7.09(1H,d,J=2.1Hz), 7.13(1H,d,J=2.1Hz), 7.16(1H,d,J=2.2Hz), 7.26(1H,s), 7.34(1H,d,J=9.0Hz), 7.46(1H,d, J=6.3Hz), 7.50(1H,d,J=6.8Hz), 7.6-7.8(2H,m), 7.96(1H,t,J=2.0Hz), 8.16(1H,s), 10.19 (1H,s)

### Example 196

To a suspension of 1,1-dioxo-2,3-dihydro-1H-benzo[b]thiepin-4-carboxylic acid (135mg) and 1-hydroxybenzotriazole (92mg) in dichloromethane (3ml) was added 1-ethyl-3-(3-dimethylaminopropyl)carbodiimide hydrochloride (163mg) and the mixture was stirred for 30 minutes. To the mixture 3-(imidazol-1-yl)aniline (90mg) and N,N-dimethylaminopyridine (83mg) was added and the mixture was stirred for 24 hours. The mixture was diluted with dichloromethane, washed with water and brine, dried over potassium carbonate and evaporated. The residue was triturated with methanol, collected by filtration and dried to give 1,1-dioxo-2,3-dihydro-benzo[b]thiepin-4-carboxylic acid(3-imidazol-1-yl-phenyl)-amide (50mg).
ESI-mass;m/z380(M+H⁺)
¹H-NMR(DMSO-d6): δ ;3.05(2H,t,J=6.6Hz), 3.80(2H,t,J=6.6Hz), 7.13(1H,s), 7.37(1H, d,J=9.0Hz), 7.4-7.8(7H,m), 7.99(1H,s), 8.06(1H,d,J=7.4Hz), 8.18(1H,s), 10.42(1H,s)

### Example 197

To a suspension of 7-methyl-2,3-dihydro-benzo[b]oxepin-4-carboxylic acid (143mg) in dichloromethane (3ml) was added oxalyl chloride (0.15ml) and N,N-dimethylformamide (1drop) and the mixture was stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was crystalized from 2-propanol and collected by filtration to give 7-methyl-2,3-dihydro-benzo[b]oxepin-4-carboxylic acid (3-imidazol-1-yl-phenyl)-amide (178mg).
APCI-mass;m/z346(M+H⁺)
¹H-NMR(DMSO-d6): δ ;2.27(3H,s), 2.96(2H,t,J=4.4Hz), 4.23(2H,t,J=4.7Hz), 6.88(1H, d,J=8.2Hz), 7.0-7.2(2H,m), 7.2-7.3(2H,m), 7.34(1H,d,J=9.0Hz), 7.48(1H,d,J=8.0Hz), 7.6-7.8(2H,m), 7.98(1H,t,J=1.9Hz), 8.17(1H,s), 10.14(1H,s)

### Example 198

To a suspension of 8,9-dihydro-7H-benzocycloheptene-6-carboxylic acid (132mg) in dichloromethane (3ml) was added oxalyl chloride (0.15ml) and N,N-dimethylformamide (1 drop) and the mixture was stirred at ambient temperature for 2 hours. The solvents were evaporated under reduced pressure to give crude acid chloride. To a suspension of 3-(imidazol-1-yl)aniline (111mg) in dichloromethane (3ml) and pyridine (0.17ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was crystalized from methanol and collected by filtration to give 8,9-dihydro-7H-benzocycloheptene-6-carboxylic acid(3-imidazol-1-yl-phenyl)-amide (195mg).
APCI-mass;m/z330(M+H⁺)
¹H-NMR(DMSO-d6): δ ;2.0-2.2(2H,m), 2.60(2H,t,J=6.3Hz), 2.7-2.9(2H,m), 7.13 (1H,s), 7.2-7.5(6H,m), 7.47(1H,t,J=8.0Hz), 7.6-7.8(2H,m), 7.99(1H,t,J=2.0Hz), 8.17 (1H,s), 10.19(1H,s)

### Example 199

To a suspension of 7-iodo-2,3-dihydro-benzo[b]oxepin-4-carboxylic acid (142mg) in dichloromethane (3ml) was added oxalyl chloride (0.15ml) and N,N-dimethylformamide (1drop) and the mixture was stirred at ambient temperature for 2 hours. The solvents were evaporated under reduced pressure to give crude acid chloride. To a suspension of 3-(imidazol-1-yl)aniline (72mg) in dichloromethane (3ml) and pyridine (0.11ml) was added a solution of acid chloride (obtained above) in dichloromethane (3ml) dropwise and the mixture was stirred at ambient temperature for 2 hours. The mixture was diluted with dichloromethane, washed with water and brine. The separated organic layer was dried over magnesium sulfate and evaporated under reduced pressure. The residue was crystalized from methanol and collected by filtration to give 7-iodo-2,3-dihydro-benzo[b]oxepin-4-carboxylic acid(3-imidazol-1-yl-phenyl)-amide (162mg).
APCI-mass;m/z458(M+H⁺)
¹H-NMR(DMSO-d6): δ ;2.97(2H,t,J=4.2Hz), 4.26(2H,t,J=4.5Hz), 6.81(1H,d,J-8.5Hz), 7.13(1H,s), 7.22(1H,s), 7.34(1H,d,J=8.0Hz), 7.46(1H,d,J=8.0Hz), 7.56(1H,dd,J=2.2Hz, 8.5=Hz), 7.7-7.8(2H,m), 7.82(1H,d,J=2.0Hz), 7.96(1H,s), 8.16(1H,s), 10.15(1H,s)

### Example 200

To a suspension of 7-fluoro-2,3-dihydro-benzo[b]oxepin-4-carboxylic acid (83mg) and 5-amino-3-chloro-2-(pyridine-2-ylmethyl)aminopyridine (97mg) in dichloromethane (5ml) was added 1-ethyl-3-(3-dimethylaminopropyl)carbodiimide hydrochloride (115mg) and N,N-dimethylaminopyridine (24mg) and the mixture was stirred for 24 hours and diluted with dichloromethane-methanol (10:1). The solution was washed with water, aqueous sodium hydroxide (1N) and brine. The separated organic layer was dried over magnesium sulfate and evaporated. The residue was triturated with methanol, collected by filtration and dried to give 7-fluoro-2,3-dihydro-benzo[b]oxepin-4-carboxylic acid[5-chloro-6-[(pyridine-2-ylmethyl)amino]pyridine-3-yl]amide (139mg).
APCI-mass;m/z425(M+H⁺)
¹H-NMR(DMSO-d6): δ ;2.94(2H,t,J=4.5Hz), 4.22(2H,t,J=4.7Hz), 4.66(2H,d,J=5.7Hz), 6.9-7.4(7H,m), 7.71(1H,dt,J=1.8Hz,7.7Hz), 8.01(1H,d,J=2.2Hz), 8.18(1H,d,J=2.2Hz), 8.50(1H,d,J=3.6Hz), 9.88(1H,s)

### Example 201

To a suspension of 7-fluoro-2,3-dihydro-benzo[b]oxepin-4-carboxylic acid (104mg) and 3-[1,2,4]triazol-1-yl-phenylamine (80mg) in dichloromethane (5ml) was added 1-ethyl-3-(3-dimethylaminopropyl)carbodiimide hydrochloride (134mg) and N,N-dimethylaminopyridine (31mg) and the mixture was stirred for 24 hours and diluted with dichloromethane. The solution was washed with water, aqueous sodium hydroxide (1N) and brine. The separated organic layer was dried over magnesium sulfate and evaporated. The residue was triturated with methanol, collected by filtration and dried to give 7-fluoro-2,3-dihydro-benzo[b]oxepin-4-carboxylic acid (3-[1,2,4]triazol-1-yl-phenyl)-amide (74mg).
APCI-mass;m/z351 (M+H⁺)
¹H-NMR(DMSO-d6): δ ;2.99(2H,t,J=4.4Hz), 4.25(2H,t,J=4.7Hz), 7.00(1H,dd,J=5.2Hz, 8.9Hz), 7.12(1H,dt,J=3.0Hz,8.3Hz), 7.22(1H,s), 7.33(1H,dd,J=3.0Hz,10.0Hz), 7.5-7.9 (3H,m), 8.2-8.3(2H,m), 9.27(1H,s), 10.25(1H,s)

### Example 202

To a suspension of 7-fluoro-2,3-dihydro-benzo[b]oxepin-4-carboxylic acid (104mg) and 3-pyrimidin-5-yl-phenylamine (86mg) in dichloromethane (5ml) was added 1-ethyl-3-(3-dimethylaminopropyl)carbodiimide hydrochloride (134mg) and N,N-dimethylaminopyridine (31mg) and the mixture was stirred for 24 hours and diluted with dichloromethane. The solution was washed with water, aqueous sodium hydroxide (1N) and brine. The separated organic layer was dried over magnesium sulfate and evaporated. The residue was triturated with methanol, collected by filtration and dried to give 7-fluoro-2,3-dihydro-benzo[b]oxepin-4-carboxylic acid (3-pyrimidin-5-yl-phenyl)-amide (133mg)
APCI-mass;m/z362(M+H⁺)
¹H-NMR(DMSO-d6): δ ;2.99(2H,t,J=4.4Hz), 4.25(2H,t,J=4.7Hz), 7.01(1H,dd,J=5.2Hz, 9.0Hz), 7.12(1H,dt,J=3.0Hz,8.3Hz), 7.22(1H,s), 7.33(1H,dd,J=3.0Hz,9.8Hz), 7.5-7.6 (2H,m), 7.8-7.9(1H,m), 8.08(1H,s), 9.09(2H,s), 9.21(1H,s), 10.16(1H,s)

### Example 203

To a suspension of 7-fluoro-2,3-dihydro-benzo[b]oxepin-4-carboxylic acid (104mg) and 3-(2,3-dimethyl-3H-imidazol-4-yl)-phenylamine (99mg) in dichloromethane (5ml) was added 1-ethyl-3-(3-dimethylaminopropyl)carbodiimide hydrochloride (134mg) and N,N-dimethylaminopyridine (31mg) and the mixture was stirred for 24 hours and diluted with dichloromethane. The solution was washed with water, aqueous sodium hydroxide (1N) and brine. The separated organic layer was dried over magnesium sulfate and evaporated. The residue was triturated with methanol, collected by filtration and dried to give 7-fluoro-2,3-dihydro-benzo[b]oxepin-4-carboxylic acid [3-(2,3-dimethyl-3H-imidazol-4-yl)-phenyl]-amide (108mg).
APCI-mass;m/z378(M+H⁺)
¹H-NMR(DMSO-d6): δ ;2.36(3H,s), 2.98(2H,t,J=5.0Hz), 3.55(3H,s), 4.25(2H,t, J=4.7Hz), 6.86(1H,s), 7.00(1H,dd,J=5.2Hz,8.9Hz), 7.0-7.2(4H,m), 7.3-7.5(2H,m), 7.69(1H,d,J=8.2Hz), 7.77(1H,s), 10.08(1H,s)

### Example 204

To a suspension of 3-(2,3-dimethyl-3H-imidazol-4-yl)-aniline (94mg) and 3-naphtalen-1-yl-acrylic acid (99mg) in dichloromethane (5ml) was added 1-ethyl-3-(3-dimethylaminopropyl)carbodiimide hydrochloride (144mg) and N,N-dimethylaminopyridine (31mg) and the mixture was stirred for 24 hours and evaporated. The residue was purified with column chromatography (silica gel 20g, 3% methanol in dichloromethane) to give N-[3-(2,3-dimethyl-3H-imidazol-4-yl)-phenyl]-3-naphthalen-1-yl-acrylamide (64mg).
APCI-mass;m/z368(M+H⁺)
¹H-NMR(DMSO-d6): δ ;2.37(3H,s), 3.57(3H,s), 6.9-7.0(2H,m), 7.15(1H,d,J=7.7Hz), 7.44(1H,t,J=7.9Hz), 7.6-7.8(4H,m), 7.9-8.1(4H,m), 8.26(1H,d,J=7.6Hz), 8.39(1H.d, J=15.5Hz), 10.42(1H,s)

### Example 205

This was prepared in a manner similar to Example 14 to give (2E)-N-[3-chloro-5-[[imino(phenyl)methyl]amino]phenyl]-3-(1H-indole-3-yl)-2-propenamide.
mp:184-187°C
MS:415(M+1)
NMR(DMSO, δ ):6.60(3H,br s), 6.78(1H,d,J=15.8Hz), 6.97-7.28(2H,m), 7.30-7.69 (5H,m), 7,78(1H,d,J=15.8Hz), 7.83-8.05(5H,m), 10.09(1H,s), 11.68(1H,s)

### Example 206

To a suspension of 3-(4,5-dimethylimidazol-1-yl)aniline (94mg) and 6-chloro-2H-chromenyl-3-carboxylic acid (106mg) in dichloromethane (5ml) was added 1-ethyl-3-(3-dimethylaminopropyl)carbodiimide hydrochloride (144mg) and N,N-dimethylaminopyridine (31mg) and the mixture was stirred for 24 hours. The mixture was diluted with dichloromethane and washed with water, aqueous sodium hydroxide (1N) and brine. The separated organic layer was dried over magnesium sulfate and evaporated. The residue was triturated with methanol, collected by filtration and dried to give 6-chloro-2H-chromenyl-3-carboxylic acid[3-(4,5-dimethyl-imidazol-1-yl)-phenyl]-amide (73mg).
APCI-mass;m/z380,382(M+H⁺)
¹H-NMR(DMSO-d6): δ ;2.10(3H,s), 2.11(3H,s), 5.02(2H,s), 6.92(1H,d,J=8.6Hz), 7.12(1H,d,J=8.3Hz), 7.31(1H,dd,J=8.3Hz,2.6Hz), 7.4-7.5(2H,m), 7.50(1H,t,J=8.0Hz), 7.63(1H,s), 7.7-7.9(2H,m), 10.33(1H,s)

### Example 207

To a suspension of 2-(pyridine-2-ylmethylamino)-5-aminopyridine (100mg) and 7-fluoro-2,3-dihydro-benzo[b]oxepin-4-carboxylic acid (104mg) in dichloromethane (5ml) was added 1-ethyl-3-(3-dimethylaminopropyl)carbodiimide hydrochloride (144mg) and N,N-dimethylaminopyridine (31mg) and the mixture was stirred for 24 hours and evaporated. The residue was purified with column chromatography (silica gel 20g, 2-3% methanol in dichloromethane) to give 7-fluoro-2,3-dihydro-benzo[b]oxepin-4-carboxylic acid[6-[(pyridine-2-ylmethyl)amino]-pyridine-3-yl]-amide (109mg).
APCI-mass;m/z391(M+H⁺)
¹H-NMR(DMSO-d6): δ ;2.94(2H,t,J=4.7Hz), 4.22(2H,t,J=4.7Hz), 4.54(2H,d,J=6.0Hz), 6.9-7.4(7H,m), 7.6-7.8(2H,m), 8.18(1H,d,J=2.4Hz), 8.50(1H,d,J=4.1Hz) 9.71(1H,s)

### Example 208

To a suspension of 3-amino-5-(pyridine-3-yl)pyridine dihydrochloride (80mg) and pyridine (0.27ml) in dichloromethane (5ml) was added a solution of the fluorene-1-carbonyl chloride (75mg) in dichloromethane (2ml) dropwise and stirred for 6 hours. The mixture was diluted with dichloromethane and washed with a aqueous solution of sodium hydrogen carbonate and brine. The separated organic layer was dried over sodium sulfate and evaporated. The residue was purified with column chromatography (silica gel 25g, 3% methanol in dichloromethane) to give 9H-fluorene-1-carboxylic acid [3,3']bipyridyl-5-ylamide (103mg).
ESI-mass;m/z386(M+Na⁺)
¹H-NMR(DMSO-d6): δ ;4.24(2H,s), 7.3-7.5(2H,m), 7.5-7.7(2H,m), 7.83(1H,d, J=6.8Hz), 7.99(1H,d,J=6.5Hz), 8.1-8.2(2H,m), 8.57(1H,t,J=3.8Hz), 8.66(1H,dd, J=4.8Hz,1.5Hz), 8.72(1H,d,J=2.0Hz), 8.95(1H,d,J=2.0Hz), 9.03(1H,d,J=2.2Hz), 10.69 (1H,s)

### Example 209

To a solution of 9H-fluorene-1-carboxylic acid(5-hydrazinocarbonyl-pyridine-3-yl)-amide (0.37g) in n-butanol (15ml) were added methyl isocyanate (79mg) and 1,8-diazabicyclo[5.4.0]undec-7-ene (80µl), and the mixture was heated to 120°C for 5 hours. Evaporation gave a residue which was taken up into water. The resultant precipitate was collected by filteration, washed with water, and dried in vacuo to give 9H-fluorene-1-carboxylic acid[5-(4-methyl-5-thioxo-4,5-dihydro-1H-[1,2,4]triazol-3-yl)-pyridine-3-yl]-amide (203mg).
APCI-mass;400(m/z,[M+H]⁺)
NMR(DMSO-d₆,d);3.62(3H,s), 4.23(2H,s), 7.36-7.44(2H,m), 7.55-7.66(2H,m), 7.82 (1H,d,J=7.0Hz), 8.00(1H,d,J=6.3Hz), 8.17(1H,d,J=7.0Hz), 8.61(1H,d,J=2.1Hz), 8.70 (1H,d,J=1.9Hz), 9.13(1H,d,J=2.4Hz) 10.79(1H,s), 14.03(1H,brs)

### Example 210

To a solution of 9H-fluorene-1-carboxylic acid[5-(4-methyl-5-thioxo-4,5-dihydro-1H-[1,2,4]triazol-3-yl)-pyridine-3-yl]-amide (120mg) in acetic acid (5ml) was added a solution of sodium nitrite (83mg) in water(0.4ml), and the mixture was heated to 100°C for 1 hour. After cooling to ambient temperature the resultant precipitate was collected by filteration, washed with water, and dried in vacuo to give 9H-fluorene-1-carboxylic acid[5-(4-methyl-4H-[1,2,4]triazol-3-yl)-pyridine-3-yl]-amide (112mg).
APCI-mass;368(m/z,[M+H]⁺)
NMR(DMSO-d₆,d);3.88(3H,s), 4.24(2H,s), 7.36-7.44(2H,m), 7.57-7.66(2H,m), 7.82(1H,d,J=7.1Hz) 8.00(1H,d,J=6.3Hz), 8.17(1H,d,J=7.1Hz), 8.71(1H,d,J=2.2Hz), 8.76(1H,d,J=1.9Hz 8.95(1H,s), 9.16(1H,d,J=2.3Hz) 10.83(1H,s)

### Example 211

To a solution of 3-methyl-2-trifluoromethyl-1H-indole-7-carboxylic acid (60mg), 6-(2-methyl-pyridine-3-yloxy)pyridine-3-ylamine (50mg), and 1-[3-(dimethylamino)propyl]-3-ethylcarbodiimide hydrochloride (66mg) in dichloromethane (3ml) was added 4-dimethylaminopyridine (15mg) at ambient temperature. After stirring at ambient temperature for 48 hours, the reaction mixture was diluted with ethyl acetate, washed in turn with water, aqueous potassium carbonate and brine, and dried over potassium carbonate. Evaporation under reduced pressure gave a residue which was triturated with methanol to give 3-methyl-2-trifluoromethyl-1H-indole-7-carboxylic acid[6-(2-methyl-pyridine-3-yloxy)-pyridine-3-yl]amide (43mg).
APCI-mass;427(m/z,[M+H]⁺)
NMR(DMSO-d₆,d);2.32(3H,s), 2.43(3H,d,J=2.0Hz), 7.17(1H,d,J=8.8Hz), 7.23-7.40 (2H,m), 7.51(1H,d,J=8.1Hz), 7.85-8.05(2H,m), 8.23-8.40(2H,m), 8.48(1H,d,J=2.5Hz), 10.58 (1H,s), 11.46(1H,s)

## Claims

1. A compound of the formula (I):
R¹-A-X-NHCO-Y-R²
wherein
R¹ is heterocyclic group which may have suitable substituents, or phenyl which may have suitable substituents,
R² is condensed phenyl which may have suitable substituents, phenyl which may have suitable substituents, or thienyl which may have suitable substituents,
A is a group of the formula:
-(CH₂)ₜ-(O)ₘ-
or 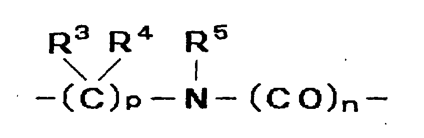 in which R³ and R⁴ are each hydrogen or linked together to form imino,
R⁵ is hydrogen or lower alkyl,
t is 0, 1 or 2,
p, m and n are each 0 or 1,
X is phenylene which may have suitable substituents, or bivalent heterocyclic group containing nitrogen which may have suitable substituents,
Y is bond, lower alkylene, or lower alkenylene,
and a salt thereof.

2. The compound of claim 1, wherein
R¹ is imidazolyl;
imidazolyl which have 1 to 3 lower alkyl;
imidazolyl which have 1 to 3 lower alkanoyl;
imidazolyl which have 1 to 3 halogen and/or lower alkyl;
pyridyl;
pyridyl which have 1 to 3 lower alkyl;
pyridyl which have 1 to 3 lower alkoxy;
pyridyl which have 1 to 3 halogen;
pyridyl which have 1 to 3 halogen and/or trihalo(lower)alkyl;
phenyl;
phenyl which have 1 to 3 lower alkyl;
phenyl which have 1 to 3 lower alkylamino;
phenyl which have 1 to 3 lower alkanoylamino;
phenyl which have 1 to 3 lower alkylsulfonylamino;
phenyl which have 1 to 3 halogen;
phenyl which have 1 to 3 lower alkoxy;
phenyl which have 1 to 3 halogen and/or lower alkoxy;
phenyl which have unsaturated heteromonocyclic group containing 1 to 4 nitrogen atom(s);
phenyl which have unsaturated heteromonocyclic group containing 1 or 2
salfur atom(s) and 1 to 3 nitrogen atom(s);
phenyl which have unsaturated heteromonocyclic group containing 1 or 2
oxygen atom(s) and 1 to 3 nitrogen atom(s);
phenyl which have oxidopyridyloxy;
oxochromenyl;
piperazinyl;
triazolyl ;
triazolyl which have 1 to 3 lower alkyl;
triazolyl which have 1 to 3 thioxo and/or lower alkyl;
triazolyl which have unsaturated heteromonocyclic group containing 1 to 4 nitrogen atom(s);
pyrimidinyl;
pyrazinyl;
quinolinyl;
isoquinolinyl;
thienyl;
thiazolyl which have 1 to 3 halogen;
benzothiazolyl;
isoxazolyl which have 1 to 3 lower alkyl;
triazinyl which have 1 to 3 lower alkyl;
pyridazinyl which have 1 to 3 halogen;
thiadiazolyl;
benzimidazolyl which have 1 to 3 lower alkyl;
benzimidazolyl which have 1 to 3 halogen;
benzimidazolyl which have 1 to 3 lower alkoxy;
benzisoxazolyl;
indolyl which have 1 to 3 lower alkyl,
R2 is fluorenyl;
indolyl which have 1 to 3 lower alkyl;
phenyl;
phenyl which have 1 to 3 halogen;
phenyl which have 1 to 3 lower alkyl;
phenyl which have 1 to 3 lower alkoxy;
phenyl which have 1 to 3 trihalo(lower)alkyl;
phenyl which have 1 to 3 cyano;
phenyl which have halophenyl;
phenyl which have phenyl;
phenylsulfanyl;
phenyl which have lower alkylphenyl;
phenyl which have thienyl;
phenyl which have halothienyl;
pyridyl;
thienyl which have phenyl;
carbazolyl;
carbazolyl which have 1 to 3 halogen;
carbazolyl which have 1 to 3 trihalo(lower)alkyl and/or lower alkyl;
naphthyl;
2,3-dihydorobenzo[b]oxepinyl;
2,3-dihydorobenzo[b]oxepinyl which have 1 to 3 lower alkyl;
2,3-dihydorobenzo[b]oxepinyl which have 1 to 3 halogen;
2,3-dihydoro-benzo[b]oxepinyl which have 1 to 3 lower alkanesulfonyl;
2,3-dihydoro-benzo[b]oxepinyl which have 1 to 3 lower alkoxy;
2,3-dihydoro-benzo[b]thiepinyl which have dioxo;
8,9-dihydoro-benzocycloheptyl;
chromenyl which have 1 to 3 halogen;
indolyl;
indolyl which have 1 to 3 trihalo(lower)alkyl and/or lower alkyl,
A is a group of the formula:
-(CH₂)ₜ-(O)ₘ-
or 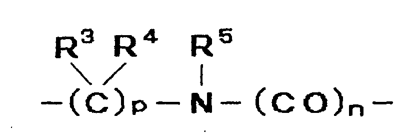 in which R³ and R⁴ are each hydrogen or linked together to form imino,
R⁵ is hydrogen or lower alkyl,
t is 0, 1 or 2,
p, m and n are each 0 or 1, X is phenylene;
phenylene which have 1 to 3 halogen;
phenylene which have 1 to 3 cyano;
phenylene which have 1 to 3 lower alkyl;
phenylene which have 1 to 3 lower alkoxy;
phenylene which have 1 to 3 lower alkylsulfonylamino;
phenylene which have 1 to 3 halogen and/or lower alkoxy;
phenylene which have saturated heteromonocyclic group containing 1 or 2 oxygen atom(s) and 1 to 3 nitrogen atom(s);
pyridinylene;
pyridinylene which have 1 to 3 halogen;
pyridinylene which have 1 to 3 trihalo(lower)alkyl;
pyridinylene which have 1 to 3 lower alkyl;
pyridinylene which have saturated heteromonocyclic group containing 1 to 4 nitrogen atom(s);
piperadinylene which have unsaturated heteromonocyclic group containing 1 to 4 nitrogen atom(s);
pyrimidinylene;
isoindolinylene, Y is vinylene;
1-propenylene;
methylene;
ethylene.

3. A compound of the formula (I):
R¹-A-X-NHCO-Y-R²
wherein
R¹ is phenyl,
R² is phenyl which have trihalo(lower)alkyl,
A is a group of the formula: 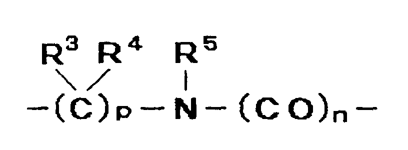 in which R³ and R⁴ are linked together to form imino,
R⁵ is hydrogen,
p is 1,
n is 0,
X is phenylene which have halogen and lower alkoxy,
Y is vinylene,
and a salt thereof.

4. A compound of the formula (I):
R¹-A-X-NHCO-Y-R²
wherein
R¹ is unsaturated heteromonocyclic group containing 1 to 4 nitrogen atom(s) which have 1 to 3 lower alkyl,
R² is indolyl which have 1 to 3 lower alkyl,
A is bond,
X is phenylene which have 1 to 3 halogen,
Y is bond,
and a salt thereof.

5. The compound of claim 4, wherein
R¹ is 4,5-di(lower alkyl)imidazolyl,
R² is 2,3-di(lower alkyl)indolyl,
X is phenylene which have 1 halogen.

6. A compound of the formula (I):
R¹-A-X-NHCO-Y-R²
wherein
R¹ is unsaturated heteromonocyclic group containing 1 to 4 nitrogen atom(s) which have 1 to 3 lower alkyl,
R² is carbazolyl,
A is bond,
X is phenylene which have 1 to 3 lower alkoxy,
Y is bond,
and a salt thereof.

7. The compound of claim 6, wherein
R¹ is 4,5-di(lower alkyl)imidazolyl,
X is phenylene which have 1 lower alkoxy.

8. A process for preparing a compound of the formula:
R¹-A-X-NHCO-Y-R²
or a salt thereof,
subjecting a compound of the formula:
R¹-A-X-NH₂
or a salt thereof,
to amidation reaction with a compound of the formula:
HOOC-Y-R²
or a salt thereof,
wherein
R¹ is heterocyclic group which may have suitable substituents, or phenyl which may have suitable substituents,
R² is condensed phenyl which may have suitable substituents, phenyl which may have suitable substituents, or thienyl which may have suitable substituents,
A is a group of the formula:
-(CH₂)ₜ-(O)ₘ-
or 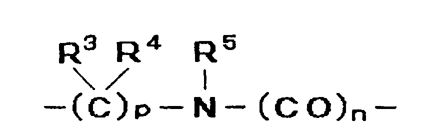 in which R³ and R⁴ are each hydrogen or linked together to form imino,
R⁵ is hydrogen or lower alkyl,
t is 0, 1 or 2,
p, m and n are each 0 or 1,
X is phenylene which may have suitable substituents, or bivalent heterocyclic group containing nitrogen which may have suitable substituents,
Y is bond, lower alkylene, or lower alkenylene.

9. A process for preparing a compound of the formula: 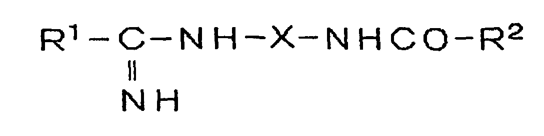 or a salt thereof,
subjecting a compound of the formula: 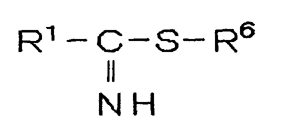 or a salt thereof,
to condensation reaction with a compound of the formula:
H₂N-X-NHCO-Y-R²
or a salt thereof,
wherein
R¹ is heterocyclic group which may have suitable substituents, or phenyl which may have suitable substituents,
R² is condensed phenyl which may have suitable substituents, phenyl which may have suitable substituents, or thienyl which may have suitable substituents,
X is phenylene which may have suitable substituents, or bivalent heterocyclic group containing nitrogen which may have suitable substituents,
Y is bond, lower alkylene, or lower alkenylene,
R⁶ is lower alkyl.

10. A process for preparing a compound of the formula: 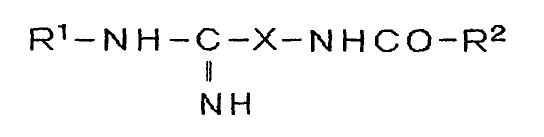 or a salt thereof,
subjecting a compound of the formula:
H₂N-R¹
or a salt thereof,
to amidation reaction with a compound of the formula: 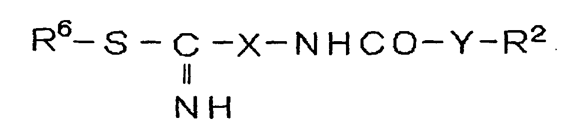 or a salt thereof,
wherein
R¹ is heterocyclic group which may have suitable substituents, or phenyl which may have suitable substituents,
R² is condensed phenyl which may have suitable substituents, phenyl which may have suitable substituents, or thienyl which may have suitable substituents,
X is phenylene which may have suitable substituents, or bivalent heterocyclic group containing nitrogen which may have suitable substituents,
Y is bond, lower alkylene, or lower alkenylene,
R⁶ is lower alkyl.

11. A process for preparing a compound of the formula:
R¹-NH-CH₂-X-NHCO-Y-R²
or a salt thereof,
subjecting a compound of the formula:
R¹-NH₂
or a salt thereof,
to reaction with a compound of the formula: 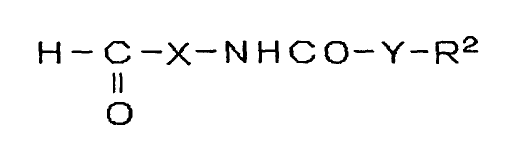 or a salt thereof,
wherein
R¹ is heterocyclic group which may have suitable substituents, or phenyl which may have suitable substituents,
R² is condensed phenyl which may have suitable substituents, phenyl which may have suitable substituents, or thienyl which may have suitable substituents,
X is phenylene which may have suitable substituents, or bivalent heterocyclic group containing nitrogen which may have suitable substituents,
Y is bond, lower alkylene, or lower alkenylene.

12. A pharmaceutical composition, which has a compound of claim 1 and a pharmaceutically acceptable carriers or excipients.

13. A process for preparing a pharmaceutical composition, which comprises a compound of claim 1 in admixture with a pharmaceutically acceptable carriers or excipients.

14. A use of a compound of claim 1 as a medicament.

15. A use of a compound of claim 1 as a 5-HT antagonist.

16. A use of a compound of claim 1 as a 5-HT_{2C} antagonist.

17. A use of a compound of claim 1 for preparing a medicament for treating 5-HT-mediated diseases.

18. A method for treating 5-HT-mediated diseases which comprises administering a compound of claim 1 to human or animals.
